(19) Europäisches Patentamt / European Patent Office / Office européen des brevets

(11) EP 2 764 099 B1

# (12) EUROPEAN PATENT SPECIFICATION

(45) Date of publication and mention of the grant of the patent:
**22.11.2017 Bulletin 2017/47**

(21) Application number: **12838648.9**

(22) Date of filing: **04.10.2012**

(51) Int Cl.:
***C12N 15/09*** *(2006.01)* ***C07K 14/78*** *(2006.01)*
***C12N 5/00*** *(2006.01)*

(86) International application number:
**PCT/IB2012/055334**

(87) International publication number:
**WO 2013/050962 (11.04.2013 Gazette 2013/15)**

## (54) ECM COMPOSITION, TUMOR MICROENVIRONMENT PLATFORM AND METHODS THEREOF

ECM-ZUSAMMENSETZUNG, TUMORMIKROUMGEBUNGSPLATTFORM UND VERFAHREN DAFÜR

COMPOSITION D'ECM, PLATEFORME À MICROENVIRONNEMENT TUMORAL ET LEURS PROCÉDÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.10.2011 IN CH33102011**

(43) Date of publication of application:
**13.08.2014 Bulletin 2014/33**

(73) Proprietor: **MITRA RXDX INDIA PRIVATE LIMITED**
**Bangalore, 560037 (IN)**

(72) Inventors:
- **SUNDARAM, Mallikarjun**
  **Bangalore**
  **Karnataka 560 037 (IN)**
- **MAJUMDER, Biswanath**
  **Bangalore**
  **Karnataka 560 099 (IN)**
- **JAIN, Misti**
  **Bangalore**
  **Karnataka 560 041 (IN)**
- **THIAGARAJAN, Saravanan**
  **Bangalore**
  **Karnataka 560 068 (IN)**
- **PINTO, Dency**
  **Bangalore**
  **Karnataka 560 037 (IN)**
- **RADHAKRISHNAN, Padhma**
  **Bangalore**
  **Karnataka 560 037 (IN)**

(74) Representative: **Heinze, Ekkehard et al**
**Meissner Bolte Patentanwälte**
**Rechtsanwälte Partnerschaft mbB**
**Widenmayerstraße 47**
**80538 München (DE)**

(56) References cited:
**WO-A1-2010/120329 US-A- 5 643 787**
**US-A1- 2006 099 675**

- **CHRIS S. HUGHES ET AL: "Matrigel: A complex protein mixture required for optimal growth of cell culture", PROTEOMICS, vol. 10, no. 9, 16 May 2010 (2010-05-16), pages 1886-1890, XP055081148, ISSN: 1615-9853, DOI: 10.1002/pmic.200900758 & CHRIS S. HUGHES ET AL: "Matrigel: A complex protein mixture required for optimal growth of cell culture; Supporting Information", PROTEOMICS, vol. 10, no. 9, 16 February 2010 (2010-02-16), pages 1886-1890, XP055178806, ISSN: 1615-9853, DOI: 10.1002/pmic.200900758**
- **SANDRA Z HASLAM ET AL: "Tumour-stroma interactions Reciprocal regulation of extracellular matrix proteins and ovarian steroid activity in the mammary gland", BREAST CANCER RESEARCH, vol. 3, no. 6, 2 August 2001 (2001-08-02), pages 365-372, XP055178653, ISSN: 1465-5411**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- N. E. CAMPBELL ET AL: "Extracellular Matrix Proteins and Tumor Angiogenesis", JOURNAL OF ONCOLOGY, vol. 72, no. 6, 1 January 2010 (2010-01-01), pages 787-13, XP055178652, ISSN: 1687-8450, DOI: 10.1016/S0945-053X(02)00010-0
- VAN BRUGGEN, M.C.J ET AL.: 'Antigen specificity of anti-nuclear antibodies complexed to nucleosomes determines glomerular basement membrane binding in vivo' EUROPEAN JOURNAL OF IMMUNOLOGY vol. 27, 1997, pages 1564 - 1569, XP055147426
- LUDWIG, T.E. ET AL.: 'Derivation of human embryonic stem cells in defined conditions' NATURE BIOTECHNOLOGY vol. 24, no. 2, 2006, pages 185 - 187, XP002422483
- CUKIERMAN, E. ET AL.: 'Taking cell-matrix adhesions to the third dimension' SCIENCE vol. 294, 2001, pages 1708 - 1712, XP055147428
- FROELING, F.E.M. ET AL.: 'Organotypic culture model of pancreatic cancer demonstrates that stromal cells modulate E-cadherin, B-catenin, and ezrin expression in tumor cells' THE AMERICAN JOURNAL OF PATHOLOGY vol. 175, no. 2, 2009, pages 636 - 648, XP055147429

## Description

## TECHNICAL FIELD

This application relates to the field of cancer and the development of prognostics and therapeutics for cancer. More specifically, the invention provides for Extra Cellular Matrix [ECM] composition, tumor microenvironment platform for culturing tumor tissue and methods thereof.

The present disclosure relates to a 'Clinical response predictor' and its application in various cancers for chemotherapy, targeted, biological drugs and broadly agents that have anti-tumor effect. The present disclosure further relates to a method for long term culture of tumor tissue, wherein said culture provides human ligands and tumor tissue microenvironment to mimic physiologically relevant signalling systems. The disclosure further relates to a method for screening tumor tissue for the presence of specific markers for determining the viability of said cells for indication of tumor status. The disclosure also relates to method of predicting response of a tumor subject and method of screening or developing anti-cancer agent.

## BACKGROUND AND PRIOR ART OF THE DISCLOSURE

Various patient segregation tools known in the art are classified broadly as below:

### Biomarkers:

There are various biomarkers that are based on the analysis of patient's tumor, normal tissue, serum, urine, saliva as well as other parts of the body &/or secreted/excreted material. For eg: Her2 is a protein as well as a gene based marker that segregates the patients who over express the protein Her2 from those who under express them. Detailed clinical investigation has been carried out in turn to show that those who have higher levels of the Her2 protein respond significantly better to the monoclonal antibody Herceptin In this context Her2 has been approved as a "Biomarker" to predict the outcome of Herceptin treatment for the patient under consideration. There are other biomarkers such as EGFR, C-MET whose presence or absence, or the expression profile is used to predict the efficacy of the targeted drugs under consideration.

For the use of a biomarker, both the axis of an XY plane need to be defined; i.e., one needs to define the quantity or quality of the biomarker on one axis, and the clinical response on the other axis. Prior to the use of the biomarker, one needs to develop an extensive amount of data for the fixed combination of the quality &/ or quantity of the biomarker as well as the clinical outcome. Once such a database has been developed, for a new patient for the same disease and the same drug, measurement of the quality or quantity of the biomarker can be used to estimate the clinical outcome if the patient is administered that particular drug. Thus a biomarker driven approach is largely constrained by many input factors such as the drug used, the disease in which it is used, and the biomarker that is used.

### Chemomarkers:

There are tests that are used to predict the efficacy of chemotherapeutics such as Cisplatin in different cancers. These tests measure the presence or absence, or the extent of presence of surrogate markers. Chemomarkers suffer from the same deficiencies of biomarkers.

### Patient's current or prior disease state:

HPV positive patients who also have Head & Neck squamous cell carcinoma respond to chemotherapy better than HPV negative Head & Neck squamous cell carcinoma patients. In this case, patients' HPV status is used as a gauge to predict their response to drugs for Head & neck squamous cell carcinoma in the event that they do develop Head & neck Squamous cell carcinoma. There is a limited amount of information available under this prognostic category. This information is largely correlation based and not necessarily causation based.

### Chemosensitivity test:

In this category of tests, patient's tumor sample is taken, homogenised into tumor cells, and this system is treated with various chemotherapeutics in *in vitro* system. Alternately, patient's tumor sample is treated with various chemotherapeutics in *in vitro* system without homogenization. These *in vitro* tests come under different names (eg. Monolayer assay or clonogenic assay from Oncotest GMBH, chemosensitivity assay from Chemofx, Extreme Drug Resistance (EDR) test from Oncotech).

Fundamental deficiency of this model is that the patient's tumor microenvironment is not captured in these chemosensitivity tests. For example, it has been claimed in several literature that in vitro cell or tissue based systems are not representative of clinical outcome.

**Cell line based in vitro and in vivo xenograft system.**

In recent times, there has been enormous number of literature published on tumor growth *in vitro* & *in vivo* pre-clinical testing based on tumor growth inhibition as well as for predicting clinical efficacy. All of the prior art methods have inherent limitations of them being not able to mimic the local microenvironment of the tumor samples and consequently poor correlation to clinical outcome that prevent their use as reliable assays for predicting clinical outcomes.

Only 10% of all the cancer drugs that enter the phase I clinical trials successfully enter the market. This low success rate is one of the main reason the cost of oncology drugs are exorbitantly high; the low success rate can be attributed to the low prediction power of current *in vitro* and *in vivo* tests in the field of oncology. Until recently, conventional studies based on 2D cell mono-layers have demonstrated their significant limitations in that the tissue architecture in three-dimensional (3-D) network of extracellular matrix components, cell-to-cell and cell-to-matrix interactions that governs differentiation, proliferation and function of cells *in vivo* is, in fact, lost under the simplified 2D monolayer condition. In the absence of specific structure as well as loss of stromal components and other cells associated with tumors, functional assays to study tumor signalling and pathways associated with tumor-maintenance, initiation and progression cannot be accurately studied. However, the prior art models are flawed in that they do not use the intact tumor microenvironment; this leads to loss of function and also change in signalling systems resulting from the lack of human derived ligands in the cell media used. More recently, the limited success of current small-molecule-inhibitors in many epithelial human cancers highlights the need to develop better techniques to more accurately predict response to therapy, preferably tailored to the individual cancer and its unique genetic and epigenetic alterations. Tumor -stroma interactions have long been recognized as important facets in the pathogenesis and dissemination of malignancy. Significant evidence supporting the role of peri-tumoral tissues in tumor maintenance includes the presence of genetic mutations in the stroma of several types of cancers and the role played by stromal cells in the acquisition of resistance to therapy. For a cultured tumor to be representative of actual cancer, it is essential that the tumor, as it proliferates *in vitro,* maintain its tissue organization and structure, its oncogenic properties, its differentiated functions, and any cellular heterogeneity that may have been present *in vivo.* If human tumors growing *in vitro* can satisfy the above criteria and, in addition, can be grown at high frequency for long periods of time in culture, they should prove valuable for basic studies in cancer biology as well as for clinically relevant testing.

The studies provided in this disclosure address the important question of whether human tumors can indeed satisfy the above criteria *in vitro.* Previous studies that use standard primary cell-culture systems and cell-line based sub cutaneous or orthotopic xenografts have advanced the understanding of tumor behavior; however, these methods have inherent limitations in evaluating the role of the tumor microenvironment in modulating carcinogenesis and tumor progression as the cell line based models have widely been recognised as homogenous models and that is one of the fundamental reason on why they do not adequately represent a heterogeneous disease such as cancer. In contrast, the instant disclosure relates to developing a systems biology approach to create an *in vitro* patient segregation tool that mimics human tumor microenvironment on plate and hence results in potential applications in different fields of cancer treatment, both in prognostics as well as translational biology. The instant disclosure also confirms the hypothesis with several examples both for prognostics as well as for translational biology applications. Further, the use of the patient segregation tool of the instant disclosure is also applicable in the development of prognostic, companion diagnostic, and translational biology applications for auto-immune disorders and inflammatory diseases.

The non patent literature prior art document CHRIS S. HUGHES ET AL, "Matrigel: A complex protein mixture required for optimal growth of cell culture", PROTEOMICS, (20100516), vol. 10, no. 9 and Supporting Information discloses that numerous cell types require a surface for attachment to grow and proliferate. Certain cells, particularly primary and stem cells, necessitate the use of specialized growth matrices along with specific culture media conditions to maintain the cells in an undifferentiated state. A gelatinous protein mixture derived from mouse tumor cells and commercialized as Matrigel is commonly used as a basement membrane matrix for stem cells because it retains the stem cells in an undifferentiated state. However, Matrigel is not a well-defined matrix, and therefore can produce a source of variability in experimental results. In this study, they present an in-depth proteomic analysis of Matrigel using a dynamic iterative exclusion method coupled with fractionation protocols that involve ammonium sulfate precipitation, size exclusion chromatography, and one-dimensional SDS-PAGE. The ability to identify the low mass and abundance components of Matrigel illustrates the utility of this method for the analysis of the extracellular matrix, as well as the complexity of the matrix itself.

The non patent literature prior art document SANDRA Z HASLAM ET AL, "Tumour-stroma interactions: Reciprocal regulation of extracellular matrix proteins and ovarian steroid activity in the mammary gland", BREAST CANCER RESEARCH, (20010802), vol. 3, no. 6 is a review focusing on the interactions among mammary stroma-derived extracellular matrix (ECM) proteins, integrins and ovarian hormone-dependent proliferation in normal and neoplastic mammary cells both *in vivo* and *in vitro. In vitro* studies show that fibronectin is required for progesterone-induced proliferation of normal mammary epithelial cells and that specific ECM proteins also regulate interactions between growth factors and ovarian hormones. Studies with human breast cancer cell lines have shown that laminin inhibits estrogen-induced proliferation and estrogen-response-element-mediated transcription *in vitro* and also inhibits estrogen-induced proliferation *in vivo.* Reciprocally, ovarian steroids regulate the expression of ECM proteins and their cellular receptors, integrins, during mammary gland development *in vivo.* The fibronectin-specific integrin, $\alpha_5\beta_1$ is regulated by ovarian steroids and its expression is positively correlated with developmental stages of peak proliferation. These studies suggest that the coordinated regulation of ovarian hormone responsiveness and ECM/integrin expression may be critical to normal mammary gland development and breast cancer growth and progression.

The non patent literature prior art document N. E. CAMPBELL ET AL, "Extracellular Matrix Proteins and Tumor Angiogenesis", JOURNAL OF ONCOLOGY, (20100101), vol. 72, no. 6, doi:10.1016/S0945-053X(02)00010-0, ISSN 1687-8450, pages 787 - 13 is a review focusing on the role that the extracellular matrix and ECM proteins play in the regulation of tumor angiogenesis. Much of the mass of a solid tumor is comprised of the stroma which is richly invested with extracellular matrix. Within this matrix are a host of matricellular proteins that regulate the expression and function of a myriad of proteins that regulate tumorigenic processes. One of the processes that is vital to tumor growth and progression is angiogenesis, or the formation of new blood vessels from preexisting vasculature. Within the extracellular matrix are structural proteins, a host of proteases and resident pro- and anti-angiogenic factors that control tumor angiogenesis in a tightly regulated fashion.

## BRIEF DESCRIPTION OF THE ACCOMPANYING FIGURES

In order that the disclosure may be readily understood and put into practical effect, reference will now be made to exemplary embodiments as illustrated with reference to the accompanying figures. The figure together with a detailed description below, are incorporated in and form part of the specification, and serve to further illustrate the embodiments and explain various principles and advantages, in accordance with the present disclosure where:

**Figure 1** shows schematic diagram depicting the development and validation of "Clinical Response Predictor" technology.
**Figure 2** shows importance of paracrine factors in explant model.
**Figure 3A** shows importance of Extracellular matrix in explant model and **3B** shows importance of microenvironment in explant model.
**Figure 4A-G** shows autologous ligands and Extra Cellular Matrix retain the microenvironment and signaling network of patient tumors in culture. Image magnification: 20X.
**Figure 5A-C** shows the composition of ECM and its effects on the viability and proliferation.
**Figure 6** shows comparison of the effects of different TPM on proliferation and activating cancer signaling proteins.
**Figure 7A-C** shows that early passages of human tumor xenografts retain molecular characteristics of original patient tumors.
**Figure 8A-C** shows that patient tumor and the xenograft derived from the same exhibit identical response outcome to anti-cancer therapy when tested in a tumor explant culture model..
**Figure 9A-H** shows that antitumor effects of TPF and Cetuximab on patient tumor explant culture is similar to response of human tumor xenografts as tested by in vivo efficacy experiments.
**Figure 10** shows correlation of "Clinical Response Predictor" guided drug response platform with efficacy in vivo.
**Figure 11** shows a schematic diagram depicting the development and validation of "Clinical Response Predictor" technology.
**Figure 12** shows the clinical validation of "clinical response predictor" analysis data in Head and Neck Cancer. M score is calculated using "clinical response predictor" and predicted outcome is correlated with clinical outcome of patient. M-score of greater than 60 is obtained for 30 patients tumors and these patients are predicted to have complete response and over 90% of these patients indeed had clinical outcome matching "clinical response predictor" analysis. Similarly about 29 patients with M-score less than 25 are predicted to be non-responders and 100% of the patients showed non-response post treatment.
**Figure 13A-S** shows the efficacy data obtained by "Clinical Response Predictor" Analysis for cancer patients treated with drugs or combinations of drugs.

## DETAILED DESCRIPTION OF THE DISCLOSURE

The present disclosure relates to an Extra Cellular Matrix [ECM] composition comprising components collagen 1, collagen 3, collagen 4, collagen 6, Fibronectin, Vitronectin, Cadherin, Filamin A, Vimentin, Laminin, Decorin, Tenascin

C, Osteopontin.

The present disclosure also relates to such composition, further comprising one or more components selected from the group consisting of Basement membrane proteins, Cytoskeletal proteins and Matrix proteins.

The present disclosure also relates to a method to obtain Extra Cellular Matrix [ECM] composition as mentioned above, said method comprising:

combining the components of the ECM collagen 1, collagen 3, collagen 4, collagen 6, Fibronectin, VitroNectin , Cadherin, FilaminA, Vimentin, Laminin, Decorin, Tenascin C, Osteopontin. The present disclosure also relates to such method, wherein the components further comprise of one or more components selected from the group consisting of Basement membrane protein, Cytoskeletal protein and Matrix protein to obtain the ECM composition.

The present disclosure also relates to a tumor microenvironment platform for culturing tumor tissue, said microenvironment comprising ECM composition as mentioned above, culture medium optionally along with serum, plasma or autologous PBMCs and drug.

The present disclosure also relates to a method for obtaining tumor microenvironment platform for culturing tumor tissue, said method comprising act of coating platform with ECM composition as mentioned above and adding culture medium optionally alongwith serum, plasma or autologous PBMCs and drug, to the platform to obtain the tumor microenvironment platform.

The present disclosure also relates to a method of organotypic culturing of tumor tissue, said method comprising act of culturing the tumor tissue on tumor microenvironment platform as mentioned above to obtain the organotypic culture.

The present disclosure also relates to a method of predicting response of a tumor subject to drug(s), said method comprising acts of:

a. culturing the subject's tumor tissue on tumor microenvironment platform as claimed in claim 3, to obtain cultured tumor tissue;
b. treating the cultured tumor tissue with the drug(s) and conducting assay;
c. converting the assay's readout into numeric metric to obtain sensitivity index and thereby, predicting the response of the subject to the drug(s); and
d. optionally, correlating the sensitivity index to clinical response of the subject to the drug(s).

The present disclosure also relates to a method of predicting response of a tumor subject to drug(s), said method comprising acts of:

a. culturing the subject's tumor tissue on tumor microenvironment platform as claimed in claim 3, to obtain cultured tumor tissue;
b. treating the cultured tumor tissue with the drug(s);
c. assessing tumor response to the drug by plurality of assays to obtain assessment score for each of the plurality of assays;
d. assigning a weightage score for each of the plurality of assays;
e. multiplying the assessment score of each of the plurality of assays with weightage score of corresponding assay of the plurality of assays to obtain independent assay score for each of the plurality of assays;
f. combining the independent assay score of each of the plurality of assays to obtain sensitivity index and thereby predicting the response of the subject to the drug(s); and
g. optionally, correlating the sensitivity index with clinical response of the subject to the drug(s).

The present disclosure also relates to a method of screening or developing anti-cancer agent, said method comprising acts of:

a. culturing subject's tumor tissue on tumor microenvironment platform as claimed in claim 3, to obtain cultured tumor tissue;
b. treating the cultured tumor tissue with the agent, assessing tumor response to the agent by assay to determine effect of said agent on the tumor cell.

The present disclosure also relates to a method for screening tumor cells for specific markers, said method comprising act of:

a. culturing subject's tumor tissue on tumor microenvironment platform as claimed in claim 3, to obtain cultured tumor tissue;

b. treating the cultured tumor tissue with drug(s) and assessing tumor response to the drug by assay; and
c. conducting microarray and Nucleic Acid analysis to screen for the biomarkers.

In another embodiment of the above ECM composition the composition is tumor specific; and wherein the collagen 1 is at concentration ranging from about 0.01 μg/ml to about 100 μg/ml, preferably at about 5 μg/ml or about 20 μg/ml or about 50 μg/ml; the collagen 3 is at concentration ranging from about 0.01 μg/ml to about 100 μg/ml, preferably at about 0.1 μg/ml or about 1 μg/ml or about 100 μg/ml; the collagen 4 is at concentration ranging from about 0.01 μg/ml to about 500 μg/ml, preferably at about 5 μg/ml or about 20 μg/ml or about 250 μg/ml; the collagen 6 is at concentration ranging from about 0.01 μg/ml to about 500 μg/ml, preferably at about 0.1 μg/ml or about 1 μg/ml or about 10 μg/ml; the Fibronectin is at concentration ranging from about 0.01 μg/ml to about 750 μg/ml, preferably at about 5 μg/ml or about 20 μg/ml or about 500 μg/ml; the Vitronectin is at concentration ranging from about 0.01 μg/ml to about 95 μg/ml, preferably at about 5 μg/ml or about 10 μg/ml; the Cadherin is at concentration ranging from about 0.01 μg/ml to about 500 μg/ml, preferably at about 1 μg/ml and about 5 μg/ml; the Filamin A is at concentration ranging from about 0.01 μg/ml to about 500 μg/ml, preferably at about 5 μg/ml or about 10 μg/ml; the Vimentin is at concentration ranging from about 0.01 μg/ml to about 100 μg/ml, preferably at about 1 μg/ml or about 10 μg/ml; the Laminin is at concentration ranging from about 0.01 μg/ml to about 100 μg/ml, preferably at about 5 μg/ml or about 10 μg/ml or about 20 μg/ml; the Decorin is at concentration ranging from about 0.01 μg/ml to about 100 μg/ml, preferably at about 10 μg/ml or about 20 μg/ml; the Tenascin C is at concentration ranging from about 0.01 μg/ml to about 500 μg/ml, preferably at about 10 μg/ml or about 25 μg/ml; and the Osteopontin is at concentration ranging from about 0.01 μg/ml to about 150 μg/ml, preferably at about 1 μg/ml or about 5 μg/ml.

In yet another embodiment of the disclosure, said tumor tissue is obtained from source selected from group comprising central nervous system, bone marrow, blood, spleen, thymus, heart, mammary gland, liver, pancreas, thyroid, skeletal muscle, kidney, lung, intestine, stomach, oesophagus, ovary, bladder, testis, uterus, stromal tissue and connective tissue or any combinations thereof, and the tumor or the tumor tissue is obtained surgically or by biopsy or as xenograft or any combinations thereof; and the tumor or the tumor tissue is divided into small pieces of about 100 μm to about 3000 μm sections.

In still another embodiment of the disclosure, in the above-referenced tumor micro environment platform the tumor tissue is obtained from source selected from group comprising central nervous system, bone marrow, blood, spleen, thymus, heart, mammary gland, liver, pancreas, thyroid, skeletal muscle, kidney, lung, intestine, stomach, oesophagus, ovary, bladder, testis, uterus, stromal tissue and connective tissue or any combinations thereof; wherein the tumor or the tumor tissue is obtained surgically or by biopsy or as xenograft or any combinations thereof; and the tumor or the tumor tissue is divided into small pieces of about 100 μm to about 3000 μm sections; wherein the culturing of the tumor tissue is carried out at temperature ranging from about 30°C to about 40°C, preferably about 37°C; for time duration of about 2 to 10 days, preferably about 3 to 7 days; and about 5% $CO_2$; and wherein the coating platform is selected from group comprising plate, base, flask, dish, petriplate and petridish.

In still another embodiment of the disclosure, the above-referenced microenvironment platform is for maintaining signaling networks of tumor cell; and for maintaining an intact tissue micro-environment, cellular architecture and integrity of tumor stroma interaction.

In still another embodiment of the disclosure, the culture medium is selected from group comprising Dulbecco's Modified Eagle Medium [DMEM] or RPMI1640 [Roswell Park Memorial Institute Medium] at concentration ranging from about 60% to about 100%, preferably about 80% 2ml; heat inactivated FBS (Foetal Bovine Serum) at concentration ranging from about 0.1% to about 40%, preferably about 2% wt/wt; Penicillin-Streptomycin at concentration ranging from about 1% to about 2%, preferably about 1% wt/wt; sodium pyruvate at concentration ranging from about 10mM to about 500mM, preferably about 100mM; nonessential amino acid is L-glutamine at concentration ranging from about 1mM to about 10mM, preferably about 5mM; and HEPES ((4-(2-hydroxyethyl)-1-piperazineethanesulfonic acid) at concentration ranging from about 1mM to about 20mM, preferably about 10mM; the serum, is at concentration ranging from about 0.1% to about 10%, preferably about 2%.

In still another embodiment of the disclosure, in the above-referenced methods the tumor is selected from group comprising stomach, colon, head & neck, brain, oral cavity, breast, gastric, gastrointestinal, oesophageal, colorectal, pancreatic, lung, liver, kidney, ovarian, uterine, bone, prostate, testicular, glioblastoma, astrocytoma, melanoma, thyroid, bladder, non-small cell lung, small cell lung, haemotological cancers including AML, Acute Myeloid Leukemia, CML, Chronic Myelogenous Leukemia, ALL, Acute Lymphocytic Leukemia, TALL, T-cell Acute Lymphoblastic Leukemia, NHL, Non-Hodgkins Lymphoma, DBCL, Diffuse B-cell Lymphoma, CLL, Chronic Lymphocytic Leukemia, and multiple myeloma or any combinations thereof.

In still another embodiment of the disclosure, in the above-referenced methods the assay is selected from group comprising assay for cell viability, cell death, cell proliferation, tumor morphology, tumor stroma content, cell metabolism, senescence or any combinations thereof; and wherein the assay for the cell viability and the cell metabolism is selected from group comprising WST assay, ATP uptake assay and glucose uptake assay; the assay for the cell death

is selected from group comprising LDH assay, Activated Caspase 3 assay, Activated Caspase 8 assay and Nitric Oxide Synthase assay, TUNEL; the assay for the cell proliferation is selected from group comprising Ki67 assay, ATP/ADP ratio assay and glucose uptake assay; and the assay for the tumor morphology and the tumor stroma is H&E, Haemaotxylin & Eosin staining; or any combinations thereof.

In still another embodiment of the disclosure, the method is used for deciding treatment for the subject from group comprising chemotherapy, targeted therapy, surgery, radiation or any combinations thereof; and wherein the sensitivity index correlates to complete clinical response, partial clinical response and no clinical response when the sensitivity index is greater than 60, between 20 to 60 and less than 20 respectively.

In still another embodiment of the disclosure, in the above-referenced methods the assigning a weightage score for each of the plurality of assays is based on nature of the drug used.

In still another embodiment of the disclosure, in the above-referenced methods the microarray and the Nucleic Acid analysis of DNA, RNA or micro RNA is carried out to detect pathway modulation before and after the drug treatment; and wherein the microarray and the Nucleic Acid analysis is confirmed using assay selected from group comprising Real-time PCR, RTPCR, Immunohistochemical, IHC, analysis and phospho-proteomic profiling.

## Overview of the Instant Method

**The overview of the protocol used in the present disclosure is provided as below:**

The first step is tissue sample collection and blood sample collection post obtaining patient informed consent. The samples are obtained through clinical collaboration using IRB approved procedures. Once, the tumor is obtained from the respective patient source, it is subjected to either the explants and/ or xenograft treatment method.

In the explants treatment method, the "Clinical Response Predictor" is performed for assessment of response in primary patient tumor.

Alternatively, when the tumor source is a xenograft, tumor from mice is excised and further subjected to explant analysis as described below. In another embodiment, the tumor is obtained and is implanted into the mice, thereafter it is allowed to grow, then excised and then subjected to the instant "Clinical Response Predictor" analysis.

Post obtaining the, tissue sample, it is divided for obtaining various inputs from a) explant assay, b) Histology based assays like IHC (immunohistochemistry) /H&E, and c) efficacy analysis from primary tumor derived xenografts.

A) Tissue sample given for explant analysis is sliced using Leica Vibratome to generate sections of about 100-3000 μm thickness. These sections are cultured in plates coated with the cancer type specific ECM composition in quadruplicate with media containing autologous sera and also various drugs optionally, for a period of about 48-96 hours. Media with drug and enriched with serum/plasma/PBMCs/serum derived ligands is changed every 24 hours. Post this period MTT/WST analysis is done to assess percent cell viability (end point assay). The supernatant from the media culture is removed every 24 hrs and assessed for proliferation (using ATP and glucose utilization experiments) and cell death (by assessment of lactate dehydrogenase assays and caspase-3 and caspase 8 measurements) to give kinetic response trends. Results are quantified against a drug untreated control. Significantly loss in cell viability/proliferation compared to untreated control is indicative of response to drug/combination and also increased cell death. The tissue sections both treated and untreated are also given for histological evaluation at the end of the culture period.

B) The tissues given for histological evaluation are assessed for apoptosis by TUNEL and activated caspase 3 assay. Also cell proliferation is assayed for standard proliferation markers like Ki67. H&E is also routinely performed to assess mitotic figures, necrosis and general gross features of the tissue.

C) Select tumors are implanted in immunocompromised mice as a xenograft to generate tumor bank for the purpose of tissue expansion and maintenance through serial passage in immunocompromised mice.

In a specific approach, xenograft study is done in order to validate the "Clinical Response Predictor" response. The sample given for xenograft study is used to implant about 3-5 immuno-compromised SCID mice to generate primary tumor xenografts that are subsequently subjected to drug efficacy estimation. As captured above, the xenograft methodology is not a routine procedure, but is a part of the validation of the instant "Clinical Response Predictor" response. As part of the "Clinical Response Predictor" response analysis, it is observed that the chemotherapeutics and targeted therapies tested in explant and xenograft system are identical to the regimen prescribed by the clinician for the patient from whom the tumor tissue is obtained.

Regardless of the solid cancer type tested, the same procedure as mentioned above is followed. The only procedural difference between the different types of solid cancers is the panel of drugs tested and the ECM composition of the coated plate. Further, the serum derived ligands are unique to each patient tested in the explant model.

Once the xenograft tumor tissue volume reaches around 500 $mm^3$, the explant system is further validated by testing the tissue in explant system identical to the parental patient tissue and correlating the efficacy data from all these preclinical readouts, i.e readouts of the xenograft system, explant system and parental patient tissue system. The time taken to generate clinical data and efficacy data for primary tumor derived xenografts is between 3-8 months. However, the turnaround time for explant analysis and histological evaluation is about 1 week.

D) All preclinical outcomes, such as cell viability, cell death by apoptosis, by cytoxicity, and also proliferation status are finally integrated to give a single score called M-score. This M-score was initially built using a training cohort. It is found that low M-score is indicative of poor response whereas high M-score corresponds to better response in clinical setting. This is further validated using a validation cohort of ~ 100 head and neck tumors, wherein M-score for the tumors are generated and correlated with clinical outcome.

The "Clinical Response Predictor" preclinical outcome is obtained in about a week and clinical outcome is gathered after about 6 months of therapy. Thereafter, the results obtained from preclinical and clinical outcomes are correlated. The same "Clinical Response Predictor" preclinical procedure is used to identify responders and nonresponders; and it is compared to the clinical outcome in multiple solid cancers.

In an exemplary method, in conjunction with the assays for "Clinical Response Predictor", tissue samples can also be assessed to determine the genetic material of the tumor tissue to understand biology of the tumor. The tumor tissue is subjected to nucleic acid isolation for assessing RNA and miRNA microarray analysis, gene analysis for specific mutations, exome sequencing of DNA and Genetic profiling.

In an exemplary method, the drug development/tumor signature/drug resistance and companion diagnostics is done in the following manner. By comparing the genetic profile of un-treated tumor samples with that of treated samples, the pathways that have been affected due to drug treatment are deduced. By looking at the total mRNA profile, the pathways that have been modulated as a result of treatment and its effect on drug response are correlated. The DNA sequence of responders and non-responders are compared to get a signature for either response or non-response. In this way, a signature for either outcome is deduced. In the case of drug resistance, the genetic material is isolated from the resistant cells in the explants and look at the pathway modulation in comparison with the untreated samples to understand the biology behind resistance. For the development of companion diagnostics, the explant read out is used to segregate responders and non-responders, and the underlying genetic information is used to reduce this to genetic signature for use as a companion diagnostic for that particular drug treatment.

In an exemplary method, one of the advantages of "Clinical Response Predictor" is the ability to both maintain intact tissue micro-environment and cellular architecture, while also preserving the integrity of the tumor-stroma interaction. It is in this biophysical and biochemical context that cells display bona fide tissue and organ specificity. Here, a method of explant culture using tissue slices to maintain the cellular architecture and microenvironment is described. The culture media is also additionally supplemented with patient derived ligands to mimic physiologically relevant signalling pathways similar to the native environment. Additionally the explant testing platform system utilizes the ECM composition that is specific for that type of cancer. In this way the explant system is a system that mimics the native host environment as closely as possible. This unique system allows us to address specific questions related to tumor signalling and the effect of small molecule inhibitors that target specific pathways within tumor environment.

In an exemplary method, the method of creation of local tumor micro-environment *in vitro* that mimics patient's tumor micro-environment is carried out. The method for long term organotypic culturing of both tumor and stromal tissue is carried out, wherein said culture provides human ligands to mimic physiologically relevant signalling systems. An organotypic culture comprising of human immune effectors and angiogenic factors to phenocopy tissue microenvironment of the host is done. In the organotypic culture the tumor tissue is obtained from solid tumors including tumors of head & neck (HNSCC), brain, oral cavity, breast, gastric, oesophageal, colorectal (CRC), pancreatic, lung, liver, kidney, ovarian, uterine, bone, prostate, testicular, and other tissues of either human or mouse origin as well as haemotological cancers including Acute Myeloid Leukemia (AML), chronic myelogenouis leukemia (CML), Acute lymphocytic leukemia (ALL), T-cell acute lymphoblastic leukemia (TALL), non-hodgkins lymphoma (NHL), diffuse Bcell lymphoma (DBCL) and chronic lymphocytic leukemia (CLL). The organotypic culture is for maintaining tumor tissue viability and signalling network by culturing said tissue in plates pre-coated with a cocktail of extra cellular proteins or defined Extra Cellular Matix specific for the stage and type of cancer obtained from a tissue type selected from the group consisting of cancers of head & neck, oral cavity, breast, ovary, uterus, gastro-intestinal, colorectal, pancreatic, prostate, glioblastoma, astrocytoma, melanoma, thyroid, kidney, bladder, non-small cell lung, small cell lung, liver, bone and other tissues of either human or mouse origin.

In another method, the organotypic culture is supplemented with ligands isolated from human serum, wherein the serum is autologus human serum, heterologus human serum. Further, the organotypic culture is supplemented with autologus human serum or heterologus human serum or with ligands isolated from autologus human plasma or with ligands isolated from heterologus human plasma or with ligands isolated from autologus human blood or with ligands

isolated from heterologus human blood or with ligands isolated from non-human serum, plasma or blood or with PBMCs isolated from autologous blood.

In yet another method, the organotypic culture is also supplemented with immune factors isolated from human blood such that it is from autologus human blood or from heterologus human blood. The organotypic culture is supplemented with autologus human plasma or with heterologus human plasma or with autologus human blood or with heterologus human blood or with immune factors isolated from non human serum, plasma or blood. The organotypic culture is also supplemented with angiogenic factors isolated from human serum such as autologus human serum, heterologus human serum. The organotypic culture is supplemented with angiogenic factors isolated from non human serum, plasma or blood or with commercially available angiogenic factors.

In still another method, tissue in said organotypic culture is viable for greater than 7 days in culture. The said culture conditions and tumor tissue are also used to study signaling networks. Further, the tumor tissue in the organotypic culture is excised and processed to maintain maximal tissue viability. The said organotypic culture is also used for screening, culture and *ex vivo* expansion of cancer cells. In another embodiment, further processing and cryopreserving of the resulting organotypic culture is also done.

In another method, the application of the instant tumor microenvironment is in the selection of the optimal treatment option for the pateint under investigation. The tumor microenvironment is also used in - selection of anti-cancer drugs for the patient under investigation, selection of anti-cancer drugs to combine with the drugs that has been selected for the patient under investigation, deciding the treatment option for the patient from among chemotherapy, targeted therapy, surgery, radiation or a combination thereof, deciding whether the patient will respond to chemotherapy, targeted therapy, surgery, Radiation or a combination thereof, selection of non-cancer drugs for the treatment of cancer patient under investigation.

In another method, the application of the instant tumor microenvironment is in the development of anticancer drugs. The tumor microenvironment is also used - in the pre-clinical or clinical development of anti-cancer drugs, to identify the types of cancers for which the anti-cancer drug under investigation has optimal activity, to identify the optimal standard of care drugs that can be combined with the anti-cancer drug under investigation to provide optimal activity, to identify the optimal doses for the anti-cancer drug under investigation to provide optimal activity, to identify the optimal doses for standard of care drugs that can be combined with the anti-cancer drug under investigation to provide optimal activity, to identify the optimal patients who can be administered the anti-cancer drug under investigation to provide optimal activity either alone or in combination with standard of care drugs.

In another method, the application of the instant tumor micro-environment is in the development of companion diagnostic tests for chemotherapies, targeted drugs. The tumor microenvironment is also used - in the development of companion diagnostic tests for chemotherapeutics or targeted drugs including biologics, to establish the "responders and non-responders" for chemotherapeutics or targeted drugs including biologics, molecular profiling of the thus selected "responders and non-responders" for chemotherapeutics or targeted drugs including biologics, which is used to develop the companion diagnostic test to pre-select the patients likely to respond to the chemotherapeutic or targeted drugs including biologics, as a functional companion diagnostic test to pre-select the patients likely to respond to the chemotherapeutic or targeted drugs including biologics.

In still another method, the application of the instant patient segregation tool is also in the development of drugs for auto-immune diseases and inflammatory disorders and in the development of companion diagnostic tests for the drugs used for auto-immune diseases and inflammatory disorders.

In another example, a method for screening tumor cells for the presence of specific markers is presented, wherein the method comprises of IHC and other techniques; and determining the viability of said cells, wherein growth and proliferation are indicative of tumor status.

In another example, a method for screening agents for their effect on tumor is presented, wherein the method comprises act of contacting candidate agents with a culture and determining the effect of said agent on the tumor cells in said culture.

In another example, the aforementioned methods/applications uses tissue slice which is from human origin or from animal origin. Further, said tissue is from the central nervous system, bone marrow, blood (e.g. monocytes), spleen, thymus heart, mammary glands, liver, pancreas, thyroid, skeletal muscle, kidney, lung, intestine, stomach, oesophagus, ovary, bladder, testis, uterus or connective tissue. In continuation, in the above methods said cells are stem cells or the cells are from more than one organ or the cells are from a healthy organ or organs or the cells are from a diseased organ or organs or the cells have been genetically altered or the cells are from a transgenic animal organ.

In another example, the present disclosure relates to a method for screening tumor cells for specific markers comprising act of - culturing the subject's tumor tissue on the present tumor microenvironment platform as claimed in claim 3 and treating the cultured tumor tissue with the drug(s) to assess tumor response to the drug by plurality of assays to obtain assessment score for each of the plurality of assays. Thereafter microarray analysis of mRNA and micro RNA is done to detect pathway modulation post treatment compared to pre treatment profile to identify putative biomarkers; confirmation of the same of targets is done using RTPCR and IHC.

The following examples further elaborate and illustrate the aspects of the present disclosure. However, these examples should not be construed to limit the scope of the instant disclosure.

## EXAMPLES

The present disclosure presents the various aspects of the invention by way of the following illustrative examples, wherein example 1 relates to preparation and coating of suitable ECM composition on cell plates which is used in the instant "Clinical Response Predictor" analysis. The setup of the "Clinical Response Predictor" analysis system is elaborated in Example 2. Examples 1 and 2 also illustrate the significance of coating the plates for the instant analysis with cancer specific ECM and adding serum derived ligands in the instant "Clinical Response Predictor" process. Example 3 provides the Explant protocol (i.e protocol of the "Clinical Response Predictor") wherein the source of the tumor tissue can be either from the patient or xenograft thereof; and the methodology of generating the xenograft tumor tissue is provided in Example 4. Example 5 presents the protocol used for determining therapeutic efficacy of drugs in tumor xenografts of SCID/nude mice, in order to validate the results obtained by "Clinical Response Predictor" Analysis. The "Clinical Response Predictor" system is then subjected to preclinical validation as illustrated in Example 6 and clinical validation in Example 14. The protocols of the assays employed in the "Clinical Response Predictor" Analysis have been provided in Example 7 and the concept of M score is presented in Example 8. The "Clinical Response Predictor" system is further tested to predict the response of multiple solid cancers in Examples 9, 13 and 14. Example 10 shows the entire protocol of "Clinical Response Predictor" comparing the results obtained with clinical outcome in order to validate the instant analysis. Example 11 and 12 provide for experimental data in order to showcase that the instant "Clinical Response Predictor" analysis is a better response predictor than biomarkers cell lines respectively.

### Example 1: Preparation and Coating of Suitable ECM Composition on Cell Plates:

Source of the tumor is primary tumor tissue from patient, derived by standard protocols. Alternatively, primary human tumor tissue is implanted sub-cutaneously in immune-compromised SCID mice to generate primary human tumor xenografts for a variety of solid cancers. Following tumor volume measurement of around $1000mm^3$, tumor is excised from the xenograft. ECM is isolated from either patient tumor or from xenograft tumor tissue according to the protocol protocol provided below.

**Isolation of human ECM and its characterization:**

Surgically removed fresh tumor tissues are dissected, cut into 1-2mm sections, and suspended in dispase solution (Stem cell Technologies Inc.) and incubated for 15min at 48°C. The tissues are homogenized in a high salt buffer solution containing 0.05M Tris pH 7.4, 3.4M sodium chloride, 4mM of EDTA, 2mM of N-ethylmaleimide and protease (Roche) and phosphatise inhibitors (Sigma). The homogenized mixture is centrifuged at 7000g for 15min and supernatant is discarded. The pellet is incubated in 2M urea buffer (0.15M sodium chloride and 0.05M Tris pH 7.4) and stirred overnight at 48°C. The mixture is then finally centrifuged at 14,000g for 20min, and resuspended in the 2M urea buffer and stored at -80°C in aliquots. Protein estimation is done using DC protein assay kit (modified Lowry, Bio-Rad) to estimate the quantity of ECM proteins isolated for quantification. Coating of tissue culture dishes are carried out with protein extracts at 37°C for 3hr.

Following ECM isolation from a variety of tumor tissue for different indications, the composition of these ECM is analyzed by mass spectrometry, the results of which are illustrated in tables 1A to 1G. Distribution and abundance of different compositions of the Extra Cellular Matix isolated from different primary tumors (HNSCC, stomach, pancreatic and colon cancers) are represented in Tables 1A to 1G. Samples are purified and subjected to LCMS analysis. Abundance of major matrix proteins is indicated for each tumor type. As illustrated in the aforementioned tables, the components required for the ECM coating is specific to each cancer type. Hence, all the above data helps in identifying the composition of the ECM to be coated on the wells towards the specific tumor/cancer type. The concentrations of the components required in the ECM mix are provided in the below table 1, which is a summarization of the tables 1A to 1G.

**Table 1: Concentration of constituents of ECM composition**

| S.No | Hu-ECM List | Coating concentration ($\mu$g/ml) |
|---|---|---|
| 1 | collagen1 | about 0.01 to about 100, preferably about 5, about 20 and about 50 |
| 2 | collagen3 | about 0.01 to about 100, preferably about 0.1, about 1, about 10 and about 100 |
| 3 | collagen4 | about 0.01 to about 500, preferably about 5, about 20 and about 250 |

(continued)

| S.No | Hu-ECM List | Coating concentration ($\mu$g/ml) |
|---|---|---|
| 4 | collagen6 | about 0.01 to about 500, preferably about 0.1, about 1 and about 10 |
| 5 | FN | about 0.01 to about 750, preferably about 5, about 20 and about 500 |
| 6 | VN | about 0.01 to about 95, preferably about 5 and about 10 |
| 7 | Cadherin | about 0.01 to about 500, preferably about 1 and about 5 |
| 8 | FilaminA | about 0.01 to about 500, preferably about 5 and about 10 |
| 9 | Vimentin | about 0.01 to about 100, preferably about 1 and about 10 |
| 10 | Laminin | about 0.01 to about 100, preferably about 5, about 10 and about 20 |
| 11 | Decorin | about 0.01 to about 100, preferably about 10 and about 20 |
| 12 | Tenascin C | about 0.01 to about 500, preferably about 10 and about 25 |
| 13 | Osteopontin | about 0.01 to about 150, preferably about 1 and about 5 |

Post assessment of ECM of different types of primary xenograft tumors in comparison with the primary donor tumor, coating experiments are also performed for testing ECM's from the same type of solid cancer (eg Colon) but isolated from different primary tumor xenografts (different primary donors). The differently coated ECM plates are also analyzed with respect to their ability to provide support/scaffold for the tissues tested in explants. All the above data is collated to arrive at the final ECM to be coated on the plate towards a specific tumor/cancer type.

The viability of tumor tissues on differentially coated ECM Matrix is monitored for a period of about 3 to about 7 days at 37°C at 5% $CO_2$.

**Table 1A: STOMACH**

| **The table below lists the components of ECM that have been isolated from gastric tumors and analyzed by LCMS.** | | | |
|---|---|---|---|
| **S.No** | **Protein** | **Name** | **% range (n=6)** |
| 1 | B1 | Collagen alpha-1 (I) chain | 0.7-49.5 |
| 2 | B2 | Collagen alpha-2 (I) chain | 0.4-39.8 |
| 3 | B15 | Collagen alpha-1 (III) chain | 1.1-38.3 |
| 4 | B33 | COL1A1 and PDGFB fusion protein | 0.8-21 |
| 5 | B34 | Tuberin isoform 1 | 0.5-15 |
| 6 | B35 | Tuberin isoform 4 | 1-19.3 |
| 7 | B36 | Tuberin isoform 5 | 0.3-35.8 |
| 8 | B37 | Protocadherin alpha-8 isoform 1 precursor | 0.9-6.3 |
| 9 | B38 | Protocadherin alpha-8 isoform 2 precursor | 0-3.5 |
| 10 | B39 | Integrin alpha-M isoform 2 precursor | 1-44.4 |
| 11 | B40 | Integrin alpha-M isoform 1 precursor | 1.5-12.7 |
| | | | |
| 1 | C1 | Actin, cytoplasmic 1 | 0.2-25 |
| 2 | C2 | Actin, cytoplasmic 2 | 0.4-37 |
| 3 | C3 | Actin, a cardiac muscle | 2-33.7 |
| 4 | C4 | Actin, a skeletal muscle | 0.2-21.3 |
| 5 | C9 | Cytokeratin, type 1 | 0.1-35.5 |
| 6 | C10 | Cytokeratin, type 2 | 0.6-21.7 |

(continued)

| The table below lists the components of ECM that have been isolated from gastric tumors and analyzed by LCMS. | | | |
|---|---|---|---|
| S.No | Protein | Name | % range (n=6) |
| 7 | C19 | Actin, aortic smooth muscle 1 | 0.4-28.9 |
| 8 | C20 | Actin, gamma-enteric smooth muscle isoform 2 precursor | 0.2-19.2 |
| 9 | C21 | Actin, aortic smooth muscle 2 | 0-9.7 |
| 10 | C30 | Dystonin | 0.2-11.3 |
| | | | |
| 1 | R23 | Protein S100-A8 | 1-20.3 |
| 2 | R25 | Annexin A1 | 3-15.1 |
| 3 | R34 | Protein S100-A9 | 2.8-12.8 |
| 4 | R64 | Hyaluronan synthase 2 | 0.2-8.6 |
| 5 | R65 | MICAL C-terminal-like protein | 0.1-6.6 |
| 6 | R66 | Chloride channel CLIC-like protein 1 | 1-16.6 |
| 7 | R67 | GDNF family receptor alpha-1 | 0.2-23.7 |
| 8 | R68 | Rab proteins geranylgeranyltransferase component A 1 | 0.9-25.7 |
| 9 | R69 | Basonuclin-1 | 0.9-30.9 |
| 10 | R70 | Eukaryotic translation initiation factor 2-alpha kinase 4 | 0.9-13.8 |
| 11 | R71 | Diacylglycerol kinase delta | 0.8-5.2 |
| 12 | R72 | AMSH-like protease | 0.8-18.3 |
| 13 | R73 | Tau-tubulin kinase 1 | 0.7-6.1 |
| 14 | R74 | Rho-associated protein kinase 2 | 0.6-26 |
| 15 | R75 | NAD-dependent deacetylase sirtuin-1 | 0.8-11.1 |
| 16 | R76 | Glycogen phosphorylase, brain form | 0.6-5 |
| 17 | R77 | Oxysterol-binding protein 1 | 0.2-10.6 |
| | | | |
| 1 | O6 | Histone H4 | 0.5-3.6 |
| 2 | O35 | POTE ankyrin domain family member F | 0.2-8 |
| 3 | O37 | POTE ankyrin domain family member E | 0.7-6 |
| 4 | O38 | POTE ankyrin domain family member I isoform 2 | 0.7-7.6 |
| 5 | O41 | Uncharacterized protein | 0.1-5.2 |
| 6 | O42 | Serum albumin preproprotein | 0.4-8.1 |
| 7 | O43 | Alpha-1-acid glycoprotein 1 precursor | 0.2-14 |
| 8 | O44 | Immunoglobulin superfamily member 2 precursor | 0.2-20.4 |
| 9 | O45 | Limkain-b1 isoform 1 | 0.1-9 |
| 10 | O46 | Limkain-b1 isoform 3 | 0.1-9 |
| 11 | O47 | Limkain-b1 isoform 2 | 0.1-9.2 |

(continued)

| The table below lists the components of ECM that have been isolated from gastric tumors and analyzed by LCMS. | | | |
|---|---|---|---|
| **S.No** | **Protein** | **Name** | **% range (n=6)** |
| 12 | O48 | Inhibitor of growth protein 2 | 0.1-9.9 |
| 13 | O49 | Hemoglobin subunit gamma-2 | 0.5-4.6 |
| 14 | O50 | Hemoglobin subunit epsilon [Homo sapiens] | 0.5-18.6 |
| 15 | O51 | Hemoglobin subunit delta | 0.5-22.5 |
| 16 | O52 | Hemoglobin subunit beta | 0.5-17.2 |
| 17 | O53 | Hemoglobin subunit gamma-1 | 0.5-31.1 |
| 18 | O54 | Ubiquitin-like modifier-activating enzyme 7 | 0.1-39 |
| 19 | O70 | NudC domain-containing protein 2 | 0.2-36.6 |
| 20 | O71 | Apoptogenic protein 1, mitochondrial | 0.2-8.6 |
| 21 | O78 | Ribosome-binding protein | 0.8-30.4 |
| 22 | O80 | Protein C15orf2 | 0.1-21 |
| 23 | O81 | Ribosomal L1 domain-containing protein 1 | 0.9-51.4 |
| 24 | O82 | Guanine nucleotide-binding protein | 0.8-38.3 |
| 25 | O83 | Krueppel-like factor | 0.1-14.3 |
| 26 | O84 | T cell receptor beta chain | 0.1-5 |

**Table 1B: CRC**

| The table below lists the components of ECM that have been isolated from colorectal tumors and analyzed by LCMS. | | | |
|---|---|---|---|
| **S.No** | **Protein** | **Name** | **% range (n=7)** |
| 1 | M1 | Myosin 1 | 0.3-25.5 |
| 2 | M9 | Myosin 9 | 0.2-12.4 |
| 3 | M24 | Isoform 9 of Fibronectin | 0.4-20.7 |
| 4 | M25 | Isoform 10 of Fibronectin | 0.3-23.1 |
| 5 | M26 | Fibronectin isoform 4 preproprotein | 0.2-11.2 |
| 6 | M31 | Isoform DPI of Desmoplakin | 0.1-15.6 |
| 7 | M37 | Mucin-12 | 0.2-33.7 |
| 8 | M38 | Elastin microfibril interfacer 1 | 0-11.6 |
| 9 | M39 | Obscurin isoform b | 0.2-12.3 |
| 10 | M40 | Obscurin isoform a | 0.1-22.2 |
| 11 | M41 | CAP-Gly domain-containing linker protein 2 isoform 2 | 0.1-20.1 |
| 12 | M42 | CAP-Gly domain-containing linker protein 2 isoform 1 | 0.2-4.8 |
| 13 | M43 | Obscurin-like protein 1 isoform 1 precursor | 0.1-5 |
| 14 | M44 | Obscurin-like protein 1 isoform 2 precursor | 0.1-13.5 |
| 15 | M45 | Obscurin-like protein 1 isoform 3 precursor | 0.1-17.4 |
| | | | |

(continued)

| The table below lists the components of ECM that have been isolated from colorectal tumors and analyzed by LCMS. | | | |
|---|---|---|---|
| **S.No** | **Protein** | **Name** | **% range (n=7)** |
| 1 | B1 | Collagen alpha-1 (I) chain | 1.1-28.3 |
| 2 | B2 | Collagen alpha-2 (I) chain | 0.9-42.8 |
| 3 | B3 | Isoform 1 of Collagen alpha-3 (VI) chain | 0-22.5 |
| 4 | B15 | Collagen alpha-1 (III) chain | 0.9-36.7 |
| 5 | B17 | Vesicle-associated membrane protein 3 | 0.6-9.2 |
| 6 | B37 | Protocadherin alpha-8 isoform 1 precursor | 4.6-5.7 |
| 7 | B38 | Protocadherin alpha-8 isoform 2 precursor | 0.8-5.2 |
| 8 | B42 | Collagen alpha-6(IV) chain isoform B precursor | 0.3-2.3 |
| 9 | B43 | Collagen alpha-6(IV) chain isoform A precursor | 0.4-1.9 |
| 10 | B44 | Collagen, type XXII, alpha 1 | 0.1-14.5 |
| 11 | B45 | Stabilin-2 precursor | 0.1-3.5 |
| 12 | B46 | Semaphorin-4G isoform 1 | 0.2-13.8 |
| 13 | B47 | Semaphorin-4G isoform 2 | 0.2-22.7 |
| 14 | B48 | Protocadherin Fat 1 precursor | 0.9-34.6 |
| 15 | B49 | Tetraspanin-11 | 0.2-4.2 |
| 16 | B50 | Collagen alpha-1(XX) chain | 0.3-18.6 |
| | | | |
| 1 | C1 | Actin, cytoplasmic 1 | 0.2-30.9 |
| 2 | C2 | Actin, cytoplasmic 2 | 0.1-19.2 |
| 3 | C4 | Actin, a skeletal muscle | 0.1-23.8 |
| 4 | C6 | Tubulin-a | 0.3-29.9 |
| 5 | C7 | Tubulin, b | 0.3-14.8 |
| 6 | C10 | Cytokeratin, type 2 | 1.1-35.2 |
| 7 | C12 | 69KDa protein | 0.5-17.7 |
| 8 | C15 | Coronin 1 A | 0.3-24.8 |
| 9 | C16 | Junction plakoglobin | 0.3-9.9 |
| 10 | C18 | Isoform 1 of Filamin-A | 0.9-15.8 |
| 11 | C22 | Vimentin | 0.2-25.5 |
| 12 | C23 | Plastin 2 | 0.2-22.4 |
| 13 | C28 | Dynein | 0.2-37.8 |
| 14 | C35 | Neurofilament heavy polypeptide | 0.5-13.6 |
| | | | |
| 1 | R1 | Calmodulin | 0.1-18.7 |
| 2 | R24 | Putative zinc finger protein 137 | 0.5-25.9 |
| 3 | R28 | Deformed epidermal autoregulatory factor 1 homolog | 0.6-16.6 |
| 4 | R29 | Glyceraldehyde-3-phosphate dehydrogenase, testis-specific | 0.6-19.5 |

(continued)

| The table below lists the components of ECM that have been isolated from colorectal tumors and analyzed by LCMS. | | | |
|---|---|---|---|
| **S.No** | **Protein** | **Name** | **% range (n=7)** |
| 5 | R30 | Transcription factor MAFK | 0.6-5.4 |
| 6 | R31 | Tryptophan 2,3-dioxygenase | 0.5-8.8 |
| 7 | R32 | Erythroid membrane-associated protein | 0.2-14.6 |
| 8 | R33 | Alkyldihydroxyacetonephosphate synthase | 0.2-15.4 |
| 9 | R37 | Calcium-binding mitochondrial carrier protein | 0.3-17.3 |
| 10 | R74 | Rho-associated protein kinase 2 | 0.6-18.1 |
| 11 | R79 | Trypsin-1 preproprotein | 0.3-32.9 |
| 12 | R83 | Chromodomain-helicase-DNA-binding protein 7 | 0.1-10.1 |
| 13 | R86 | HMG box transcription factor BBX isoform 1 | 14.5 |
| 14 | R87 | Pleckstrin homology domain-containing family | 0.2-21.9 |
| 15 | R88 | Exonuclease GOR | 0.3-33.8 |
| 16 | R89 | Neurobeachin isoform 1 | 0.4-18.9 |
| 17 | R90 | Neuralized-like protein 2 | 0.4-11.2 |
| 18 | R91 | 2',5'-phosphodiesterase 12 | 0.3-25.7 |
| 19 | R92 | E3 ubiquitin-protein ligase NEDD like | 0.3-27.8 |
| 20 | R93 | Zinc finger protein 84 isoform 1 | 0.1-22.9 |
| 21 | R94 | Calcium homeostasis ER protein | 0.2-9.5 |
| 22 | R95 | DNA primase large subunit | 0.2-20.4 |
| 23 | R96 | Rho GTPase-activating protein 22 | 0.9-28.6 |
| 24 | R97 | Protein Niban isoform 2 | 0.4-38.8 |
| 25 | R98 | ARF-GAP with coiled-coil, ANK repeat and PH domain-containing protein 3 | 0.4-14 |
| 26 | R99 | Serine protease HTRA1 | 0.2-11.7 |
| 27 | R100 | Fas apoptotic inhibitory molecule | 0.3-16.2 |
| 28 | R101 | Aspartate aminotransferase, cytoplasmic | 0.1-16 |
| 29 | R102 | Mitogen-activated protein kinase kinase kinase 4 isoform b | 0.2-22.2 |
| 30 | R103 | Protein kinase C delta | 0.1-9.9 |
| | | | |
| 1 | O20 | Hb subunits (alpha) | 0.2-15.2 |
| 2 | O23 | Ig a-1 chain C region | 0.1-27.8 |
| 3 | O41 | Uncharacterized protein | 0.3-17.5 |
| 4 | O42 | Serum albumin preproprotein | 0.7-14.2 |
| 5 | O51 | Hemoglobin subunit delta | 0.1-17.4 |
| 6 | O52 | Hemoglobin subunit beta | 0.1-28.5 |
| 7 | O53 | Hemoglobin subunit gamma-1 | 0.1-15.2 |
| 8 | O55 | Paraneoplastic antigen-like protein 6B | 0.1-16.1 |

(continued)

| The table below lists the components of ECM that have been isolated from colorectal tumors and analyzed by LCMS. | | | |
|---|---|---|---|
| S.No | Protein | Name | % range (n=7) |
| 9 | O56 | Paraneoplastic antigen-like protein 6A | 0.5-16.1 |
| 10 | O57 | Ribosome biogenesis protein BRX1 | 0-15.4 |
| 11 | O83 | Krueppel-like factor | 0.3-19.4 |
| 12 | O94 | Max-like protein X isoform gamma | 0.5-13 |
| 13 | O95 | Ribonucleoprotein PTB-binding 1 | 0.3-9.3 |
| 14 | O96 | ATP-binding cassette sub-family G member 8 | 0.1-13.0 |
| 15 | O97 | Cell division cycle 7 homolog | 0.9-3 |
| 16 | O98 | Neurolysin, mitochondrial precursor | 0.1-23.6 |
| 17 | O99 | Hypothetical protein LOC254778 | 0.5-18.1 |
| 18 | O101 | Phosphate carrier protein, mitochondrial isoform b precursor | 0.1-27.8 |
| 19 | O102 | TANK-binding kinase 1-binding protein 1 | 0.1-26.2 |
| 20 | O103 | KRT8P11, keratin 8 pseudogene 11 | 0.9-7 |
| 21 | O104 | Radical SAM domain-containing protein 1, mitochondrial precursor | 0.3-20 |
| 22 | O105 | PRO2619 | 0.5-8.4 |

**Table 1C: CaBr**

| The table below lists the components of ECM that have been isolated from breast tumors and analyzed by LCMS. | | | |
|---|---|---|---|
| S.No | Protein | Name | % range (n=6) |
| 1 | M15 | Troponin C, skeletal muscle | 0.1-11.9 |
| 2 | M33 | Lumican | 0.5-26.7 |
| 3 | M34 | Decorin isoform c | 0-14.2 |
| 4 | M35 | Decorin isoform a | 0.4-6 |
| | | | |
| 1 | B1 | Collagen alpha-1 (I) chain | 0.8-43.6 |
| 2 | B2 | Collagen alpha-2 (I) chain | 0.1-40.9 |
| 3 | B3 | Isoform 1 of Collagen alpha-3 (VI) chain | 0.3-25.9 |
| 4 | B5 | Isoform 4 of Collagen alpha-3 (VI) chain | 0.1-15.8 |
| 5 | B6 | Isoform 2C2 of Collagen alpha-2 (VI) chain | 0.5-35.2 |
| 6 | B7 | Isoform 2C2A' of Collagen alpha-2 (VI) chain | 0.4-14.9 |
| 7 | B8 | Isoform 2C2A of Collagen alpha-2 (VI) chain | 0.5-39.8 |
| 8 | B15 | Collagen alpha-1 (III) chain | 0.4-12.6 |
| 9 | B20 | Isoform 5 of Collagen alpha-3 (VI) chain | 0.8-6.6 |
| 10 | B21 | Collagen alpha-1 (XII) chain long isoform | 0.2-4.5 |
| 11 | B22 | Collagen alpha-1 (XII) chain short isoform | 0.6-14.4 |

(continued)

| The table below lists the components of ECM that have been isolated from breast tumors and analyzed by LCMS. | | | |
|---|---|---|---|
| S.No | Protein | Name | % range (n=6) |
| 12 | B23 | Collagen alpha-1 (XIV) chain | 0.1-14.3 |
| 13 | B24 | Collagen alpha-1 (VI) chain | 0.6-23.8 |
| 14 | B25 | Fermitin family homolog 3 | 0-5 |
| 15 | B26 | Ventricular zone-expressed PH domain-containing protein homolog 1 isoform 1 | 0.5-11.5 |
| 16 | B27 | Protocadherin gamma-B6 isoform 2 | 0.1-8.4 |
| 17 | B28 | Protocadherin gamma-B6 isoform 1 | 0.1-17.4 |
| 18 | B29 | Ventricular zone-expressed PH domain-containing protein isoform 1 | 1-8.7 |
| 19 | B30 | Ventricular zone-expressed PH domain-containing protein homolog 1 isoform 2 | 0.9-6.9 |
| 20 | B31 | Transmembrane protein 63C | 0.7-8.3 |
| 21 | B32 | Von Willebrand factor, type C | 2.1-8 |
| | | | |
| 1 | C8 | Actin, gamma-enteric smooth muscle isoform 1 precursor | 0.9-48 |
| 2 | C9 | Cytokeratin, type 1 | 0.9-26 |
| 3 | C10 | Cytokeratin, type 2 | 3.0-51 |
| 4 | C22 | Vimentin | 0.3-15 |
| 5 | C25 | Vinculin isoform meta-VCL | 0.1-22.3 |
| 6 | C26 | Vinculin isoform VCL | 0.2-12.2 |
| 7 | C27 | Phostensin | 0.8-13.6 |
| 8 | C28 | Dynein | 1-14.7 |
| 9 | C29 | Outer dense fiber protein 2 | 0.2-6.2 |
| | | | |
| 1 | R9 | Fructose biphosphatealdolase A | 0.5-8 |
| 2 | R10 | PyruvateKinase | 1.6-15.2 |
| 3 | R22 | Olfactomedin-4 | 0.7-15.4 |
| 4 | R25 | Annexin A1 | 0.5-9.3 |
| 5 | R26 | CreatineKinase M-type | 2.2-10 |
| 6 | R27 | Polyribonucleotide nucleotidyltransferase 1, mitochondrial precursor | 0.5-13.6 |
| 7 | R28 | Deformed epidermal autoregulatory factor 1 homolog | 0.1-11.3 |
| 8 | R48 | Insulin-like growth factor II | 0.4-13 |
| 9 | R49 | DDAH2 | 0.2-8.9 |
| 10 | R50 | Fumarate hydratase, mitochondrial precursor | 0.3-6.6 |
| 11 | R51 | Nuclear receptor coactivator 5 | 0.9-9.3 |
| 12 | R52 | Protein BEX5 | 1-13.8 |

(continued)

| The table below lists the components of ECM that have been isolated from breast tumors and analyzed by LCMS. | | | |
|---|---|---|---|
| **S.No** | **Protein** | **Name** | **% range (n=6)** |
| 13 | R53 | Protein THEMIS | 0.2-9.8 |
| 14 | R54 | Oligodendrocyte transcription factor | 0.5-17.7 |
| 15 | R55 | Sodium channel protein type 8 subunit alpha | 0.1-9.2 |
| 16 | R56 | Transcription factor HIVEP2 | 0.9-10 |
| 17 | R57 | Serine/threonine-protein kinase SMG1 | 1-20.1 |
| 18 | R58 | Putative Ras GTPase-activating protein 4B | 0.7-8.7 |
| 19 | R59 | Ras GTPase-activating protein 4 | 0.3-13.7 |
| 20 | R60 | Humanin-like protein 3 | 1.6-8 |
| 21 | R61 | Homeobox protein Nkx-6.1 | 2-16.9 |
| 22 | R62 | Sepiapterin reductase | 3.2-16.9 |
| 23 | R63 | Adenylate cyclase 3 | 1-9.4 |
| | | | |
| 1 | O8 | Histone H1 | 0.3-9.8 |
| 2 | O41 | Uncharacterized protein | 0-14.9 |
| 3 | O42 | Serum albumin preproprotein | 1.2-17.2 |
| 4 | O60 | 39S ribosomal protein L20 | 0.4-15.5 |
| 5 | O66 | Putative uncharacterized SMG1-like protein | 1.5-35 |
| 6 | O67 | Cardiotrophin-like cytokine factor 1 isoform 1 | 2-9.4 |
| 7 | O68 | Ankyrin repeat domain 13B | 0.9-12.1 |
| 8 | O69 | Leucine-rich repeat LGI family member | 1.4-23 |
| | | 2 precursor | |
| 9 | O70 | NudC domain-containing protein 2 | 0.5-12.1 |
| 10 | O71 | Apoptogenic protein 1, mitochondrial | 1-14.2 |
| 11 | O72 | 60S ribosomal protein L39 | 4-23.7 |
| 12 | O73 | Anaphase-promoting complex subunit | 0-14.1 |
| 13 | O74 | Translationally-controlled tumor protein | 0.9-6 |
| 14 | O75 | Translation initiation factor eIF-2B subunit | 1-8.6 |
| 15 | O76 | Putative Rab5-interacting protein | 0.5-8 |
| 16 | O77 | Pancreatic secretory trypsin inhibitor | 0.1-7.2 |

**Table 1D: H and N**

| The table below lists the components of ECM that have been isolated from head and neck squamous cell carcinoma tumors and analyzed by LCMS. | | | |
|---|---|---|---|
| **S.No** | **Protein** | **Name** | **% range (n=6)** |
| 1 | M1 | Myosin 1 | 0.5-27 |
| 2 | M2 | Myosin 2 | 0.1-6.4 |

(continued)

| The table below lists the components of ECM that have been isolated from head and neck squamous cell carcinoma tumors and analyzed by LCMS. | | | |
|---|---|---|---|
| **S.No** | **Protein** | **Name** | **% range (n=6)** |
| 3 | M9 | Myosin 9 | 0.2-11 |
| 4 | M17 | Isoform 1 of Fibronectin | 0.1-7 |
| 5 | M18 | Isoform 3 of Fibronectin | 0-28 |
| 6 | M19 | Isoform 4 of Fibronectin | 0.1-12.5 |
| 7 | M20 | Isoform 5 of Fibronectin | 0.1-9.3 |
| 8 | M21 | Isoform 6 of Fibronectin | 0.1-10.5 |
| 9 | M22 | Isoform 7 of Fibronectin | 0.1-10.5 |
| 10 | M23 | Isoform 8 of Fibronectin | 0.1-3.3 |
| 11 | M24 | Isoform 9 of Fibronectin | 0.1-9 |
| 12 | M25 | Isoform 10 of Fibronectin | 0.1-8 |
| 13 | M26 | Fibronectin isoform 4 preproprotein | 0-5.1 |
| 14 | M27 | Isoform 14 of Fibronectin | 0.1-21 |
| 15 | M28 | Isoform 15 of Fibronectin | 0.1-4.6 |
| 16 | M29 | Isoform 13 of Fibronectin | 0-6.8 |
| 17 | M30 | Isoform 11 of Fibronectin | 0.1-12 |
| 18 | M36 | CEA-related cell adhesion molecule 16 | 0.6-5.4 |
| | | | |
| 1 | B1 | Collagen alpha-1 (I) chain | 2.3-21.7 |
| 2 | B2 | Collagen alpha-2 (I) chain | 1.4-24.1 |
| 3 | B3 | Isoform 1 of Collagen alpha-3 (VI) chain | 1-11.6 |
| 4 | B4 | Isoform 2 of Collagen alpha-3 (VI) chain | 1-8.4 |
| 5 | B5 | Isoform 4 of Collagen alpha-3 (VI) chain | 1-18.2 |
| 6 | B6 | Isoform 2C2 of Collagen alpha-2 (VI) chain | 0.3-6 |
| 7 | B7 | Isoform 2C2A' of Collagen alpha-2 (VI) chain | 0.3-4.7 |
| 8 | B8 | Isoform 2C2A of Collagen alpha-2 (VI) chain | 0.3-9.5 |
| 9 | B15 | Collagen alpha-1 (III) chain | 0.2-13 |
| 10 | B33 | COL1A1 and PDGFB fusion protein | 0.2-9 |
| 11 | B41 | Adipocyte plasma membrane-associated protein | 0.5-13.9 |
| | | | |
| 1 | C1 | Actin, cytoplasmic 1 | 1-5.4 |
| 2 | C2 | Actin, cytoplasmic 2 | 0.4-4.6 |
| 3 | C9 | Cytokeratin, type 1 | 0.5-19 |
| 4 | C10 | Cytokeratin, type 2 | 6.6-35.8 |
| 5 | C12 | 69KDa protein | 0.2-7.9 |
| 6 | C22 | Vimentin | 0.1-14.8 |
| 7 | C23 | Plastin 2 | 0.2-6 |

(continued)

| The table below lists the components of ECM that have been isolated from head and neck squamous cell carcinoma tumors and analyzed by LCMS. | | | |
|---|---|---|---|
| S.No | Protein | Name | % range (n=6) |
| 8 | C24 | Actin-like protein 10 | 0.1-10.2 |
| 9 | C31 | Septi n-11 | 0.9-7.2 |
| 10 | C32 | Glial fibrillary acidic protein isoform 1 | 0.5-4.1 |
| 11 | C33 | Glial fibrillary acidic protein isoform 3 | 0.2-5 |
| 12 | C34 | Glial fibrillary acidic protein isoform 2 | 0.1-3.4 |
| | | | |
| 1 | R22 | Olfactomedin-4 | 1-5.4 |
| 2 | R35 | UPF0696 protein | 0.6-7.6 |
| 3 | R36 | Fragile X mental retardation 1 neighbor protein | 1.3-7.2 |
| 4 | R37 | Calcium-binding mitochondrial carrier protein | 2-7.1 |
| 5 | R38 | Nucleolar complex protein 2 homolog | 0.4-6.7 |
| 6 | R39 | Zinc finger HIT domain-containing protein 1 | 0.6-5.7 |
| 7 | R40 | 28 kDa heat- and acid-stable phosphoprotein | 0.5-3.5 |
| 8 | R41 | ADP-ribosylation factor-like protein 16 | 0.3-6.1 |
| 9 | R42 | Transcription factor LBX2 | 0.2-7 |
| 10 | R43 | Coiled-coil domain-containing protein 28B | 1-2.5 |
| 11 | R44 | Gastric juice peptide 1 | 0.2-6.1 |
| 12 | R45 | Survival of motor neuron-related-splicing factor 30 | 0.1-3 |
| 13 | R46 | DCN1-like protein 1 | 0.8-6.9 |
| 14 | R47 | R-spondin-1 | 0.6-4 |
| 15 | R77 | Oxysterol-binding protein 1 | 1.8-6.6 |
| 16 | R78 | NMDA receptor-regulated protein 2 isoform a | 0.5-6.9 |
| 17 | R79 | Trypsin-1 preproprotein | 0.1-6.4 |
| 18 | R80 | Histone-lysine N-methyltransferase SUV39H1 | 0.9-5.2 |
| 19 | R81 | E3 ubiquitin-protein ligase RING1 | 0.6-4.6 |
| 20 | R82 | Cleavage stimulation factor subunit 2 | 0.7-4.9 |
| 21 | R83 | Chromodomain-helicase-DNA-binding protein 7 | 0.9-3.9 |
| 22 | R84 | Translation factor GUF1, mitochondrial | 0.1-8 |
| 23 | R85 | CREB/ATF bZIP transcription factor | 0.6-9 |
| | | | |
| 1 | O8 | Histone H1 | 0.2-12 |
| 2 | O20 | Hb subunits (alpha) | 0.3-5.2 |
| 3 | O26 | Beta globin | 1.2-7 |
| 4 | O29 | Dermicidin | 0.6-4.4 |
| 5 | O31 | Polypeptide associated complex subunit a | 1-8.7 |
| 6 | O33 | Complement 1 Q binding protein | 0.1-7.2 |

(continued)

| The table below lists the components of ECM that have been isolated from head and neck squamous cell carcinoma tumors and analyzed by LCMS. | | | |
|---|---|---|---|
| **S.No** | **Protein** | **Name** | **% range (n=6)** |
| 7 | O41 | Uncharacterized protein | 1.3-12 |
| 8 | O42 | Serum albumin preproprotein | 0-6.3 |
| 9 | O52 | Hemoglobin subunit beta | 0.6-4 |
| 10 | O58 | Low-density lipoprotein receptor-related protein 10 | 0.6-8 |
| 11 | O59 | Protein FAM150A | 0.6-2 |
| 12 | O60 | 39S ribosomal protein L20 | 0.5-7 |
| 13 | O61 | TOMM20-like protein 1 | 0.5-6.7 |
| 14 | O62 | 60S ribosomal protein L10a | 0.1-7 |
| 15 | O63 | UPF0711 protein | 0.1-6 |
| 16 | O64 | SWI5 homolog | 0.1-3 |
| 17 | O65 | Neuroendocrine secretory protein 55 | 0.1-9.2 |
| 18 | O85 | WD repeat-containing protein 93 | 0.9-6.6 |
| 19 | O86 | SWI/SNF complex subunit SMARCC2 | 0.1-5.9 |
| 20 | O87 | Regulator of nonsense transcripts | 0-4.9 |
| 21 | O88 | MAP7 domain-containing protein 1 | 0.6-5.3 |
| 22 | O89 | Nuclear factor of activated T-cells, cytoplasmic 1 isoform A | 0.5-8 |
| 23 | O90 | NHS-like protein 1 isoform 2 | 0.4-1.7 |
| 24 | O91 | Eukaryotic translation initiation factor 4H isoform 2 | 0.6-3.6 |
| 25 | O92 | Serum amyloid A protein preproprotein | 0.1-5.8 |
| 26 | O93 | DNA repair protein RAD52 homolog | 1-8.1 |

**Table 1E: pancreas**

| The table below lists the components of ECM that have been isolated from pancreatic tumors and analyzed by LCMS. | | | |
|---|---|---|---|
| **S.No** | **Protein** | **Name** | **% range (n=6)** |
| 1 | M31 | Isoform DPI of Desmoplakin | 0.2-16 |
| 2 | M32 | Isoform DPII of Desmoplakin | 0.2-13 |
| | | | |
| 1 | B1 | Collagen alpha-1 (I) chain | 0.8-12.8 |
| 2 | B2 | Collagen alpha-2 (I) chain | 0.6-23.8 |
| 3 | B3 | Isoform 1 of Collagen alpha-3 (VI) chain | 0.08-4 |
| 4 | B4 | Isoform 2 of Collagen alpha-3 (VI) chain | 0.08-3.4 |
| 5 | B5 | Isoform 4 of Collagen alpha-3 (VI) chain | 0.08-6.5 |
| 6 | B15 | Collagen alpha-1 (III) chain | 0.2-8 |
| 7 | B33 | COL1A1 and PDGFB fusion protein | 0.3-6 |

(continued)

| The table below lists the components of ECM that have been isolated from pancreatic tumors and analyzed by LCMS. | | | |
|---|---|---|---|
| **S.No** | **Protein** | **Name** | **% range (n=6)** |
| 8 | B37 | Protocadherin alpha-8 isoform 1 precursor | 0.2-8 |
| 9 | B38 | Protocadherin alpha-8 isoform 2 precursor | 0.2-7.9 |
| 10 | B51 | Neurexin-2-beta isoform alpha-2 precursor | 0.1-7.5 |
| 11 | B52 | Neurexin-2-beta isoform alpha-1 precursor | 0.1-9.2 |
| | | | |
| 1 | C9 | Cytokeratin, type 1 | 2.5-42.4 |
| 2 | C10 | Cytokeratin, type 2 | 3.6-63.8 |
| 3 | C12 | 69KDa protein | 0.5-19.3 |
| 4 | C16 | Junction plakoglobin | 0.1-8 |
| 5 | C22 | Vimentin | 0.4-8.7 |
| 6 | C32 | Glial fibrillary acidic protein isoform 1 | 0.5-9.6 |
| 7 | C33 | Glial fibrillary acidic protein isoform 3 | 0.2-4.9 |
| 8 | C34 | Glial fibrillary acidic protein isoform 2 | 0.3-9.4 |
| | | | |
| 1 | R4 | Peptidyl-prolylcis-trans isomerase | 0.1-8.1 |
| 3 | R18 | 78 Kda glucose regulated protein | 0.1-8.6 |
| 4 | R37 | Calcium-binding mitochondrial carrier protein | 0.9-5.5 |
| 5 | R53 | Protein THEMIS | 0.3-9.8 |
| 6 | R104 | AMY-1-associating protein expressed in testis 1 | 0.1-5.1 |
| 7 | R105 | Homeobox protein Meis3 isoform 2 | 0.1-6.7 |
| 8 | R106 | Acyl-coenzyme A oxidase-like protein | 0.1-1.3 |
| 9 | R107 | Zinc finger RNA-binding protein | 0-1.3 |
| 10 | R108 | Ras-GEF domain-containing family member 1C | 0.1-4 |
| 11 | R109 | Sacsin | 0.3-9.9 |
| 12 | R110 | Fatty acid amide hydrolase | 0-9.5 |
| 13 | R111 | DNA polymerase zeta catalytic subunit | 0-2.4 |
| 14 | R112 | LON peptidase N-terminal domain and RING finger protein 1 | 1.8-8 |
| 15 | R113 | Acyloxyacyl hydrolase isoform 2 preproprotein | 3-20.5 |
| | | | |
| 1 | O16 | 60 KDA HSP | 0-7 |
| 2 | O20 | Hb subunits (alpha) | 0.3-5 |
| 3 | O21 | Iq kappa chain C region | 0.2-7.4 |
| 4 | O22 | Protein Tro alpha1 H, myeloma | 0.35-6.5 |
| 5 | O23 | Ig a-1 chain C region | 0.2-7 |
| 6 | O24 | SNC73 protein mRNA | 0.1-12.3 |

(continued)

| The table below lists the components of ECM that have been isolated from pancreatic tumors and analyzed by LCMS. | | | |
|---|---|---|---|
| **S.No** | **Protein** | **Name** | **% range (n=6)** |
| 7 | O41 | Uncharacterized protein | 1.7-20 |
| 8 | O42 | Serum albumin preproprotein | 3.8-19.3 |
| 9 | O51 | Hemoglobin subunit delta | 7.8-10.6 |
| 10 | O52 | Hemoglobin subunit beta | 0.2-13.5 |
| 11 | O53 | Hemoglobin subunit gamma-1 | 0-4.4 |
| 12 | O68 | Ankyrin repeat domain 13B | 0-2.8 |
| 13 | O85 | WD repeat-containing protein 93 | 0.6-8.8 |
| 14 | O93 | DNA repair protein RAD52 homolog | 0-2.4 |
| 15 | O99 | Hypothetical protein LOC254778 | 0.3-1.8 |
| 16 | O101 | Phosphate carrier protein, mitochondrial isoform b precursor | 1.3-7 |
| 17 | O106 | ELAV-like protein 3 | 1.7-23.2 |
| 18 | O107 | Signal peptide, CUB and EGF-like domain-containing protein 2 | 1.1-10 |
| 19 | O108 | Vacuolar protein sorting-associated protein 45 | 0-1.9 |
| 20 | O109 | Mitotic-spindle organizing protein 2B | 2-10.9 |
| 21 | O110 | Trafficking protein particle complex subunit 8 | 0-2.7 |
| 22 | O111 | AF4/FMR2 family member 1 isoform 2 | 0-15.1 |
| 23 | O112 | PDZ and LIM domain protein 3 isoform a | 0.2-11.4 |
| 24 | O113 | COBW domain-containing protein 1 isoform 2 | 0.5-6 |
| 25 | O114 | Alpha fetoprotein | 0.3-14.1 |

**Table 1F: OVARY**

| The table below lists the components of ECM that have been isolated from ovarian tumors and analyzed by LCMS. | | | |
|---|---|---|---|
| **S.No** | **Protein** | **Name** | **Percent relative abundance** |
| | | | **468** |
| 1 | M19 | Isoform 4 of Fibronectin | 0-22.9 |
| 2 | M36 | CEA-related cell adhesion molecule 16 | 0.3-13.8 |
| 3 | M46 | Matrix extracellular phosphoglycoprotein | 0.5-9.2 |
| | | | |
| 1 | B15 | Collagen alpha-1 (III) chain | 0-37.7 |
| 2 | B36 | Tuberin isoform 5 | 1-13.3 |
| 3 | B49 | Tetraspanin-11 | 0.3-23.4 |
| 4 | B54 | FCH domain only protein 1 | 0.2-5 |
| 5 | B55 | Junctional sarcoplasmic reticulum protein 1 | 0-11.5 |

(continued)

| The table below lists the components of ECM that have been isolated from ovarian tumors and analyzed by LCMS. | | | |
|---|---|---|---|
| S.No | Protein | Name | Percent relative abundance |
| | | | 468 |
| 6 | B56 | Claudin-10 | 1-12.1 |
| | | | |
| 1 | C7 | Tubulin, beta | 0.1-7.8 |
| 2 | C9 | Cytokeratin, type 1 | 2-40.6 |
| 3 | C10 | Cytokeratin, type 2 | 0.7-37.1 |
| | | | |
| 1 | R115 | DNA-directed RNA polymerase I | 0.1-16.8 |
| | | | |
| 1 | O8 | Histone H1 | 0.9-7 |
| 2 | O63 | UPF0711 protein | 0.1-1.2 |
| 3 | O69 | Leucine-rich repeat LGI family member 2 precursor | 1-13.1 |
| 4 | O74 | Translationally-controlled tumor protein | 0.5-6.7 |
| 5 | O99 | Hypothetical protein LOC254778 | 0-2.4 |
| 6 | O116 | T-cell receptor alpha chain (Mb11a) | 0.2-8.8 |
| 7 | O120 | Prorelaxin H2 | 0-6.9 |
| 8 | O121 | Interleukin-22 | 0.1-12.1 |
| 9 | O122 | Cancer/testis antigen family 45 | 0.3-11.5 |

**Table 1G: BRAIN**

| The table below lists the components of ECM that have been isolated from glioblastoma tumors and analyzed by LCMS. | | | |
|---|---|---|---|
| S.No | Protein | Name | Percent relative abundance |
| | | | 435 |
| 1 | M15 | Troponin C, skeletal muscle | 1-12.8 |
| | | | |
| 1 | B1 | Collagen alpha-1 (I) chain | 0.6-22.8 |
| 2 | B2 | Collagen alpha-2 (I) chain | 0.3-32.5 |
| 3 | B53 | Protein lin-7 homolog C | 0.9-13.3 |
| | | | |
| 1 | C10 | Cytokeratin, type 2 | 3.5-25 |
| 2 | C36 | drebrin-like protein isoform b | 1.6-21.6 |
| 3 | C37 | drebrin-like protein isoform a | 0.4-11.1 |
| 4 | C38 | drebrin-like protein isoform c | 0.1-23.8 |

(continued)

| The table below lists the components of ECM that have been isolated from glioblastoma tumors and analyzed by LCMS. | | | |
|---|---|---|---|
| **S.No** | **Protein** | **Name** | **Percent relative abundance** |
| | | | **435** |
| | | | |
| 1 | R36 | Fragile X mental retardation 1 neighbor protein | 1-22.7 |
| 2 | R114 | Sclerostin domain-containing protein 1 | 0.3-25.5 |
| 3 | R115 | DNA-directed RNA polymerase I | 0.3-15.4 |
| 4 | R116 | R-spondin-3 | 1-23.4 |
| | | | |
| 1 | O7 | Histone H2 | 3.1-20 |
| 2 | O23 | Ig a-1 chain C region | 1-33.4 |
| 3 | O41 | Uncharacterized protein | 1.5-30.5 |
| 4 | | | |
| 5 | O115 | immunoglobulin heavy chain variable region | 0.4-13.4 |
| 6 | O116 | T-cell receptor alpha chain (Mb11a) | 1.3-30 |
| 7 | O117 | Small acidic protein | 0.6-10.3 |
| 8 | O118 | Protein FAM19A5 | 3-9.7 |
| 9 | O119 | Putative protein FAM220BP | 0.2-18.5 |

In the above tables 1A to 1G, the 'B' prefixed proteins are Basement membrane proteins; 'C' prefixed proteins are Cytoskeletal proteins; 'R' prefixed proteins are regulatory proteins; 'M' prefixed proteins are Matrix Proteins; and 'O' prefixed proteins are 'others'.

From the tables 1A to 1G it is evident that tumor samples from different sources namely H&N, Stomach, Pancreas, Colon, Oesophagus and Brain have optimal viability on plates coated with ECM mix specifically formulated for the specific tumor type. ECM mix is thus obtained for each of the solid cancer indication that is used in the explants.

**Coating Process:**

Hence, once the specific ECM mix for a tumor/cancer is determined by the above aspects, the ECM mix for the specific cancer type is prepared by adding individual ECM proteins and other relevant constituents; and mixing the contents to form cocktail (cancer specific). ECM coating on about 96 well plates is achieved by using applicator sticks to uniformly coat the sides of the well. In other cases, about 200$\mu$l of ECM extract is added to the each well and allowed to dry for about 2 hrs in an incubator at about 37°C. The coated plates are washed thrice with sterile 1XPBS and stored at about -20°C for long term storage.

**Example 2: Explant Setup**

The autologous serum ligands and autologous plasma ligands and autologous PBMCs are obtained from the patient as per standard protocols.

**Example 2.1: Addition of autologous serum ligands and ECM coating recreate tumor microenvironment in culture to mimic native tumor intracellular signaling and viability.**

In another embodiment, Figures 2, 3(A) and 3(B) illustrate the nature of the instant explants system which is

designed to mimic host tumor microenvironment as closely as possible. The primary goal is to maintain tumor tissue architecture and this is where the importance of ECM component of cell plates becomes relevant. Both structural integrity and functional integrity are crucial when it comes to understanding the biology of tumor network and in elucidating drug response or resistance. Further, the instant system is devised such that it aims at maintaining the tissue microenvironment intact both from a signalling perspective as well as structural one. This is done by supplementing media with autologous serum derived ligands in explant culture, which is important for providing factors that are part of the native signalling network. This is especially relevant when it comes to testing small molecule inhibitors or targeted therapeutics or even chemotherapies that evince their action through specific pathways. By providing an environment where both structural integrity and functional signalling integrity is maintained, the instant explant model shows improved viability of the explants in culture and also that this preclinical model is clinically relevant.

Autologous serum derived ligands supplemented explants culture is required for maintaining intact native signalling networks. Addition of autologous serum derived ligands maintain signalling network crucial for mimicking native tissue micro-environment (Figure 2). Panels A and B of Figure 3 are explants cultured in the absence of autologous serum derived ligands and Panels D and E are explants cultured in medium supplemented with autologous serum derived ligands.

On performing IHC for cMet [MET or MNNG HOS Transforming gene], explants cultured in the presence of autologous serum derived ligands show markedly enhanced presence of cMet, testifying to the fact that the autologous serum drived ligands supplemented medium contains paracrine factors crucial for mimicking native signalling network. Figure 3A illustrates that plates coated with cancer type specific ECM components provide appropriate support/scaffold to help maintain intact tissue architecture which is crucial for mimicking native tissue micro-environment. Explants cultured on cancer type specific ECM components coated plates improve cell viability and provides support/scaffold to maintain tissue architecture. H&E staining of tumor tissue cultured on plates with ECM coating (panels C and E) and without (panels B and D). Figure 3B shows that explants cultured in ECM coated plates along with media supplemented with autologus serum derived ligands show improved cell viability.

**Example 2.2: Autologous ligands and Extra Cellular Matix composition retain the microenvironment and signaling network of patient tumors in culture.**

Biopsy tumors from HNSCC patients are sectioned (~200micron) and cultured in 96 well plates with about 10% FBS (control) or about 2% autologous serum and about 8% FBS (Autologous serum) in RPMI media for about three days. Cell viability is measured by WST assay. Percent cell viability is calculated and presented as Box and whisker plot (Figure 4(a)). Horizontal line in the middle portion of the box denotes mean. Bottom and top boundaries are $25^{th}$ and $75^{th}$ percentiles respectively, lower and upper whiskers, $5^{th}$ and $95^{th}$ percentiles respectively. ***P* < 0 compared to control.

IHC data showing specific effect of autologous serum on proliferation of tumors is plotted as in Figure 4(b). Tumors sections are stained with H&E and antibodies against Ki67 for evaluating morphological changes and cell proliferation. The image magnification is 20X.

Figure 4(c) shows the effects of Extra Cellular Matix composition on tumor viability. Inner surface of culture plate is coated with gelatin, collagen, matrigel or Extra cellular Matrix (ECM) isolated from HNSCC and tumor sections are cultured for 3 days. Cell viability is measured by WST and percent cells viable is calculated (mean $\pm$ s.e.m.). **P* < 0.01comapred to T72 control (analysis of variance). *n* = 8. Figure 4(d) shows IHC profiles of explant tumors at about 3 days post culture in presence of ECM composition. H&E (left) and Ki-67(right). Ki-67 score implying the better effect of ECM composition compared with other types matrix support as indicated in Figure 4(e).

Combination of Autologous Serum and ECM composition shows greater effects on proliferation than single complement, Figure 4(f). Tumor sections are cultured for about 72hours with control, autologous serum (about 2%), ECM composition (about 100ug/ml) alone or combination of both. Ki-67 score indicates benefit of combined presence of ECM composition and autologous serum in culture. **P* < 0.01 compared to T72 control, ***P* < 0.05 compared to ECM composition alone and AS alone (analysis of variance) n = 5. Corresponding IHC data in Figure 4(g) reveal similar increase in Ki-67 positive cell upon addition of ECM composition and autologous serum. Tumor tissues are embedded in paraffin and sectioned (5 micron) and stained anti Ki-67 antibodies.

**Example 2.3: Composition of ECM and its effects on the viability and proliferation.**

Extra Cellular Matix composition (ECM) is coated on plates before culturing of tumor tissue as per the percent composition of the components of ECM indicated in Figure 5(a). Explants are cultured for about 72 hours in plates coated with different doses (1, 10 and 100·g/ml) of ECM isolated from heterologous human tumor sources. Percentage of tumor cell viability (mean$\pm$s.e.m) is measured by WST. **P* < 0.05, ***P* < 0.01 compared to the T0 and T72 control respectively by ANOVA. As seen in Figure 5(b) ECM increases the viability of tumors in a dose dependent manner. Maintenance of

overall intra-tumoral heterogeneity and integrity is determined by H&E (Figure 5(c) top), and tumor cell proliferation (Figure 5(c) bottom) in explant settings is evaluated using Ki-67 antibodies. Data representative of 5 independent experiments performed in triplicates.

**Example 2.4 Comparison of the effects of different ECM Composition on proliferation and activating cancer signalling proteins.**

Inner surface of culture plate is coated with gelatin, collagen, matrigel or Extra Cellular Matix (ECM) isolated from colon cancer and tumor sections are cultured for 3 days. IHC (immunohistochemistry) profiles of explant tumors shown in Figure 6(a) indicate that patient derived ECMs exert greater effect on proliferation (Ki67) and phosphorylation of ERK1/2 than standard single matrix protein. Figure 6(b) shows the corresponding Ki-67 score. *$p < 0.01$ compared to T72 control, by ANOVA ($n = 5$). Scatter plot of Ki-67 score displayed in Figure 6(c) indicate patient derived ECM positively affect explants in multiple independent experiments performed under similar conditions. Each dot represents one experiment (n=33). Horizontal line represents mean of all samples. ***$P < 0.001$ compared with control. The above results indicate that culturing tumor in the presence of the appropriate ECM improves viability and signaling of tumor tissue to mimic host tumor microenvvironment.

**Example 3:**

Once the tumor tissue is obtained from the patient or xenograft source, it is subjected to Explant Protocol/"Clinical Response Predictor" as below:

**Explant Protocol:**

1. About 3x3x3 mm small pieces of tumor slice (all uniform size and free of necrotic mass) is generated.
2. Tumor sample is divided into multiple small pieces using Leica Vibratome to generate about 100-3000 μm sections and cultured in triplicate in 96 well flat bottom plates that have been previously coated with appropriate ECM composition.
3. Tumor tissues are maintained in conditioned media of about 2ml (DMEM supplanted with about 2% heat inactivated FBS along with 1% Penicillin-Streptomycin, sodium pyruvate 100mM, nonessential amino acid, L-glutamine 4mM and HEPES 10mM. The culture media is supplemented with about 2% serum derived ligands or about 2% of plasma derived ligands after 12 hours or about 100,000 PBMCs per 96 well is seeded with about 10% of autologous plasma and cultured for about 72hrs at about 37°C with about 5% $CO_2$ under humid conditions.
4. Change the media at the time of serum/plasma/PBMC addition.
5. The media is changed every 24 hours along with supplements.
6. 5μl of spent media is used to determine cell viability, cell metabolism, cell death and cell physiology of tumor tissue.
7. At the end of culture period ranging from about 48 hours to about 120 hours, the tissue is asseSsed for various parameters. Post this period MTT/WST analysis is performed to assess percent cell viability. The supernatant from the media culture is removed every 24 hrs and assessed for proliferation (using ATP and glucose utilization experiments) and cell death (by assessment of lactate dehydrogenase assays and caspase-3 and caspase 8 measurements) to give kinetic response trends. Results are quantified against a drug untreated control. Significantly loss in cell viability/proliferation compared to untreated control is indicative of response to drug/combination and also increased cell death. The tissue sections both treated and untreated are also given for IHC and histological evaluation at the end of the culture period. The tissues given for histological evaluation are assessed for apoptosis by TUNEL and activated caspase 3 assay. Also cell proliferation is assayed for standard proliferation markers like Ki67. H&E is also routinely performed to assess mitotic figures, necrosis and general gross features of the tissue.

**Example 4:**

Once the ECM composition is determined and it is coated onto the wells, the tumor from the patient or the xenograft source is subjected to explant analysis. To generate human tumor xenograft for explants analysis, the tumor is initially implanted into the SCID mice and thereafter the excised tumor is subjected to explants protocol and "Clinical Response Predictor" anaylsis. The protocol for the same is provided below:

**Animal Implantation and Tumor Xenograft Generation:**

*Sample Preparation:*

1. Transfer freshly removed human tumor sample in about 50ml tube containing DPBS (about 5ML).
2. Remove sample and dissect sample for variety of experiments.
3. Transfer remaining sample to a sterile petri dish containing about 2 ml DPBS.
4. Cut tumor into pieces with sterile scalpel blade about the size of a pencil eraser (about 5x5x5 mm). Care should be taken to make the pieces as uniform as possible. *Animal Preparation:*
5. Pick up the animals using a conventional grasp with the index and middle fingers placed around the neck and over the front legs.
6. Rinse the surface of female SCID mice aged about 5-6 weeks with about 70% ETOH.

*Implantation of sample into mice:*

7. Use both flanks for solid implantation at subcutaneous space.
8. After a mice body surface is rinsed, it is placed (ventral side down) in a properly sized nose cone or on the lid of the mice cage with dorsal side facing upwards.
9. Using gauze square saturated with about 70% (vol/vol) ethanol wipe the area from the mid-spine to the base of the tail to prepare for the insertion of tumor with trochar.
10. Immediately before implantation bathe/rinse the tumor piece into about 100X P/S.
11. Take solid piece of tumor (about $5mm^3$) on the tip of trochar and push it inside using sterile forcep or scalpel without letting tumor sample dry.
12. Insert the tip of the trochar into mice subcutaneous space horizontally above the base of the tail, directly cover the flank and introduce a pocket in the subcutaneous space and insert up to the middle of dorsal side on both flank (one at a time) while holding the plunger part and needle part in a fixed position and without damaging the peritoneum.
13. Push the individual piece of tumor (about 50 mg or about 5 $mm^3$) into the pocket created using trochar.
14. Gently remove the trochar without disturbing the inserted material.
15. Properly mark the mice using nontoxic material (head/ body/tail/no mark etc.).

*Animal follow-up:*

16. Return the mice to a clean cage.
17. Palpable tumor (about $50mm^3$) is noticed first and then it starts growing.
18. Monitor the mice daily and measure the tumor growth weekly, using caliper measurement as described below. Briefly, tumor growth is monitored weekly by bioluminescent imaging or external caliper measurements (tumor size = [length $\times$ width $\times$ height] $\times$ 0.52) for about 5-16 weeks.
19. When the tumor reaches a maximum size of about 700 $mm^3$, euthanize the animal and remove the tumor and follow the same procedure.
20. Removed tumors are divided into multiple pieces for different studies. Additionally, potential metastasis sites such as lungs and lymph nodes, abdomen and in select cases brain are removed and sent in formalin.
21. If animal has a slow growth of tumor it is implanted at early stage to rescue growth. All animals are euthanized at the end of 16 weeks.

**Example 5:**

The protocol for validating the 'Clinical Response Predictor' by xenograft analysis is provided below:

**Determination of Therapeutic Efficacy Of Drugs In Tumor Xenografts Of Scid/Nude Mice:** ***Animal Preparation:***

1. Use the mice from P1 or subsequent passage (P1 or $P_{N,\ N>1}$) for efficacy studies.

***Animal Follow-up* & *Dosing:***

2. Monitor the mice daily and measure the tumor growth weekly, using caliper measurement as described below. Tumor volume is calculated using the following formula:

$$\text{Tumor volume (mm}^3\text{)} = L \times W^2/2;$$

where L=length (mm), W=width (mm).

3. When the tumor reaches a size of about 150-200 $mm^3$, dose the animals with appropriate drug (about 1 dose/week) for about 5 weeks. Monitor the mice daily and measure the tumor growth bi-weekly, using caliper measurement as described above.
4. Follow the animals for about 4 weeks post treatment for tumor regression/growth.
5. At the end of study, euthanize the mice as per the standard euthanization procedure using $CO_2$ chambers and collect tumor samples.

The detailed list of drugs that is used for testing in the instant disclosure is given in the below Table 2. This list is only for illustrative purpose and is non-limiting and non-exhaustive.

**Table 2: List of drugs administered in the "Clinical Response Predictor" Analysis**

| Cancer Type | Sub-Type | Drug Combination (Standard of Care) |
|---|---|---|
| a) H&N cancer | Nasopharynx | Cisplatin |
| | | Carboplatin & Paclitaxel |
| | | Cisplatin & 5-FU |
| | SCC | Cisplatin |
| | | Docetaxel, Cisplatin & 5-FU |
| | | Cetuximab |
| | Salivary tumor | Cisplatin & 5-FU |
| | | |
| b) Colorectal cancer | Resectable | Oxaliplatin, 5-FU, leucovorin |
| | | 5-FU & Leucoverin |
| | Un-Resectable | Oxaliplatin, 5-FU, leucovorin |
| | | 5-FU & Leucoverin |
| | | Irinotecan, 5-FU, Leucoverin |
| | | Irinotecan, 5-FU, Leucoverin, Bevacizumab |
| | | Irinotecan & Cetuximab |
| | | Panitumumab |
| | | |
| | | Epirubicin, Cisplatin & Capecitabine |
| | Locally Advanced | Docetaxel, Cisplatin, Infusional 5-FU |
| | | Epirubicin, Cisplatin & Capecitabine |
| | Metastatic | Docetaxel, Cisplatin, Infusional 5-FU |
| | | |

(continued)

| Cancer Type | Sub-Type | Drug Combination (Standard of Care) |
|---|---|---|
| c) Stomach & Oesophagus cancer | Metastatic | Epirubicin, Cisplatin & Capecitabine |
| | Gastric, Gastro-esophageal (Her2+) | Herceptin |
| | GI Stromal | Imatinib |
| | GI stromal Resistant to Imatinib | Sunitinib |
| d) Pancreas, Gall Bladder, Bile cancer | Gall Bladder | Cisplatin & Gemictabine |
| | Colangiocarcinoma | Cisplatin & Gemictabine |
| | Adenocarcinoma | Cisplatin & Gemictabine |
| | Reseatced Pancreatic Carcinoma | 5-FU & Leucovorin |
| | Ca-Pancreas | Erlotinib |
| | | |
| e) Liver cancer | Hepatocellular Carcinoma | Sorafenib |
| | | |
| f) Ovarian cancer | Germ cell cancer | Bleomycin, Etoposide, Cisplatin |
| | Platinum sensitive Relapsed Ca | Trabectidin, PLD Doxorubicin |
| | Platinum resistant Ca | Docetaxel |
| | Soft tissue carcinoma | Trabectidin, PLD Doxorubicin |
| | Advanced Ca (progress/ recurrence) | Carboplatin & Gemcitabne. |
| | peritoneal carcinoma | Docetaxel |
| | Fallopian Tube Carcinoma | Docetaxel |
| | Relapsed epithilial Carcinoma | Doxirubicin (PLD) and Carboplatin. |
| | Papillary Ca | Carboplatin & Paclitaxel |
| | Peritoneal Ca | Carboplatin & Paclitaxel |
| | Fallopian tube carcinoma | Carboplatin & Paclitaxel |
| | Invasive epithilial Ca | Carboplatin & Paclitaxel |
| | | |
| g) Breast Cancer | Primary | Cisplatin and Gemcitabine |
| | | Cyclophosphamide & Paclitaxel |

(continued)

| Cancer Type | Sub-Type | Drug Combination (Standard of Care) |
|---|---|---|
| | | Cyclophosphamide, Doxorubicin and Docetaxel |
| | | Docetaxel and Cyclophosphamide |
| | | Docetaxel, Cyclophosphamide, Epirubicin and Fluorouracil |
| | | Filgrastim, Cyclophosphamide, Doxorubicin and Fluorouracil |
| | | Filgrastim, Cyclophosphamide, Epirubicin and Fluorouracil |
| | | Gemcitabine and Docetaxel |
| | Her2+ primary | Trastuzumab, Cyclophosphamide, Doxorubicin and Paclitaxel |
| | | Cyclophosphamide, Paclitaxel and Trastuzumab |
| | | Docetaxel, Carboplatin and Trastuzumab |
| | | Docetaxel, Trastuzumab, Fluorouracil, Epirubicin and Cyclophosphamide |
| | | |
| | Hormonal | LHRH agonist and tamoxifen |
| | | Tamoxifen |
| | Early Ca-Br | Doxorubicin and Cyclophosphamide followed by Weekly Paclitaxel |
| Cancer Type | Sub Type | Drug combinations (SOC) |
| Breast Cancer | High risk Ca-Br | Cyclophosphamide (oral), Methotrexate and Fluorouracil |
| | Locally advanced | Doxorubicin and Cyclophosphamide followed by Docetaxel (TAXOTERE). |
| | | Cyclophosphamide, Doxorubicin and Fluorouracil |
| | | Cyclophosphamide, Epirubicin and Fluorouracil |
| | | Cyclophosphamide, Epirubicin, Fluorouracil and Filgrastim (G-CSF) |
| | Locally advanced (Her 2 +) | Doxorubicin and Cyclophosphamide followed by Docetaxel (TAXOTERE) and Trastuzumab |
| | Metastatic | Anastrozole |
| | | Capecitabine |
| | | Cyclophosphamide, Doxorubicin and Fluorouracil |
| | | Docetaxel |
| | | Docetaxel and Capecitabine |
| | | Doxorubicin |
| | | Doxorubicin and Cyclophosphamide |
| | | Enanthate |
| | | Gemcitabine |
| | | Gemcitabine and Paclitaxel |
| | | Paclitaxel |
| | | Vinorelbine |

(continued)

| Cancer Type | Sub-Type | Drug Combination (Standard of Care) |
|---|---|---|
| | Metastatic (Her2+) | Trastuzumab |
| | | Trastuzumab and Docetaxel |
| | | Trastuzumab and Paclitaxel |
| | | |
| | Metastatic | Trastuzumab and Vinorelbine |
| | | |
| | Bone metastases | Clodronate |
| | | Pamidronate |
| | | |
| | Advanced Ca-Br | Cyclophosphamide, Methotrexate and Fluorouracil |
| | | Exemestane |
| | | Letrozole |
| | | Megestrol |
| | | |
| | Advanced Ca-Br (Her2+) | Trastuzumab, Paclitaxel and Carboplatin |
| | | |
| | Inflammatory Ca-Br | Cyclophosphamide, Epirubicin and Fluorouracil |
| | | Cyclophosphamide, Doxorubicin and Fluorouracil |
| | | Filgrastim, Cyclophosphamide, Epirubicin and Fluorouraci |

**Example 6: Preclinical Validation of Explant System:**

Tumor xenografts (HTX) generated from tumors are known to be similar to patient tumor and hence efficacy read out obtained from such a system is indicative of patient's response to that treatment. In the instant Explant system, sensitivity of HTX when treated with drug combinations is very similar to response outcome from explant for the same tumor indicating that the "Clinical Response Predictor" explant system has high degree of predictability of the patients' response to drugs or combination of drugs. The same is been illustrated by the following sub-examples:

**Example 6.1: Early passages of human tumor xenografts retain molecular characteristics of original tumors.**

Early passages of human tumor xenografts retain molecular characteristics of original tumors. Figure 7(A) illustrates 3D PCA plot generated by Genespring GX software to show the tight clustering of samples of same origin and serial passage. The plot shows about 6 distinct clusters comprising of about 4 pairs of colon carcinoma and about 2 pairs of HNSCC samples. Unsupervised two dimensional hierarchal clustering of colon cancer and HNSCC is illustrated in panel B of Figure 7.

**Example 6.2: Tumor explant culture derived from early passages of human tumor xenograft and patient tumor exhibits identical antitumor effect.**

Tumor explant culture derived from early passages of human tumor xenograft and patient tumor exhibits identical antitumor effect. Explants derived from primary donor tumors (P0) and post grafts (P1 and P2) generated from it are treated with TPF (Ciplatin, Docetaxel, 5FU) or DMSO [Dimethyl Sulfoxide] as control. About seventy two hours post treatment, viability is measured by WST and percent inhibition of viability is calculated (mean ±s.e.m.) using corresponding DMSO control as 100% (Figure 8a). Ki-67 immunoreactivity pattern of explants resulting from P0 and P1, P2 tumors following TPF treatment. Image magnification is 200X (Figure 8b). Representative Ki-67 score indicating reduction

of proliferating cells within explants and measured based on the calculation of Ki-67 positive cells per field (mean ± s.e.m. of triplicates). *$P < 0.05$, **$P < 0.01$ compared to the corresponding control by ANOVA. $n$ =4 (Figure 8c). The data indicates that parental tumors and subsequent xenografts maintain identical response status to TPF and primary tumors that are originally refractory are found to maintain the same pattern in subsequent xenografts.

Antitumor effects of TPF and Cetuximab on tumor explant culture are similar to human tumor xenograft models. Biopsy tumors from HNSCC patients are sectioned (~200 micron) and cultured in ECM coated 96 well plates with about 2% autologous serum and about 8% FBS in RPMI media for about three days with DMSO (Control) and Docetaxel, Cisplatin and 5-FU (TPF). Cell viability is measured by WST and percent cell viability is calculated. Box plot in Figure 9(a) is showing significant inhibition of viability in TPF treated tumors. ** p<0.001 compared to control in multiple donors (n=20) by paired T test. The Figure 9(b) shows the corresponding IHC profile. Tumor sections treated with DMSO (control) and TPF are stained with H&E and Ki-67. Scatter plot representing explant samples that differentially showed response to TPF (normalized fold inhibition to T72 control). Each dot represents one experiment (n=50) Horizontal line represents mean of all samples. Non-responders have very low levels of inhibition compared to responders. Figure 9(c) shows tumor growth inhibition *in vivo.* The same patient tumors are grown in immunocompromized mice. Tumor bearing mice are treated daily with normal saline (Control) and (TPF) for 21 days. Tumor volumes are measured at indicated time points. Data are mean tumor volume ±s.e.m of 6 mice per groups.*'p<0.001 compared with corresponding vehicle control. Tumors are dissected, weighed and percent residual tumors are calculated. Representative IHC features of tumors at the end of treatment are illustrated in Figure 9(d). Tumors dissected from euthanized mice from both control and TPF groups are embedded, sectioned and stained with H&E, Ki-67 and TUNEL as indicated. Scale bars, 50μm and insets 100μm.

Biopsy tumors from HNSCC patients are sectioned (~200micron) and cultured in ECM coated 96 well plates with about 2% autologous serum and about 8% FBS in DMEM media for three days with DMSO (Control) and Cetuximab. Cell viability is measured by WST. Box plot of Figure 9(e) represents percent inhibition of cell viability in Cetuximab treated tumors in multiple experiments compared with corresponding controls (n=20). **' p<0.001 compared with control in multiple as calculated by paired T test. Representative IHC picture, Figure 9(f) illustrates changes in proliferation and morphology. Tumor sections treated with DMSO (control) and Cetuximab are stained with H&E and Ki-67. Scatter plot represents explant samples that differentially showed response to Cetuximab (normalized fold inhibition to T72 control). Each dot represents one experiment (n=40) Horizontal line represents mean of all samples. **$P < 0.001$ compared with control. Figure 9(g) represents the tumor growth inhibition in Cetuximab treated mice. HNSSC tumors used for Cetuximab explant culture (e) are grown in immunocompromised mice and treated with normal saline (Control) or Cetximab (Treated) three times a week for about 23 days. Tumor volumes are measured at indicated time points. Data are mean tumor volume ±s.e.m of 10 mice per groups.*'p<0.001 compared with corresponding vehicle control. Figure 9(h) represent IHC data highlighting molecular changes akin to tumor inhibition in vivo. Tumors are harvested from euthanized mice about 6 hours after the last dose of Cetuximab. Tumor sections are stained with H&E, Ki-67, TUNEL and pERK. Scale bars 50·m, and insets 100·m.

**Example 6.3: Correlation of "Clinical Response Predictor" guided drug response platform with efficacy in vivo.**

Data obtained from TPF/cetuximab treated explants are independently scored for assessing the inhibition of viability, proliferation and induction of apoptosis. Relative contribution of each assay is determined as elaborated in Example 8. A final composite "Clinical Response Predictor" response score (inhibition) is calculated by integrating all the components of the tumor inhibition and correlating it with tumor growth inhibition data obtained from *in vivo* efficacy studies using same individual patient tumors and drugs in HTX. Spearman correlation co-efficient method is used to calculate linear association. $R^2$ value signified positive correlation between in vivo response and "Clinical Response Predictor" guided response (n=15) (Figure 10). The level of tumor inhibition seen in "Clinical Response Predictor" corresponds to that seen in HTX model

**Example 7: Assays Employed in the Explant Protocol**

The tumor samples obtained from the patient or the xenograft source are thereafter subjected to the "Clinical Response Predictor" analysis by way of the following assays to obtain the M Score. The concept of M-score is elaborated in Example 7.

**Example 7.1: Assays For Determining Cell Viability:**

**a) MTT ASSAY FOR MEASURING TISSUE VIABILITY OF SOLID TUMOR EXPLANTS:**

Modified version of regular MTT assay (Veira V et al Max Loda Lab PNAS 2010) is used. Briefly, tissues are

cut precisely into equal sections by vibratome (400 micron slice) and cultured in RPMI 1640 [RPMI - Roswell Park Memorial Institute] at concentration ranging from about 60% to about 100%, preferably about 80%; for up to 72 hours. Tissue viability is assessed using an MTT 1-(4, 5- dimethyltiazol-2-yl)-3, 5-diphenylformazan assay (Sigma Aldrich) at time point T0 and also at T72. Tissue slices are incubated with 5 mg/mL of MTT at 37°C for 4 hours, harvested, and precipitated-salt extracted by incubation with 0.1 M HCl-isopropyl alcohol at room temperature for 25 min. A viability value is determined by dividing the optical density of the formazan at 570 nm by the dry weight of the explants. Baseline samples (TO) are used as calibrators (1×) to normalize inter sample variation in absorbance readings, and tissue viability is expressed as a percentage of viability relative to T0 samples. For different cancer types, tissue slices in explants are incubated with media containing different drugs at peak plasma concentration for up to 72hrs. Media containing drugs are changed every 24 hrs and MTT is performed at the T72 and T0 time point as usual. To assess drug efficacy, tissue viability at the end of the study period is graded relative to tissue viability at the T0 time point wherein the tissue is not exposed to any drug.

**b) WST ANALYSIS:**

Briefly, tissues are cut precisely into equal sections by vibratome (400 micron slice) and cultured in RPMI 1640 [RPMI - Roswell Park Memorial Institute] at concentration ranging from about 60% to about 100%, preferably about 80%; for up to 72 hours. Tissue viability is assessed using an WST ASSAY. At the end of 72 hrs incubation 40µl of CCK-8 (cell counting kit-8, Dojindo Laboratories, Japan) is added to each wells and incubation was continued for another 3 hrs at 37°C/5% $CO_2$. During the incubation the plate was gently agitated inside the incubator at about 90 rpm on the micro plate shaker. At the end of about 3 hrs incubation, tissue slices are carefully removed to the respective 10% formalin tubes and submitted for the Immuno-histochemical studies. Parallely, absorbance is measured at 450nm using micro plate reader (Bio-Rad,USA).

**c) ATP UTILIZATION ASSAY:**

Adenosine-5'-triphosphate (ATP) is a central molecule in the chemistry of all living things and is used to monitor many biological processes. ATP utilization is studied using the **StayBrite™ ATP Assay Kit** (BioVision). An accurate, reliable method to detect minute ATP levels is the Luciferase/Luciferin. The assay is fully automated for high throughput (1 sec/sample) and is extremely sensitive and is ideal for detecting ATP production.

$$\text{Luciferin} + \text{ATP} + O_2 \xrightarrow{Mg^{2+}\ \text{Luciferase}} \text{Oxyluciferin} + \text{AMP} + \text{Pyrophosphate} + CO_2 + \text{Light}$$

**Standard Curve:** To calculate absolute ATP content in samples, an ATP standard curve is generated. Add about 10 µl ATP stock solution to about 990 µl of Lysis buffer to make about $10^{-4}$ M ATP solution, into a tube labeled S1, then make about 3 - 5 more 10 fold dilutions (i.e. about 10 µl + about 90 µl Lysis Buffer to generate S2, S3, S4, containing about $10^{-5}$M, about $10^{-6}$M, about $10^{-7}$M ATP, etc.).

**Measurement:** Add about 10 µl of sample or standard into 96-well plate. Add about 90 µl of the prepared Reaction Mix into the wells, mix then read luminescence (L). (about 10 µl of $10^{-4}$ M ATP gives about 1 nmol per well, about 10 µl of $10^{-7}$ M ATP gives about 1 pmol per well, etc.). To correct for background luminescence, first add about 90 µl Reaction Mix only, read background luminescence (BL), and then add about 10 µl sample or standard into the wells, mix, and read total luminescence (L).

**Calculations:** Correct background by subtracting BL from each L reading for samples and standards. Plot the standard curve. ATP amount in the sample wells are calculated from the standard curve using linear regression. ATP concentration in samples can be calculated using the following formula:

$$C = Sa/Sv\ (\text{pmol/µl or nmol/ml, or µM})$$

Where: Sa is sample amount (in pmol) from standard curve.
Sv is sample volume (in µl) added into the sample wells.
ATP molecular weight: 507.18 g/mol.

**d) GLUCOSE ASSAY (GOD-POD METHOD)**

About 2 µl of supernatant is taken from each well of the test plate and added to a 96 well plate. As a standard,

similarly about 2 μl of Glucose standard reagent (Conc 100mg/dl) is also added to the 96 well plate in triplicates. To these wells about 200 μl of Glucose reagent (Medsource Ozone) is added and incubated for about 10min at room temperature. Absorbances are measured at about 490nm using BioRad plate reader. Graphs are plotted and analysed using Graph Pad Prism software.

**Example 7.2: Assays for Determining Cell Death**

**e) LACTATE DEHYDROGENASE ASSAY**

Assessment of Lactose Dehydrogenase is done using LDH Cytotoxicity Assay Kit (Cayman). In the first step, LDH catalyzes the reduction of $NAD^+$ to NADH and $H^+$ by oxidation of lactate to pyruvate. In the second step of the reaction, diaphorase uses the newly-formed NADH and $H^+$ to catalyze the reduction of a tetrazolium salt (INT) to highly-colored formazan which absorbs strongly at 490-520 nm. The amount of formazan produced is proportional to the amount of LDH released into the culture medium as a result of cytotoxicity.

Plate Set Up: Each plate should contain a standard curve, wells without cells, and wells containing cells with experimental treatment or vehicle.

The 96-well tissue plates are centrifuged at about 400xg for five minutes. Using a new 96-well plate transfer about 100 μl of the standards prepared above into the appropriate wells. Transfer about 100 μl of each supernatant from each well of the cultured cells to corresponding wells on the new plate. Add about 100 μl of Reaction Solution to each well using a repeating pippettor. Incubate the plate with gentle shaking on an orbital shaker for about 30 minutes at room temperature. Read the absorbance at about 490 nm with a plate reader.

**Calculations:** The average absorbance values of the wells containing assay buffer medium only (the blanks) are substracted from the absorbance values of all the other wells. A standard Curve is plotted for absorbance at 490nm as a function of LDH concentration and the equation of the line is determined. Determination of LDH activity present in the sample is calculated using the below formula:

$$\text{LDH Activity } (\mu U) = \frac{(A_{490\text{ nm}} - \text{y-intercept})}{\text{slope}}$$

$$\text{Total LDH Activity } (\mu U/ml) \text{ in sample} = \frac{\text{Value from LDH activity assay } (\mu U)}{\text{x sample volume assayed (usually 0.1 ml)}}$$

**f) CASPASE-3 ASSAY**

The CPP32/Caspase-3 Fluorometric Protease Assay Kit (BioVision) is used for assaying the DEVD-dependent caspase activity. The assay is based on detection of cleavage of substrate DEVD-AFC (AFC: 7-amino-4-trifluoromethyl coumarin). DEVD-AFC emits blue light (λ max = 400 nm); upon cleavage of the substrate by CPP32 or related caspases, free AFC emits a yellow-green fluorescence (λmax = 505 nm), which is quantified using a fluorometer or a fluorecence microtiter plate reader. Comparison of the fluorescence of AFC from an apoptotic sample with an uninduced control allows determination of the fold increase in caspase-3/CPP32 activity.

**Assay Procedure**

1. Induce apoptosis in cells by desired method. Concurrently incubate a control culture without induction.
2. Count cells and pellet about 1-5 x about 106 cells or use about 20-200 μg cell lysates (depending on the apoptosis level).

For tissue samples, tissue is homogenized in Lysis Buffer (for 1X volume of tissue, add about 3X volume of lysis buffer) to generate tissue lysate, then follow the kit procedure. 3. Resuspend cells in about 50 μl of chilled Cell Lysis Buffer.

4. Incubate cells on ice for about 10 minutes.
5. Add about 50 μl of 2X Reaction Buffer (containing about 10 mM DTT) to each sample.
6. Add about 5 μl of about 1 mM DEVD-AFC substrate (about 50 μM final concentration) and incubate at about 37°C for about 1-2 hour.
7. Read samples in a fluorometer equipped with a 400-nm excitation filter and 505-nm emission filter. For a plate-reading set-up, transfer the samples to a 96-well plate. The entire assay can also be performed directly in a 96-well plate. Fold-increase in CPP32 activity is determined by comparing these results with the level of the uninduced control.

**g) CASPASE-8 ASSAY**

FLICE/Caspase-8 Fluorometric Assay Kit (BioVision) is used for assaying the activity of caspases that recognize the sequence IETD. The assay is based on detection of cleavage of substrate IETD-AFC (AFC: 7-amino-4-trifluoromethyl coumarin). IETD-AFC emits blue light (λ max = 400 nm); upon cleavage of the substrate by FLICE or related caspases, free AFC emits a yellow-green fluorescence (λ max = 505 nm), which is quantified using a fluorometer or a fluorecence microtiter plate reader. Comparison of the fluorescence of AFC from an apoptotic sample with an uninduced control allows determination of the fold increase in FLICE activity.

**Assay Procedure**

1. Induce apoptosis in cells by desired method. Concurrently incubate a control culture without induction.
2. Count cells and pellet about 1-5 x about $10^6$ cells or use about 50-200 μg cell lysates if protein concentration has been measured.
3. Resuspend cells in about 50 μl of chilled Cell Lysis Buffer. Incubate cells on ice for about 10 minutes.
4. Add about 50 μl of about 2X Reaction Buffer (containing about 10 mM DTT) to each sample. Add about 5 μl of about 1 mM IETD-AFC substrate (50 μM final concentration). Incubate at about 37°C for about 1-2 hours.
5. Read samples in a fluorometer equipped with a 400-nm excitation filter and 505-nm emission filter. For a plate-reading set-up, transfer the samples to a 96-well plate. The entire assay can also be performed directly in a 96-well plate. Fold-increase in FLICE activity can be determined by comparing these results with the level of the uninduced control.

**Example 7.3: Assays for Determining Cell Senescence:**

**h) SENESCENCE ASSOCIATED BETA-GAL STAINING**

In case of tissue sections, snap frozen tissue in liquid nitrogen (LN2) embedded in Optimal cutting temperature (OCT) compound is mounted onto superfrost slides. The cells are then incubated at about 37°C for about 20hr with staining solution (about 40mM citric acid sodium phosphate, pH 6.0, about 1mg/ml 5-bromo-4-chloro-3-isolyl-b-D-galactoside [X-gal, Fisher], about 5mM potassium ferricyanide, about 5mM potassium ferrocyanide, about 150mM NaCl, about 2mM $MgCl_2$). After incubation, the cells are washed twice with PBS and viewed under bright-field microscopy for blue staining.

**Example 7.4: Assays for Histological Evaluation/ IHC Assays:**

**i) IMMUNO-HISTOCHEMICAL (IHC) ANALYSIS:**

Tumor is fixed in about 10% buffered formalin and embedded in paraffin. Tumor sections are cut (about 5μm) and deparaffinised in xylene followed by rehydration in decreasing grades of ethanol. Sections are stained with Haematoxylin and Eosin (H&E). Antigen retrieval is done in Vector® Antigen Unmasking Solution (Citrate based, Vector Laboratories) by exposure to microwave heating for about 30 min. Slides are allowed to cool and subsequently washed in Tris buffered saline. Quenching of endogenous peroxidase is done by incubating the sections in about 3% $H_2O_2$ for about 15 min. Protein blocking is carried out at room temperature for about 1hr with about 10% goat serum. The subsequent incubation steps are followed by washes in Tris Buffered Saline (TBS). Sections are incubated with primary antibody at aforementioned conditions followed by incubation with horse raddish peroxidase (HRP)-conjugated secondary antibody (SignalStain® Boost IHC Detection Reagent; Cell Signaling Technology) for 1hr at RT. Chromogenic development of signal is done using 3,3'-diaminobenzidine (DAB Peroxidase Substrate Kit; Vector Laboratories). Tissues are counterstained with Hematoxylin (Papanicolaous solution 1a; Merck).

Rabbit monoclonal phospho-AKT (Ser473; D9E XPTM) and Phospho-AMPKα (Thr172) (clone 40H9, Cell Signaling Technology) is used at about 1:50 and about 1:100 dilution respectively for overnight incubation at about 4°C. Rabbit monoclonal phospho-S6 Ribosomal Protein (pS6RP) (Ser235/236; D57.2.2E XPTM) and phospho-PRAS40 (Thr246, C77D7) are obtained from Cell Signaling Technology and used at about 1:200 dilution for overnight incubation at about 4°C; rabbit polyclonal GLUT1 (Abcam) at about 1:200 dilution is used for about 1hr incubation at room temperature

(RT) ranging from about 25°C to about 35°C; rabbit polyclonal Ki67 (Vector Laboratories) is used at about 1:600 dilution for about 1hr at RT. Induction of apoptosis is detected by staining for cleaved Caspase 3 using polyclonal anti-cleaved Caspase 3 (Asp175) antibody (rabbit polyclonal, Cell Signaling Technology) at about 1:600 dilution for about 1hr at RT. Matched IgG isotype control is used for each primary antibody. Each slide is independently examined by two experts and scoring/grading is performed as per H score formula.

**j) IMMUNOHISTOCHEMISTRY STAINING OF FIXED TISSUES - PHOSPHO ERK/ PHOSPHO EGFR**

The basic principle that underpins this technique is the antigen-antibody reaction which is amplified and visualized. The target antigen may be physically inaccessible to the antibody due to protein folding caused during fixation. This is overcome by a procedure called antigen retrieval, where heat is used to alter the protein folding and the antigens become more accessible. Quenching the endogenous peroxidase, protein block and blocking of endogenous biotin are important steps to avoid background staining and nonspecific binding. This standardised protocol uses a three layered detection system that involves the primary antibody (usually rabbit/mouse mAb) which binds to the target antigen; biotinylated secondary antibody (usually goat anti-rabbit IgG) which binds the primary antibody; and the avidin biotin complex (ABC; biotinylated horseradish peroxidase that binds to avidin to form a complex) which targets the biotin linked to the secondary antibody. The antibodies help in detection of antigen and signal amplification. The peroxidase enzyme, which is present in ABC, catalyses a reaction where DAB (3,3'-diaminobenzidine) produces a brown precipitate which can be visualized under a microscope, ultimately detecting the target antigen.

**Procedure:**

1. Deparaffinization and Rehydration is carried out as provided below:

| | |
|---|---|
| a. Xylene – 2 washes – 6 min each | Deparaffinization |
| b. 100% Ethanol – 1 wash – 3min | Dexylenization |
| c. 90% Ethanol – 1 wash – 3 min | |
| d. 70% Ethanol – 1 wash – 3 min | |
| e. Tap water (running) – 10 min | Rehydration |
| f. Distilled water – 1 wash – 5 min | |

2. This is followed by antigen retrieval
a. Prepare: about 600 mL distilled water + about 5.6 mL Antigen Unmasking Solution (Vector Labs #H-3300) in a 1L beaker.
b. Soak the slides in the solution for about 10 minutes.
c. Microwave the contents of the beaker and the slides as following:

| | |
|---|---|
| M-Low | : about 5 min |
| Medium | : about 5 min |
| M-High | : about 5 min |
| High | : about 5 min |

d. Cool the slides to room temperature by placing the beaker in a tap water filled bath.
e. Wash the slides with distilled water about 4 times for about 5 min each wash (Coplin Jar).
f. Wash slides in 1X PBS for 5 min (Coplin Jar).
3. Quenching of endogenous peroxidaseis done

a. Fresh Preparation: about 9 mL $H_2O_2$ (30%) + about 75 mL Distilled water
b. Incubate slides in $H_2O_2$ solution for about 15 min (Coplin Jar).
c. Wash slides in running tap water for about 2 min.
d. Wash slides in 1X PBS for about 7 min (Coplin Jar).
e. Circle tissue using hydrophobic Pap pen (this is done to keep the volume of antibodies as small as possible).

4. Protein blocking is done. This and the subsequent steps require a humidified chamber (a tray with wet whatman filter paper).

a. Prepare about 10% Goat serum (250 μL goat serum + 2.5 mL 1X PBS)
b. Add about 75 μL goat serum to each tissue section and incubate for about 1 hour.
c. Discard the serum. No wash required.

5. Avidin/Biotin Block (obtained from Vector Labs #SP2001) is carried out

a. Dispense required amounts of Avidin and Biotin in two Eppendorf tubes.
b. Add about 75 μL of Avidin to each tissue section and incubate for about 15 min.
c. Discard Avidin and rinse the slides in 1X PBS briefly (Coplin Jar).
d. Add about 75 μL of Biotin to each tissue section and incubate for about 15 min.
e. Rinse the slides in 1X PBS briefly (Coplin Jar).

6. Primary Antibody is added

a. Prepare about 1:200 Phospho-Erk/Phospho-EGFR (obtained from Cell Signalling Technology #4370/#2237) with 1X PBS.
b. Add about 75 μL to each tissue section and incubate for about 1 hour.
c. Wash slides thrice in 1X PBS for about 3 min each wash (Coplin Jar).

7. This is followed by adding the Secondary Antibody

a. Prepare 1:1000 Goat anti-Rabbit IgG (Vector Labs #BA-1000) with 1XPBS.
b. Add about 75 μL to each tissue section and incubate for about 30 min.
c. Wash slides thrice in 1X PBS thoroughly for about 5 min each wash (Coplin Jar).

8. ABC Reagent (obtained from Vector Labs; Vectastain ABC Kit Peroxidase Goat IgG #PK-4005) is added

a. Prepare reagent: about 1 drop of solution A + about 1 drop of solution B + about 2.5 mL 1X PBS. Incubate the reagent for about 30 min prior to use at room temperature. Any extra amounts can be stored at about 4°C for up to a month.
b. Add about 75 μL of reagent to each tissue section and incubate for about 30 min.
c. Wash slides thrice in 1X PBS for about 5 min each wash (Coplin Jar).

9. DAB Substrate (Vector Labs #SK4100)
The following steps are to be done in a dark room.

a. Fresh preparation DAB substrate: about 1 drop of Buffer + about 2 drops of DAB + about 1 drop of $H_2O_2$ in about 2.5 mL double distilled water.
b. Add about 75 μL of the reagent to each tissue section and observe under microscope to decide the appropriate exposure time.
c. Discard the reagent in potassium permanganate solution and wash the slides in tap water.

10. The slides are subjected to counterstaining using Hematoxylin

a. Dip the slides 2-3 times in hematoxylin.
b. Wash the slides in running tap water for about 5 min.
c. Wash the slides in about 1% Lithium carbonate solution for about 30 seconds.
d. 70% Ethanol - 1 wash - about 3 min

e. 95% Ethanol - 1 wash - about 3 min
f. 100% Ethanol - 2 wash - about 3 min
g. Xylene - 2 washes - about 3 min

11. Mounting of the slides is done with DPX (obtained from Merck #61803502501730)

a. Mount the slides with clean cover slips using DPX and let it dry.
b. Label appropriately.

**k) TUNEL STAINING OF FIXED TISSUES**

Terminal deoxynucleotidyl transferase dUTP nick end labeling (TUNEL) is a method for detecting DNA fragmentation by labeling the terminal end of nucleic acids. TUNEL is used for detecting DNA fragmentation that results from apoptotic signaling cascades. The assay relies on the presence of nicks in the DNA which can be identified by terminal deoxynucleotidyl transferase or TdT, an enzyme that will catalyze the addition of dUTPs that are secondarily labeled with a marker. It may also label cells that have suffered severe DNA damage.

**Procedure:**

1. The first step involves deparaffinization and rehydration

| | |
|---|---|
| a. Xylene – 2 washes – 6 min each | Deparaffinization |
| b. 100% Ethanol – 1 wash – 3min<br>c. 90% Ethanol – 1 wash – 3 min<br>d. 70% Ethanol – 1 wash – 3 min | Dexylenization |
| e. Tap water (running) – 10 min<br>f. Distilled water – 1 wash – 5 min | Rehydration |

2. This is followed by antigen retrieval

a. Prepare about 1:1000 Proteinase K (Qiagen # 19131) with 1XPBS.
b. Add about 50 μL to each tissue section and incubate for 1 about 5 min.
c. Wash slides in $dH_2O$ twice for about 2 min each wash (Coplin Jar).

3. Quenching is done by endogenous peroxidase

a. Fresh Preparation: about 7.5 mL $H_2O_2$ (30%) + about 67.5 mL Distilled water
b. Incubate slides in $H_2O_2$ solution for about 5 min (Coplin Jar).
c. Wash slides in running tap water for about 2 min.
d. Wash slides in 1X PBS twice for about 5 min (Coplin Jar).
e. Circle tissue using hydrophobic Pap pen (this is done to keep the volume of antibodies/reagents as small as possible).

4. Treatment with equilibration buffer is followed. This and the subsequent steps require a humidified chamber (a tray with wet whatman filter paper).

a. Add about 13 μL equilibration buffer to each tissue section and incubate for at least about 10 seconds (upto about 60 min is alright).
b. Discard the reagent. No wash required.

5. TdT enzyme is added

a. Dilute TdT enzyme in Reaction buffer in the ratio of about 3:7 (For ex: about 30 μL TdT in about 70 μL Reaction buffer).
b. Add about 15 μL to each tissue section and incubate for about 1 hour at about 37°C in a humidified chamber.
c. Discard the reagent.

6. Stop/Wash Buffer is added

a. Prepare Stop/Wash buffer by adding about 1 mL stock buffer to about 34 mL $dH_2O$.
b. Place slides in the buffer and agitate for about 15 seconds. Incubate for about 10 min at room temperature.
d. Wash slides thrice in 1X PBS for about 1 min each wash (Coplin Jar).
e. Remove an aliquot of Anti-digoxigenin Conjugate and place at room temperature.

7. Anti-Digoxigenin Conjugate is added

a. Add 15 μL of Anti-digoxigenin conjugate to each tissue section and incubate in a humidified chamber for about 30 min at room temperature.
b. Wash slides about four times in 1X PBS for about 2 min each wash (Coplin Jar).

8. The slides are treated with peroxidase substrate: DAB

a. Prepare DAB substrate - about 1:50 dilution with DAB dilution buffer.
b. Add about 15 μL of reagent to each tissue section and incubate for about 3-6 min.

Observe under microscope to determine appropriate exposure time.

c. Wash slides thrice in $dH_2O$ for about 1 min each wash (Coplin Jar).
d. Incubate slides in $dH_2O$ for about 5 min at room temperature.

9. Counterstaining using Methyl green is done

a. Counterstain in about 0.5% methyl green for about 10 min at room temperature.
b. Wash the slides in about 3 changes of $dH_2O$ in a coplin jar, dipping the slide about 10 times each in the first and second washes, followed by about 30 seconds without agitation in the third wash.
c. Wash the slides in 3 changes of 100% N-Butanol in a coplin jar, dipping the slide about 10 times each in the first and second washes, followed by about 30 seconds without agitation in the third wash.

10. Mounting

a. Dehydrate the tissue by placing in about 2 changes of Xylene, incubating for about 2 min in each jar.
b. Mount under a glass coverslip using DPX (Merck #61803502501730).

**I) HEMATOXYLIN AND EOSIN STAIN (H&E)** is a popular staining method in histology. H&E is routinely performed to assess mitotic figures, necrosis and general gross features of the tissue. The Haemaotxylin & Eosin staining (H&E) assay is also used for determining tumor stroma content.

**Example 7.4: Assays for Cell Proliferation.**

The IHC assay is also used for the assays for standard proliferation markers like Ki67 and PCNA to determine the cell proliferation.

**Example 7.6: Assays for Nucleic acid.**

Nucleic acid isolation is further assessed in RNA and miRNA microarray analysis and gene analysis for specific mutations for select samples only. Also exome sequencing can be performed for DNA for select samples only. Genetic profiling is used in select cases for understanding biology of tumor and not as a part of "Clinical Response Predictor".

**m) PURIFICATION OF TOTAL RNA FROM TISSUES:**

Purification of total RNA from tissues is done as per the Qiagen RNA extraction kit.

1. Remove RNA later stabilized tissues from the reagent using forceps.
2. Use about 30-50 mg of tumor.
3. Place the weighed tissue in about 1.2 ml tube, Add about 350 μl of RLT buffer to the tissue, immediately homogenize in a tissue microdismembrator at about 3000 rpm for about 60 seconds.
4. Collect the lysate in a separate tube, centrifuge for about 3 min at full speed. Carefully remove the supernatant by pipetting, and transfer it to a new micro centrifuge tube.
5. Add about 1 volume (about 350 μl) of about 70% ethanol to the cleared lysate, and mix immediately by pipetting.
6. Transfer the sample to an RNeasy spin column placed in about 2 ml collection tube. Close the lid gently and centrifuge for about 15 sec at about 10000 rpm. Discard the flow through.
7. Add about 350 μl of RW1 buffer to the RNeasy spin column. Close the lid gently and centrifuge for about 15 sec at about 10000 rpm. Discard the flow through.
8. Add about 10 μl DNase 1 stock solutions to about 70 μl RDD buffer. Mix by gently inverting the tube.
9. Add about 80 μl DNase 1 incubation mix to the RNeasy spin column membrane, incubate for about 15 min at room temperature.
10. Add about 350 μl buffer RW1 to the RNeasy spin column. Close the lid gently and centrifuge for about 15 sec at about 10000 rpm. Discard the flow through
11. Add about 500 μl RPE buffer to the RNeasy spin column. Close the lid gently, and centrifuge for about 15 sec at about 10000 rpm. Discard the flow through.
12. Add about 500 μl RPE buffer to the RNeasy spin column. Close the lid gently, and centrifuge for about 2 min at about 10000 rpm. Discard the flow through.
13. Place the RNeasy spin column in a new 2ml collection tube and discard the old collection tube with the flow through. Close the lid gently and centrifuge at full speed for about 1 min
14. Place the RNeasy spin column in a new 1.5 ml collection tube. Add about 35 μl RNase free water directly to the spin column membrane Close the lid gently and centrifuge for about 1min at about 10000 rpm
15. Elute the RNA and store at about -80°c.

**n) ISOLATION OF TOTAL RNA AND MICROARRAY:**

RNA later stabilized core biopsy and corresponding human tumor xenograft samples areare lysed using micro-dismembrator (Sartorius) according to the standard operating procedure. Total RNA isolated from pulverized tissues are subsequently assessed for integrity by bio-analyzer and nanodrop.

Tumor RNA (cRNA) micro array is carried out using the Agilent Sure Print G3 Human GE 8x60K Microarrays system platform (Agilent Technologies). For RNA microarray a RIN value above about 7 is used as a cut off. Approximately about 200ng RNA extracted from tumor samples or matched control are reverse transcribed finally to generate cy3/cy5 labeled amplified cRNA and is profiled using Agilent Kits and platform (Agilent Technologies). Array data are normalized using Feature extraction software and Agilent's Gene-Spring software. Further statistical analysis is carried out using software appropriate for this study. Data expressed as fold differences (both for up-regulated and downregulated genes) compared with corresponding control. Any difference below about 1.5 fold is considered as insignificant for further validation. A heat map is generated and relationship (similarity of genes) is elucidated among different primary tumor and xenografts tumor samples based on their response status. Unsupervised array is used for generating a tree showing the relatedness of primary tumor derived from CR, PR or PD with corresponding xenografts based on functional profiling in the context of drug response. ANOVA analysis of normalized data isperformed to distinguish the differentially expressed genes (at $P < 0.05$) between and among different tumors and corresponding xenograft groups.

**o) REAL TIME RT-PCR.**

Significantly expressed genes from microarray are confirmed by RT-qPCR using specific probes and primer sets using Stratagene real time PCR platform.

**p) EXOME SEQUENCING FOR MUTATION ANALYSIS.**

Genomic DNAs are isolated from primary HNSCC tumors using DNAeasy Tissue Kit (Qiagen). Following quality check exome sequencing of the DNAs is conducted for mutation analysis as per procedures described previously. Briefly, specific sequencing primers and labeled nucleotides are to generate reaction and specific gene sequences are analyzed

in Illumina Exome Sequencing platform. Differences in the mutations spectrum in clinical responder and non responder groups are determined.

**Example 8: Generation of algorithms to predict clinical outcome:**

Once the tumor is excised from the patient, it is subjected to explant analysis as described in examples 1-3 with multiple drugs (alone or in combination). Tumor response to the drug is assessed by multiple assays as described in Example 6. In parallel, clinical outcomes are measured as per established protocols. Different weightages are given to the individual assay results of explant such that the combined score that is obtained has a linear correlation to the observed clinical outcome; i.e, high combined score (>60, for example) is correlated to complete clinical response (CR), low combined score (<20, for example) is correlated to clinical non-response (NR).

Once such a scoring system is devised, this can be used to predict the clinical response of a future patient from explant analysis.

Weightages are given to the individual parameters such that the cumulative weight-averaged data has good correlation with the observed clinical outcome. Different algorithms use different individual weightages (from 0-100%) for the parameters included in the correlation.

In addition to manually assigning weightages (as shown in the 5 example algorithms shown), "Multivariant analysis" using a computer is also possible, where different weightages are assigned to arrive at the best fit formula that has the least amount of deviation between the predicted clinical response and the observed clinical response.

The raw scores obtained by the various explant assays are provided in Table 4. It also gives the clinical read out (Complete response, Partial Response or No-Response obtained as per the conventional PERCIST criteria).

**Table 4: Experimental Data from explant assays and clinical evaluation.**

| # | Explant analysis | | | | Clinical Readout | |
|---|---|---|---|---|---|---|
| Sample ID | Viability | Histology | Proliferation | Apoptosis | RECIST | Numerical Representation |
| 1 | 5 | 20 | 0 | 120 | NR | 1 |
| 2 | 27 | 42 | 20 | 100 | PR | 2 |
| 3 | 32 | 50 | 10 | 100 | PR | 2 |
| 4 | 58 | 60 | 53 | 154 | CR | 3 |
| 5 | 22 | 0 | 0 | 150 | NR | 1 |
| 6 | 25 | 50 | 50 | 70 | PR | 2 |
| 7 | 21 | 47 | 55 | 80 | PR | 2 |
| 8 | 48 | 72 | 43 | 120 | CR | 3 |
| 9 | 9 | 0 | 0 | 100 | NR | 1 |
| 10 | 24 | 38 | 27 | 120 | PR | 2 |
| 11 | 19 | 43 | 55 | 80 | PR | 2 |
| 12 | 27 | 62 | 20 | 75 | PR | 2 |
| 13 | 16 | 55 | 20 | 68 | PR | 2 |
| 14 | 32 | 42 | 35 | 70 | PR | 2 |
| 15 | 35 | 33 | 60 | 65 | PR | 2 |
| 16 | 27 | 28 | 28 | 72 | PR | 2 |
| 17 | 29 | 36 | 25 | 80 | PR | 2 |
| 18 | 23 | 50 | 75 | 75 | PR | 2 |
| 19 | 19 | 0 | 0 | 100 | NR | 1 |
| 20 | 22 | 44 | 62 | 50 | PR | 2 |
| 21 | 27 | 51 | 25 | 65 | PR | 2 |
| 22 | 25 | 0 | 0 | 100 | NR | 1 |

(continued)

| # | Explant analysis | | | | Clinical Readout | |
|---|---|---|---|---|---|---|
| Sample ID | Viability | Histology | Proliferation | Apoptosis | RECIST | Numerical Representation |
| 23 | 35 | 34 | 42 | 25 | PR | 2 |
| 24 | 20 | 19 | 35 | 55 | PR | 2 |
| 25 | 5 | 0 | 0 | 25 | NR | 1 |
| 26 | 27 | 0 | 0 | 42 | NR | 1 |
| 27 | 8 | 0 | 0 | 50 | NR | 1 |
| 28 | 35 | 50 | 36 | 50 | PR | 2 |
| 29 | 18 | 0 | 36 | 18 | NR | 1 |
| 30 | 36 | 48 | 58 | 30 | PR | 2 |
| 31 | 29 | 14 | 20 | 55 | PR | 2 |
| 32 | 32 | 34 | 16 | 20 | PR | 2 |
| 33 | 44 | 28 | 35 | 30 | PR | 2 |
| 34 | 24 | 12 | 0 | 50 | NR | 1 |
| 35 | 52 | 44 | 0 | 20 | PR | 2 |
| 36 | 29 | 10 | 22 | 42 | PR | 2 |
| 37 | 21 | 9 | 46 | 0 | PR | 2 |
| 38 | 66 | 55 | 40 | 74 | CR | 3 |
| 39 | 41 | 12 | 0 | 20 | NR | 1 |
| 40 | 11 | 17 | 27 | 54 | PR | 2 |
| 41 | 15 | 22 | 33 | 35 | PR | 2 |
| 42 | 7 | 0 | 0 | 30 | NR | 1 |
| 43 | 15 | 15 | 28 | 36 | PR | 2 |
| 44 | 62 | 50 | 64 | 70 | CR | 3 |
| 45 | 36 | 41 | 43 | 24 | PR | 2 |
| 46 | 24 | 52 | 36 | 45 | PR | 2 |

Table 5 gives numerical value for the observed clinical read-out. Value of 3, 2 and 1 are given for complete response, partial response and non- response respectively. Table 6 shows the weightages that are given for the explant assays in each of the 5 representative algorithms.

These weightages are given based on the nature of the drugs used in the explants analysis. For instance, drugs that are known to exhibit their activity by disrupting cell proliferation are given higher weightages for cell proliferation.

**Table 5: Numerical representation of clinical response, partial response and non-response.**

| | |
|---|---|
| Complete Response | 3 |
| Partial Response | 2 |
| No Response | 1 |

**Table 6: Weightage given to different explant assay results in the 5 representative algorithms.**

| Sensitivity Index Weightage (%) | | | | |
|---|---|---|---|---|
| | **Viability** | **Histology** | **Proliferation** | **Apoptosis** |
| Method 1 | 25% | 25% | 25% | 25% |
| Method 2 | 20% | 30% | 25% | 25% |
| Method 3 | 30% | 15% | 25% | 30% |
| Method 4 | 30% | 30% | 30% | 10% |
| Method 5 | 10% | 20% | 30% | 40% |

Sensitivity index (i.e. the M-score) for each patient is calculated by multiplying the raw score with the corresponding weightage factor and adding the resulting numbers, as illustrated in Table 7. For example, patient 1 has raw explant assay score of 5, 20, 0 and 120 for viability, histology, proliferation, and apoptosis respectively. Under algorithm 1 (or method 1), each of these factors is given a weightage of 25%. Thus sensitivity index for patient 1 using algorithm 1 will be calculated as follows:

$$\text{Sensitivity index} = (5*25\%) + (20*25\%) + (0*25\%) + (120*25\%) = 36$$

The Sensitivity Index thus calculated is converted into predicted clinical outcome (Table 8-12) as follows:

If the Sensitivity Index > 60, Predicted clinical outcome = 3 (= Complete response).
If 20 <Sensitivity Index <60, predicted clinical outcome = 2 (= Partial response).
If Sensitivity index < 20, predicted clinical outcome = 1 (= No Response).

**Table 7: Sensitivity index measured by the application of the weightages for the explant assays as measured by the 5 representative algorithms.**

| Sensitivity Index | | | | |
|---|---|---|---|---|
| **Method 1** | **Method 2** | **Method 3** | **Method 4** | **Method 5** |
| 36 | 37 | 41 | 20 | 53 |
| 47 | 48 | 49 | 37 | 57 |
| 48 | 49 | 50 | 38 | 56 |
| 81 | 81 | 86 | 67 | 95 |
| 43 | 42 | 52 | 22 | 62 |
| 49 | 50 | 49 | 45 | 56 |
| 51 | 52 | 51 | 45 | 60 |
| 71 | 72 | 72 | 61 | 80 |
| 27 | 27 | 33 | 13 | 41 |
| 52 | 53 | 56 | 39 | 66 |
| 49 | 50 | 50 | 43 | 59 |
| 46 | 48 | 45 | 40 | 51 |
| 40 | 42 | 38 | 34 | 46 |
| 45 | 45 | 46 | 40 | 50 |
| 48 | 48 | 50 | 45 | 54 |
| 39 | 39 | 41 | 32 | 46 |

(continued)

| Sensitivity Index | | | | |
|---|---|---|---|---|
| Method 1 | Method 2 | Method 3 | Method 4 | Method 5 |
| 43 | 43 | 44 | 35 | 50 |
| 56 | 57 | 56 | 52 | 65 |
| 30 | 29 | 36 | 16 | 42 |
| 45 | 46 | 44 | 43 | 50 |
| 42 | 43 | 42 | 37 | 46 |
| 31 | 30 | 38 | 18 | 43 |
| 34 | 34 | 34 | 36 | 33 |
| 32 | 32 | 34 | 28 | 38 |
| 8 | 7 | 9 | 4 | 11 |
| 17 | 16 | 21 | 12 | 20 |
| 15 | 14 | 17 | 7 | 21 |
| 43 | 44 | 42 | 41 | 44 |
| 18 | 17 | 20 | 18 | 20 |
| 43 | 44 | 42 | 46 | 43 |
| 30 | 29 | 32 | 24 | 34 |
| 26 | 26 | 25 | 27 | 23 |
| 34 | 33 | 35 | 35 | 33 |
| 22 | 21 | 24 | 16 | 25 |
| 29 | 29 | 28 | 31 | 22 |
| 26 | 25 | 28 | 23 | 28 |
| 19 | 18 | 19 | 23 | 18 |
| 59 | 58 | 60 | 56 | 59 |
| 18 | 17 | 20 | 18 | 15 |
| 27 | 28 | 29 | 22 | 34 |
| 26 | 27 | 27 | 25 | 30 |
| 9 | 9 | 11 | 5 | 13 |
| 24 | 24 | 25 | 21 | 27 |
| 62 | 61 | 63 | 60 | 63 |
| 36 | 36 | 35 | 38 | 34 |
| 39 | 41 | 38 | 38 | 42 |

Predicted clinical outcome is compared with observed clinical outcome to measure predictive power of the algorithms (Tables 8-12). The shaded portions depict cases where there is match between the predicted outcome and observed outcome.

**Table 8: Predictive Efficiency of algorithm 1:** Comparison between "predicted clinical outcome" and "Observed Clinical outcome". If Sensitivity index >60, Predicted clinical outcome is given a score of 3 (i.e., Complete Response). If Sensitivity index < 20, Predicted clinical outcome is given a score of 1 (i.e., Non Response). If 20 < Sensitivity index < 60, Predicted clinical outcome is given a score of 2 (i.e., Partial Response).

Algorithm 1

| Sensitivity Index | Predicted Clinical Outcome | Observed Clinical Outcome |
|---|---|---|
| 36 | 2 | 1 |
| 47 | 2 | 2 |
| 48 | 2 | 2 |
| 81 | 3 | 3 |
| 43 | 2 | 1 |
| 49 | 2 | 2 |
| 51 | 2 | 2 |
| 71 | 3 | 3 |

| | | |
|---|---|---|
| 27 | 2 | 1 |
| 52 | 2 | 2 |
| 49 | 2 | 2 |
| 46 | 2 | 2 |
| 40 | 2 | 2 |
| 45 | 2 | 2 |
| 48 | 2 | 2 |
| 39 | 2 | 2 |
| 43 | 2 | 2 |
| 56 | 2 | 2 |
| 30 | 2 | 1 |
| 45 | 2 | 2 |
| 42 | 2 | 2 |
| 31 | 2 | 1 |
| 34 | 2 | 2 |
| 32 | 2 | 2 |
| 8 | 1 | 1 |
| 17 | 1 | 1 |
| 15 | 1 | 1 |
| 43 | 2 | 2 |
| 18 | 1 | 1 |
| 43 | 2 | 2 |
| 30 | 2 | 2 |
| 26 | 2 | 2 |
| 34 | 2 | 2 |
| 22 | 2 | 1 |
| 29 | 2 | 2 |
| 26 | 2 | 2 |
| 19 | 1 | 2 |
| 59 | 2 | 3 |
| 18 | 1 | 1 |
| 27 | 2 | 2 |
| 26 | 2 | 2 |
| 9 | 1 | 1 |
| 24 | 2 | 2 |
| 62 | 3 | 3 |
| 36 | 2 | 2 |
| 39 | 2 | 2 |

**Table 9: Predictive Efficiency of algorithm 2-** Comparison between "predicted clinical outcome" and "Observed Clinical outcome". If Sensitivity index >60, Predicted clinical outcome is given a score of 3 (i.e., Complete Response). If Sensitivity index < 20,

Predicted clinical outcome is given a score of 1 (i.e., Non Response). If 20 < Sensitivity index < 60, Predicted clinical outcome is given a score of 2 (i.e., Partial Response).

Algorithm 2

| Sensitivity Index | Predicted Clinical Outcome | Clinical Outcome |
|---|---|---|
| 37 | 2 | 1 |
| 48 | 2 | 2 |
| 49 | 2 | 2 |
| 81 | 3 | 3 |
| 42 | 2 | 1 |
| 50 | 2 | 2 |
| 52 | 2 | 2 |
| 72 | 3 | 3 |
| 27 | 2 | 1 |
| 53 | 2 | 2 |
| 50 | 2 | 2 |
| 48 | 2 | 2 |
| 42 | 2 | 2 |
| 45 | 2 | 2 |
| 48 | 2 | 2 |
| 39 | 2 | 2 |
| 43 | 2 | 2 |
| 57 | 2 | 2 |
| 29 | 2 | 1 |
| 46 | 2 | 2 |
| 43 | 2 | 2 |
| 30 | 2 | 1 |
| 34 | 2 | 2 |
| 32 | 2 | 2 |
| 7 | 1 | 1 |
| 16 | 1 | 1 |
| 14 | 1 | 1 |
| 44 | 2 | 2 |
| 17 | 1 | 1 |
| 44 | 2 | 2 |
| 29 | 2 | 2 |
| 26 | 2 | 2 |
| 33 | 2 | 2 |
| 21 | 2 | 1 |
| 29 | 2 | 2 |
| 25 | 2 | 2 |
| 18 | 1 | 2 |

| | | |
|---|---|---|
| 58 | 2 | 3 |
| 17 | 1 | 1 |
| 28 | 2 | 2 |
| 27 | 2 | 2 |
| 9 | 1 | 1 |
| 24 | 2 | 2 |
| 61 | 3 | 3 |
| 36 | 2 | 2 |
| 41 | 2 | 2 |

**Table 10: Predictive Efficiency of algorithm 3:** Comparison between "predicted clinical outcome" and "Observed Clinical outcome". If Sensitivity index >60, Predicted clinical outcome is given a score of 3 (i.e., Complete Response). If Sensitivity index < 20, Predicted clinical outcome is given a score of 1 (i.e., Non Response). If 20 < Sensitivity index < 60, Predicted clinical outcome is given a score of 2 (i.e., Partial Response).

Algorithm 3

| Sensitivity Index | Predicted Clinical Outcome | Clinical Outcome |
|---|---|---|
| 41 | 2 | 1 |
| 49 | 2 | 2 |
| 50 | 2 | 2 |
| 86 | 3 | 3 |
| 52 | 2 | 1 |
| 49 | 2 | 2 |
| 51 | 2 | 2 |
| 72 | 3 | 3 |
| 33 | 2 | 1 |
| 56 | 2 | 2 |
| 50 | 2 | 2 |
| 45 | 2 | 2 |
| 38 | 2 | 2 |
| 46 | 2 | 2 |
| 50 | 2 | 2 |
| 41 | 2 | 2 |
| 44 | 2 | 2 |
| 56 | 2 | 2 |
| 36 | 2 | 1 |
| 44 | 2 | 2 |
| 42 | 2 | 2 |
| 38 | 2 | 1 |
| 34 | 2 | 2 |

| | | |
|---|---|---|
| 34 | 2 | 2 |
| 9 | 1 | 1 |
| 21 | 1 | 1 |
| 17 | 1 | 1 |
| 42 | 2 | 2 |
| 20 | 1 | 1 |
| 42 | 2 | 2 |
| 32 | 2 | 2 |
| 25 | 2 | 2 |
| 35 | 2 | 2 |
| 24 | 2 | 1 |
| 28 | 2 | 2 |
| 28 | 2 | 2 |
| 19 | 1 | 2 |
| 60 | 3 | 3 |
| 20 | 2 | 1 |
| 29 | 2 | 2 |
| 27 | 2 | 2 |
| 11 | 1 | 1 |
| 25 | 2 | 2 |
| 63 | 3 | 3 |
| 35 | 2 | 2 |
| 38 | 2 | 2 |

**Table 11: Predictive Efficiency of algorithm 4:** Comparison between "predicted clinical outcome" and "Observed Clinical outcome". If Sensitivity index >60, Predicted clinical outcome is given a score of 3 (i.e., Complete Response). If Sensitivity index < 20, Predicted clinical outcome is given a score of 1 (i.e., Non Response). If 20 < Sensitivity index < 60, Predicted clinical outcome is given a score of 2 (i.e., Partial Response).

Algorithm 4

| Sensitivity Index | Predicted Clinical Outcome | Clinical Outcome |
|---|---|---|
| 20 | 2 | 1 |
| 37 | 2 | 2 |
| 38 | 2 | 2 |
| 67 | 3 | 3 |
| 22 | 2 | 1 |
| 45 | 2 | 2 |
| 45 | 2 | 2 |
| 61 | 3 | 3 |
| 13 | 2 | 1 |

| | | |
|---|---|---|
| 39 | 2 | 2 |
| 43 | 2 | 2 |
| 40 | 2 | 2 |
| 34 | 2 | 2 |
| 40 | 2 | 2 |
| 45 | 2 | 2 |
| 32 | 2 | 2 |
| 35 | 2 | 2 |
| 52 | 2 | 2 |
| 16 | 2 | 1 |
| 43 | 2 | 2 |
| 37 | 2 | 2 |
| 18 | 2 | 1 |
| 36 | 2 | 2 |
| 28 | 2 | 2 |
| 4 | 1 | 1 |
| 12 | 1 | 1 |
| 7 | 1 | 1 |
| 41 | 2 | 2 |
| 18 | 1 | 1 |
| 46 | 2 | 2 |
| 24 | 2 | 2 |
| 27 | 2 | 2 |
| 35 | 2 | 2 |
| 16 | 2 | 1 |
| 31 | 2 | 2 |
| 23 | 2 | 2 |
| 23 | 1 | 2 |
| 56 | 2 | 3 |
| 18 | 1 | 1 |
| 22 | 2 | 2 |
| 25 | 2 | 2 |
| 5 | 1 | 1 |
| 21 | 2 | 2 |
| 60 | 2 | 3 |
| 38 | 2 | 2 |
| 38 | 2 | 2 |

**Table 12: Predictive Efficiency of algorithm 5:** Comparison between "predicted clinical outcome" and "Observed Clinical outcome". If Sensitivity index >60, Predicted clinical outcome is given a score of 3 (i.e., Complete Response). If Sensitivity index < 20,

Predicted clinical outcome is given a score of 1 (i.e., Non Response). If 20 < Sensitivity index < 60, Predicted clinical outcome is given a score of 2 (i.e., Partial Response).

**Algorithm 5**

| Sensitivity Index | Predicted Clinical Outcome | Clinical Outcome |
|---|---|---|
| 53 | 2 | 1 |
| 57 | 2 | 2 |
| 56 | 2 | 2 |
| 95 | 3 | 3 |
| 62 | 3 | 1 |
| 56 | 2 | 2 |
| 60 | 2 | 2 |
| 80 | 3 | 3 |
| 41 | 2 | 1 |
| 66 | 3 | 2 |
| 59 | 2 | 2 |
| 51 | 2 | 2 |
| 46 | 2 | 2 |
| 50 | 2 | 2 |
| 54 | 2 | 2 |
| 46 | 2 | 2 |
| 50 | 2 | 2 |
| 65 | 3 | 2 |
| 42 | 2 | 1 |
| 50 | 2 | 2 |
| 46 | 2 | 2 |
| 43 | 2 | 1 |
| 33 | 2 | 2 |
| 38 | 2 | 2 |
| 11 | 1 | 1 |
| 20 | 1 | 1 |
| 21 | 1 | 1 |
| 44 | 2 | 2 |
| 20 | 1 | 1 |
| 43 | 2 | 2 |
| 34 | 2 | 2 |
| 23 | 2 | 2 |
| 33 | 2 | 2 |
| 25 | 2 | 1 |
| 22 | 2 | 2 |
| 28 | 2 | 2 |

| | | |
|---|---|---|
| 18 | 1 | 2 |
| 59 | 2 | 3 |
| 15 | 1 | 1 |
| 34 | 2 | 2 |
| 30 | 2 | 2 |
| 13 | 1 | 1 |
| 27 | 2 | 2 |
| 63 | 3 | 3 |
| 34 | 2 | 2 |
| 42 | 2 | 2 |

Instead of giving manual weightages in Tables 6-13, a computer can be used optionally to use a multi-regression analysis method to give such weightages to the individual explant assays. In such cases, the computer will come with a polynomial fit (linear, quadriatic or higher order equation) using the observed explant data and come up with a predicted clinical outcome that has the least deviation to the observed clinical outcome.

**Example 9: Use of Explant System in Multiple Solid Cancers To Generate Response Prediction**

"Clinical Response Predictor" driven functional assay enables rapid screening of a panel of anticancer agents. A panel of established and investigational anticancer agents (both cytotoxic and targeted) is selected primarily based on their known tumor growth inhibition properties. The ex vivo efficacy of these drugs is tested for a panel of patient derived explants in 72 hours proliferation (Ki-67) and viability assay (WST). Percent inhibition is determined with reference to untreated control. The results are illustrated in Figure 11. Inhibition above 50% is considered as complete response. Inhibition below 50% but above 20% is considered as partial response. In non response groups drugs that exhibit no (0% to 20% inhibition) is considered as no response and similar to stable diseases. Drugs that show increase in cell proliferation for a particular indication is considered as progressive diseases. The results obtained indicate that the "Clinical Response Predictor" mimics tumor xenograft sample. Hence, it is further validated using clinical outcomes.

**Example 10:**

Tumor samples are collected from patients along with their serum as per standard protocols. The patients had either PETCT or CT evaluation prior to start of "Clinical Response Predictor" explant analysis. The collected samples are processed for Clinical Response Predictor explant analysis.

About 3x3x3 mm small pieces of tumor slice are generated. Tumor samples are divided into multiple small pieces using Leica Vibratome to generate about 100-300 μm sections and cultured in triplicate in 96 well flat bottom plates that have been previously coated with cancer specific ECM as indicated in Example 1. Tumor tissues are maintained in conditioned media of about 2ml (DMEM supplanted with about 2% heat inactivated FBS along with 1% Penicillin-Streptomycin, sodium pyruvate 100mM, nonessential amino acid, L-glutamine 4mM and HEPES 10mM. The culture media is supplemented with about 2% serum derived ligands after 12 hours. The drugs are optionally added at the start of culture either along with media or separately. The media is changed at the time of serum addition. The media is also changed every 24 hours along with supplements. About 5μl of spent media is used to determine cell viability, cell proliferation, histology, and cell death of tumor tissue. At the end of culture period ranging from about 72 hours, the tissue is assesed for the parameters. Post this period MTT/WST analysis is performed to assess percent cell viability. The supernatant from the media culture is removed every 24 hrs and assessed for proliferation (using ATP and glucose utilization experiments) and cell death (by assessment of lactate dehydrogenase assays and caspase-3 and caspase 8 measurements) to give kinetic response trends. Results are quantified against a drug untreated control. The tissue sections both treated with drug(s) and untreated are also given for IHC and histological evaluation at the end of the culture period. The tissues given for histological evaluation are assessed for apoptosis by TUNEL and activated caspase 3 assay. Also cell proliferation is assayed for standard proliferation markers like Ki67 and PCNA.

All preclinical outcomes, such as cell viability, cell death by apoptosis, histological evaluation and also proliferation status are finally integrated to give a single score called Sensitivity Index (or M-score), depicted in Table 3 provided below.

The patients enrolled for the instant explant analysis also had clinical treatment and were evaluated for response

at the end of 6-8 months by either PETCT or CT. The "Clinical Response Predictor" outcome (M-score) is then compared with clinical outcome. The results obtained are illustrated in the below Table 3.

The Table 3 indicates the type of tumor sample obtained from the respective patients (having one of the following types of cancer- HNSCC, Glioblastoma, Ca-Ovary, Ca-Breast, Ca-Oesophagus, CRC, Ca-Pancreas, Ca-Stomach,) and the drug or combinations of drug the patient is treated with, for both analysis *via* "Clinical Response Predictor" and clinical treatment.

As evident from the results obtained in the below table, the "Clinical Response Predictor" has successfully predicted the clinical outcome with an efficiency of about 100% for non-responders and about 88% for responders.

Tumor samples of Patient 1 having Head and Neck cancer are treated with a combination of Cisplatin+5FU+Docetaxel by the "Clinical Response Predictor". The preclinical outcomes obtained by tissue analysis through cell viability, histological evaluation, cell proliferation and cell death by apoptosis are integrated to give a Sensitivity Index (or M-score) of 8. Since the Sensitivity index of the preclinical treatment in Patient 1 is <20; the treatment is predicted to have poor clinical outcome when the same combination of drugs is administered to the patient. This is validated from the results of the RECIST data obtained for the clinical response where the patient is given a score of 1, indicating clinical non-response.

Tumor samples of Patient 3 having Head and Neck cancer are treated with a combination of Carboplatin and Paclitaxel by the "Clinical Response Predictor". The preclinical outcomes obtained by tissue analysis through cell viability, histological evaluation, cell proliferation and cell death by apoptosis are integrated to give a Sensitivity Index (or M-score) of 47. Since the Sensitivity index of the preclinical treatment in Patient 3 is >20 but <60; the treatment is predicted to have partial clinical outcome when the same combination of drugs is administered to the patient. This is validated from the results of the RECIST data obtained for the clinical response where the patient is given a score of 2, indicating partial response.

Tumor samples of Patient 38 having Head and Neck cancer are treated with a combination of Cisplatin, 5FU and Docetaxel by the "Clinical Response Predictor". The preclinical outcomes obtained by tissue analysis through cell viability, histological evaluation, cell proliferation and cell death by apoptosis are integrated to give a Sensitivity Index (or M-score) of 90. Since the Sensitivity index of the preclinical treatment in Patient 38 is >60; the treatment is predicted to have complete clinical outcome when the same combination of drugs is administered to the patient. This is validated from the results of the RECIST data obtained for the clinical response where the patient is given a score of 3, indicating complete clinical response.

**Table 3**

| | | | Clinical Response Predictor | | | | | Clinical readout |
|---|---|---|---|---|---|---|---|---|
| Sample ID | Cancer Type | Treatment | Viability | Histology | Proliferation | Apoptosis | Sensitivity Index | RECIST |
| 1 | HNSCC | Cisplatin+5FU+Docetaxel | 5 | 20 | -100 | 120 | 8 | 1 |
| 2 | Glioblastoma | Temozolomide | 27 | 42 | 20 | 100 | 49 | 2 |
| 3 | HNSCC | Carboplatin+Paclitaxel | 32 | 50 | 10 | 100 | 47 | 2 |
| 4 | Ca-Ovary | | 58 | 60 | 53 | 154 | 88 | 3 |
| 5 | Ca-Breast | Capecitabine+Lapatinib | 22 | -20 | -125 | 150 | 16 | 1 |
| 6 | Ca-Oesophagus | 5FU+Leucovorin | 25 | 50 | 50 | 70 | 48 | 2 |
| 7 | HNSCC | Cetuximab | 21 | 47 | 55 | 80 | 52 | 2 |
| 8 | Ca-Breast | | 48 | 72 | 43 | 120 | 70 | 3 |
| 9 | Ca-Oesophagus | | 9 | -68 | -80 | 100 | 10 | 1 |
| 10 | CRC | Irrinotecan+5FU | 24 | 38 | 27 | 120 | 57 | 2 |
| 11 | Ca-Pancreas | Gemcitabine+Cisplatin | 19 | 43 | 55 | 80 | 51 | 2 |
| 12 | Ca-Pancreas | Gemcitabine+Erlotinib | 27 | 62 | 20 | 75 | 41 | 2 |
| 13 | Ca-Oesophagus | 5FU+Leucovorin | 16 | 55 | 20 | 68 | 35 | 2 |
| 14 | HNSCC | Carboplatin+Paclitaxel | 32 | 42 | 35 | 70 | 46 | 2 |
| 15 | Ca-Oesophagus | Epirubicin+Cisplatin +Capecitabine | 35 | 33 | 60 | 65 | 53 | 2 |
| 16 | Ca-Pancreas | Gemcitabine+Cisplatin | 27 | 28 | 28 | 72 | 42 | 2 |
| 17 | CRC | Cetuximab | 29 | 36 | 25 | 80 | 45 | 2 |
| 18 | Ca-Breast | Vinorelbine | 23 | 50 | 75 | 75 | 58 | 2 |
| 19 | Ca-Pancreas | Gemcitabine+Cisplatin | 19 | -48 | -50 | 100 | 35 | 1 |
| 20 | Ca-Pancreas | 5FU+Leucovorin | 22 | 44 | 62 | 50 | 67 | 2 |
| 21 | Ca-Pancreas | Gemcitabine+Erlotinib | 27 | 51 | 25 | 65 | 59 | 2 |
| 22 | Ca-Oesophagus | Epirubicin+Cisplatin +Capecitabine | 25 | -35 | -70 | 100 | 28 | 1 |
| 23 | Ca-Breast | Herceptin | 35 | 34 | 42 | 25 | 51 | 2 |
| 24 | Ca-Ovary | Bleomycin+Cisplati n+Etoposide | 20 | 19 | 35 | 55 | 55 | 2 |

(continued)

| Sample ID | Cancer Type | Treatment | Clinical Response Predictor | | | | Sensitivity Index | Clinical readout |
|---|---|---|---|---|---|---|---|---|
| | | | Viability | Histology | Proliferation | Apoptosis | | RECIST |
| 25 | Ca-Breast | Cisplatin+5FU+Docetaxel | 5 | -22 | -18 | 25 | 6 | 1 |
| 26 | HNSCC | Cetuximab | 27 | -46 | -35 | 42 | 17 | 1 |
| 27 | HNSCC | Carboplatin+Paclitaxel | 8 | -58 | -10 | 50 | 24 | 1 |
| 28 | CRC | Irrinotecan+5FU | 35 | 50 | 36 | 50 | 61 | 2 |
| 29 | Ca-Ovary | Carboplatin+Paclitaxel | 18 | -62 | 36 | 18 | 36 | 1 |
| 30 | Ca-Stomach | Epirubicin+Cisplatin +Capecitabine | 36 | 48 | 58 | 30 | 62 | 2 |
| 31 | HNSCC | Cisplatin+5FU+Doc etaxel | 29 | 14 | 20 | 55 | 52 | 2 |
| | | | | | | | | |
| 32 | HNSCC | Carboplatin+Paclitaxel | 32 | 34 | 16 | 20 | 34 | 2 |
| 33 | Ca-Stomach | 5FU+Leucovorin | 44 | 28 | 35 | 30 | 55 | 2 |
| 34 | Ca-Breast | Cyclophosphamide +Doxorubicin+Paclitaxel | 24 | 12 | -20 | 50 | 27 | 1 |
| 35 | Ca-Oesophagus | 5FU+Leucovorin | 52 | 44 | 0 | 20 | 36 | 2 |
| 36 | Ca-Stomach | Cisplatin+5FU+Docetaxel | 29 | 10 | 22 | 42 | 47 | 2 |
| 37 | HNSCC | Carboplatin+Paclitaxel | 21 | 9 | 46 | 0 | 34 | 2 |
| 38 | HNSCC | Cisplatin+5FU+Docetaxel | 66 | 55 | 40 | 74 | 90 | 3 |
| 39 | Ca-Ovary | Carboplatin+Paclita xel | 41 | 12 | -10 | 20 | 26 | 1 |
| 40 | Ca-Stomach | Epirubicin+Cisplatin +Capecitabine | 11 | 17 | 27 | 54 | 46 | 2 |
| 41 | HNSCC | Cetuximab | 15 | 22 | 33 | 35 | 42 | 2 |
| 42 | Ca-Oesophagus | Cisplatin+5FU+Docetaxel | 7 | -16 | -15 | 30 | 11 | 1 |
| 43 | Ca-Oesophagus | Cisplatin+5FU+Docetaxel | 15 | 15 | 28 | 36 | 40 | 2 |
| 44 | Ca-Breast | Cyclophosphamide +Doxorubicin+Paclitaxel | 62 | 50 | 64 | 70 | 98 | 3 |
| 45 | Ca-Oesophagus | Epirubicin+Cisplatin +Capecitabine | 36 | 41 | 43 | 24 | · ·A | 2 |
| 46 | CRC | Irrinotecan+5FU | 24 | 52 | 36 | 45 | 53 | 2 |

**Example 11: "Clinical Response Predictor" Is a Better Response Predictor than Biomarkers**

As mentioned above, though biomarkers are used in the prior art as prediction tool, there are many constraints associated with the same. These constraints are overcome by the tools and methods of the present disclosure. "Clinical Response Predictor" is not limited to the drugs or the disease that has been used for the initial validation. "Clinical Response Predictor" is a platform technology. For example, once "Clinical Response Predictor" has been developed for a Colorectal cancer model for a particular drug, say 5-FU and has been shown that this model is useful in predicting the efficacy of 5-FU, the model is portable for other drugs. This is because the input constraints for "Clinical Response Predictor" are linked to the patient under consideration (or the patient derived tumor) and not the drug.

Another big difference is the difference between "driver" and "passenger" biomarkers. For many targeted drugs, patients are segregated based on whether they have a particular biomarker or not. However, presence of a given biomarker does not ascertain whether the patients will or will not respond the drug. This is because of the heterogeneous nature of cancer where multiple factors are responsible for affecting the efficacy of the drugs. In contrast, because "Clinical Response Predictor" is an unbiased approach and takes the tumor tissue as a whole in deciding whether the patient will respond to the drug, this is more relevant to determining the actual clinical outcome.

Although biomarkers (gene/protein that are differentially expressed in responders vs. non-responders to a particular drug) are available for a handful of drugs such as Herceptin (Her2 biomarker), they are not available for a wide variety of other drugs. Where available, they have low correlation to clinical outcome. E.g.: KRAS, the biomarker approved for Erbitux in Colorectal cancer has a predictive power in the range of 10-30%. This aspect of biomarkers has been illustrated in Table 13.

**Table 13: All non-responders are marked as NR and responders are marked as R.**

| Patient ID# | Clinical Response | "Clinical Response Predictor" Response | M-score | KRAS | BRAF | PIK3CA | AREG | EREG |
|---|---|---|---|---|---|---|---|---|
| 1 | R | R | 62 | WT | WT | WT | Low | High |
| 2 | NR | NR | 18 | Mut | WT | WT | High | High |
| 3 | NR | NR | 14 | WT | Mut | WT | Low | Low |
| 4 | NR | NR | 2 | WT | WT | WT | Low | Low |
| 5 | NR | NR | 5 | WT | WT | WT | Low | Low |
| 6 | NR | NR | 19 | WT | WT | Mut | Low | Low |
| 7 | NR | NR | 22 | WT | WT | WT | High | High |
| 8 | NR | NR | 11 | Mut | WT | WT | Low | Low |
| 9 | NR | NR | 18 | WT | WT | WT | Low | Low |
| 10 | NR | NR | 19 | WT | WT | Mut | Low | Low |
| 11 | NR | NR | 4 | WT | WT | WT | Low | Low |
| 12 | NR | NR | 1 | Mut | WT | WT | Low | Low |
| 13 | R | R | 72 | WT | WT | WT | High | High |
| 14 | R | R | 54 | WT | WT | WT | Low | Low |
| 15 | NR | NR | 12 | WT | Mut | WT | Low | Low |
| 16 | NR | NR | 21 | Mut | WT | WT | High | High |
| 17 | NR | NR | 16 | WT | WT | WT | Low | Low |
| 18 | NR | NR | 25 | WT | WT | WT | Low | Low |
| 19 | NR | NR | 20 | WT | WT | WT | Low | Low |
| 20 | R | R | 67 | WT | WT | WT | High | High |
| 21 | NR | NR | 11 | Mut | WT | WT | Low | Low |
| 22 | NR | NR | 3 | WT | WT | WT | Low | Low |
| 23 | NR | NR | 20 | WT | WT | WT | High | High |

(continued)

| Patient ID# | Clinical Response | "Clinical Response Predictor" Response | M-score | KRAS | BRAF | PIK3CA | AREG | EREG |
|---|---|---|---|---|---|---|---|---|
| 24 | R | R | 66 | WT | WT | WT | High | Low |
| 25 | NR | NR | 12 | Mut | WT | WT | High | High |
| 26 | NR | NR | 3 | WT | WT | WT | High | High |
| 27 | NR | NR | 9 | WT | WT | WT | High | Low |
| 28 | NR | NR | 17 | WT | WT | WT | High | High |
| 29 | NR | NR | 11 | WT | WT | WT | Low | Low |
| 30 | NR | NR | 6 | WT | WT | WT | Low | Low |
| 31 | R | R | 52 | WT | WT | WT | High | High |
| 32 | R | R | 41 | WT | WT | WT | High | High |
| 33 | R | R | 54 | WT | WT | WT | High | High |
| 34 | NR | NR | 4 | WT | WT | WT | High | High |
| 35 | NR | NR | 13 | WT | WT | WT | High | High |
| 36 | NR | NR | 24 | WT | WT | WT | High | High |
| 37 | NR | NR | 12 | WT | WT | WT | High | High |
| 38 | R | R | 56 | WT | WT | WT | High | High |
| 39 | NR | NR | 8 | WT | WT | WT | High | High |
| 40 | R | R | 63 | WT | WT | WT | High | High |
| 41 | R | R | 52 | WT | WT | WT | High | High |
| 42 | R | R | 74 | WT | WT | WT | High | High |
| 43 | NR | NR | 2 | WT | WT | WT | Low | High |
| 44 | NR | NR | 9 | WT | WT | WT | Low | High |
| 45 | NR | NR | 17 | WT | WT | WT | High | Low |
| 46 | NR | NR | 14 | WT | WT | WT | High | Low |
| 47 | NR | NR | 11 | WT | WT | WT | Low | Low |
| 48 | NR | NR | 9 | WT | WT | WT | Low | Low |
| 49 | R | R | 53 | WT | WT | WT | High | High |
| 50 | R | R | 68 | WT | WT | WT | High | High |
| 51 | NR | NR | 14 | WT | WT | WT | Low | High |
| 52 | NR | NR | 15 | WT | WT | WT | Low | Low |

The samples tested for response to Cetuximab in the above table are stage III/IV colon cancer samples. Most of the samples tested that are NR had mutations in key genes that affect response to Cetuximab, such as KRAS, BRAF and PIK3CA. Also implicated in this pathway are EGFR ligands Amphiregulin and Epiregulin. Low expression of these ligands have been shown to be cause of NR and is believed to affect response to Cetuximab. However, contrary to the expected results there are a subset of samples that are NR in the absence of these biomarkers. Furthermore, a few patients *viz.* patients numbers 2, 7, 13, 20, 23, 25, 26, 28, 31-42, 49, and 50 were found to be NR even though the expression of both the ligands Amphiregulin and Epiregulin are found to be high. However, "Clinical Response Predictor" explant analysis outcome matches the clinical outcome without been impacted by the expression of biomarkers and EGFR ligands.

Thus, one needs to differentiate between a "Driver" and a "Passenger" biomarker as the presence of a biomarker is often not a decisive factor in deciding whether a drug would or would not respond in a particular patient. Also biomarkers

are often linked to a particular drug and a particular type of cancer. In contrast, the instant "Clinical Response Predictor" model provides functional readout specific to the particular patient.

**Example 12: "Clinical Response Predictor" Is a Better Response Predictor Than Cell Lines**

Fundamental deficiency with cell line *in vitro* tests and cell line based xenograft models is that cancer is a heterogeneous disease while the cell lines are homogeneous by definition. These models are thought to oversimplify the problem.

Clinical Response Predictors use of serum/plasma/PBMCs/serum derived ligands, use of extracellular matrix individualized to the tumor type and undisturbed extracellular matrix from the autologus tumor tissue ensure that appropriate paracrine binding factors are in place for the tumor cells to remain viable; this in turn enables the study of signalling pathways involved in tumor initiation, maintenance, progression and suppression, and overcomes the defects associated with cell line based patient segregation systems available in the prior art.

This aspect has been further elaborated in the below Table 14:

**Table 14: Response to Cetuximab on cell lines, where Y indicates response to Cetuximab, N indicates no response to Cetuximab and ND indicates response to Cetuximab is not indicated.**

| S.No | Cell line | K-Ras | B-Raf | PIK3Ca | Response to Cetuximab |
|---|---|---|---|---|---|
| 1 | CaC02 | WT | WT | | Y |
| 2 | HT29 | WT | MUT | MUT "P449T" | N |
| 3 | COLO-205 | WT | MUT | | N |
| 4 | SW480 | MUT "C12" | WT | | N |
| 5 | SW620 | MUT "C12" | WT | WT | N |
| 6 | HCT116 | MUT "C13" | WT | MUT "H1047R" | N |
| 7 | LoVo | MUT "C13" | WT | WT | N |
| 8 | LS1034 | MUT "C146" | WT | | N |
| 9 | LIM1215 | WT | WT | WT | Y |
| 10 | GEO | MUT "C12" | WT | WT | Y |
| 11 | SW403 | MUT "C12" | WT | WT | Y |
| 12 | SW837 | MUT "C12" | WT | WT | Y |
| 13 | SW1463 | MUT "C12" | WT | WT | ND |
| 14 | Gp5d | MUT "C12" | WT | MUT | N |
| 15 | Co94 | MUT | WT | | ND |
| 16 | HCA46 | WT | WT | | ND |
| 17 | COLO-741 | WT | MUT | WT | ND |
| 18 | LS-174T | MUT "C12" | WT | MUT "H1047R" | Y |
| 19 | SNG-M | MUT "C12" | WT | MUT "R88Q" | ND |
| 20 | NCI-H1975 | WT | WT | MUT "G118D" | Y |
| 21 | SW948 | MUT "C12" | WT | MUT | Y |
| 22 | SKCO1 | MUT | WT | WT | Y |
| 23 | HCT8 | MUT | WT | | Y |
| 24 | COLO-201 | WT | MUT | | ND |
| 25 | COLO-320HSR | WT | WT | WT | ND |
| 26 | KM12 | WT | WT | WT | Y |

(continued)

| S.No | Cell line | K-Ras | B-Raf | PIK3Ca | Response to Cetuximab |
|---|---|---|---|---|---|
| 27 | HCA7 | WT | WT | | Y |
| 28 | HT-55 | WT | MUT | | ND |
| 29 | WIDr | WT | MUT | | Y |
| 30 | COLO-201 | WT | MUT | | ND |
| 31 | SW48 | WT | WT | WT | N |
| 32 | SW1417 | WT | MUT | WT | ND |
| 33 | N87 | WT | WT | WT | N |
| 34 | HCC70 | WT | WT | WT | N |
| 35 | COLO-201 | WT | MUT | | N |

As depicted in the Table 14 above, cell lines represent a very homogeneous model and as such have limited utility for drug development. More than 80% of cell lines that are Wild Type (WT) for K-RAS and B-RAF and PIK3Ca evince response to cetuximab. However, clinically, only 10-30% patient respond to cetuximab. This mismatch is due to the lack of clinical relevance of the cell line model. "Clinical Response Predictor" model is shown to be a clinically relevant preclinical tool in Example 11 (Table 13). Using a systems biology approach this platform captures the inherent heterogeneity of the disease to serve as a better predictor of clinical outcome to enable rational drug development.

Advantages of "Clinical Response Predictor" with regards to other technology known in the art:

➢ Genetically engineered mice models used in the prior art are good models but would be useful only when the pathways mediated by the drugs are known. Also, in a variety of cancers, and for a variety of drugs, multiple pathways are involved. This is the major deficiency of the genetically engineered mice models. The instant invention use fresh solid tissues derived from the patient. Further, cell-cell communication is not disrupted by the instant invention as the tissue is processed for the assays. The local microenvironment is also maintained in the case of explant assays.

➢ Mammaprint (from Agendia) and Oncotype-Dx (from Genomic Health) are tests that are used to rank the patients into high risk or low risk based on gene profiling. Mammaprint uses microarray expression profiling of select genes while Oncotype-Dx uses RT-PCR analysis of select genes. Neither of these tests are personalized to the patient nor do they tell what specific drug combination is best suited for the given patient. In contrast, "Clinical Response Predictor" is a functional test that uses the patient's own tumor and patient's own tumor microenvironment to decide what is the optimal drug combination for that specific patient.

➢ With regards to the chemosensitivity test, the present disclosure is able to overcome the deficiencies associated with the said test by way of following: First, the instant invention identified that certain paracrine factors are essential to ensure that functional signalling is maintained in the tumor tissues. Second, the instant invention further discovered that there is a difference in the clinical correlation of such paracrine factors are derived from autologous serum than the heterologous serum. Third, in addition to this, it is important to coat the cell plates with extracellular matrix that have been derived from the same sub-type of cancer. Fourth, it is important to keep the tissue to a particular size (about 100 μm- 300μm) to ensure that the right amount of tissue diffusion take place. Taken together, the combination of these factors result in "Clinical Response Predictor" being a reliable reflector of clinical outcome.

**Example 13: "Clinical Response Predictor" To Predict Clinical Response**

Clinical study is carried out in patients having different types of tumor to study the response to specific Cancer drugs or combinations thereof. The same drugs and their combinations are used in the "Clinical Response Predictor" analysis of the instant invention. The results obtained (M-Score based on pathway inhibition) are correlated to clinical response of the patient to a drug or combination of drugs, based on studies done on a tumor environment personalised for the specific patient.

**Example 13.1:**

The instant "Clinical Response Predictor" Analysis was tested on a 67 year old male patient with Head and Neck Cancer, the tumor site being Right pyriform sinus. The tumor sample was obtained with the consent of the patient

through surgery. The tumor obtained was analyzed, the tumor stage was determined as T3N0M0 and the sample type was categorized as primary.

The tumor sample was obtained and subjected to the present disclosure's method captured above as 'Overview of the instant method'. Thereafter, the data obtained based on the response of the tumor with respect to specific drugs is obtained and presented in the below tables. The tables below represent the response of the patient towards the drugs tested, such that the table A depicts the drugs towards which the response was observed and table B depicts the drugs towards which the response was not observed.

Table A: Responder

| | Drugs Tested | M-Score |
|---|---|---|
| $R_x1$ | Cisplatin | 66 |
| $R_x3$ | Cisplatin + 5-Fluorouracil | 37 |
| $R_x4$ | Cisplatin + Docetaxel+5-Fluorouracil | 61 |

Table B: Non-Responder

| | Drugs Tested | M-Score |
|---|---|---|
| $R_x2$ | Carboplatin + Paclitaxel | 23 |
| $R_x5$ | Cetuximab | 19 |

Based on the M-Score obtained from the above tables and the efficacy data illustrated in Figure 13(A), "Clinical Response Predictor" analysis suggests that the most optimal therapeutic option for the patient is in the administration of the drugs/their combinations in the following order:

1) Cisplatin
2) Cisplatin + Docetaxel + 5-Fluorouracil
3) Cisplatin + 5-Fluorouracil.

**Example 13.2:**

The instant "Clinical Response Predictor" Analysis was tested on a 55 year old male patient with Head and Neck Cancer, the tumor site being Right pyriform sinus. The tumor sample was obtained with the consent of the patient through surgery. The tumor obtained was analyzed, the tumor stage was determined as T3/4N2cM0 and the sample type was categorized as metastatic lymph node.

The tumor sample was obtained and subjected to the present disclosure's method captured above as 'Overview of the instant method'. Thereafter, the data obtained based on the response of the tumor with respect to specific drugs is obtained and presented in the below tables. The tables below represent the response of the patient towards the drugs tested, such that the table A depicts the drugs towards which the response was observed and table B depicts the drugs towards which the response was not observed.

Table A: Responder

| | Drugs Tested | M-Score |
|---|---|---|
| $R_x1$ | Cisplatin | 39 |
| $R_x2$ | Carboplatin + Paclitaxel | 74 |
| $R_x4$ | Cisplatin + Docetaxel + 5-Fluorouracil | 63 |

Table B: Non-Responder

| | Drugs Tested | M-Score |
|---|---|---|
| $R_x3$ | Cisplatin + 5-Fluorouraci | 21 |
| $R_x5$ | Cetuximab | 14 |

Based on the M-Score obtained from the above tables and the efficacy data illustrated in Figure 13(B), "Clinical Response Predictor" analysis suggests that the most optimal therapeutic option for the patient is in the administration of the drugs/their combinations thereof in the following order:

1) Carboplatin + Paclitaxel
2) Cisplatin + Docetaxel + 5-Fluorouracil
3) Cisplatin

**Example 13.3:**

The instant "Clinical Response Predictor" Analysis was tested on a 40 year old male patient with Colon Cancer, the tumor site being rectosigmoid colon. The tumor sample was obtained with the consent of the patient through surgery. The tumor obtained was analyzed, the tumor stage was determined as Stage IV and the sample type was categorized as metastasis.

The tumor sample was obtained and subjected to the present disclosure's method captured above as 'Overview of the instant method'. Thereafter, the data obtained based on the response of the tumor with respect to specific drugs is obtained and presented in the below tables. The tables below represent the response of the patient towards the drugs tested, such that the table A depicts the drugs towards which the response was observed and table B depicts the drugs towards which the response was not observed.

Table A: Responder

| | Drugs Tested | M-Score |
|---|---|---|
| $R_x1$ | Oxaliplatin + 5-Fluorouracil+ Leucovorin | 64 |
| $R_x3$ | Irinotecan + 5-Fluorouracil+ Leucovorin | 32 |
| $R_x7$ | Epirubicin, + Cisplatin + Capecitabine | 51 |

Table B: Non-Responder

| | Drugs Tested | M-Score |
|---|---|---|
| $R_x2$ | 5-Fluorouracil + Leucovorin | 28 |
| $R_x4$ | Irinotecan + 5-Fluorouracil + Leucovorin + Bevacizumab | 21 |
| $R_x5$ | Irinotecan + Cetuximab | 12 |
| $R_x6$ | Panitumumab | 19 |

Based on the M-Score obtained from the above tables and the efficacy data illustrated in Figure 13(C), "Clinical Response Predictor" analysis suggests that the most optimal therapeutic option for the patient is in the administration of the drugs/their combinations thereof in the following order:

1) Oxaliplatin + 5-Fluorouracil + Leucovorin
2) Epirubicin + Cisplatin + Capecitabine
3) Irinotecan + 5-Fluorouracil + Leucovorin

**Example 13.4:**

The instant "Clinical Response Predictor" Analysis was tested on a 56 year old male patient with Colon Cancer, the tumor site being perineal mass (Ca-Rectum). The tumor sample was obtained with the consent of the patient through biopsy. The tumor obtained was analyzed, the tumor stage was determined as T3N0M0 and the sample type was categorized as Recc.

The tumor sample was obtained and subjected to the present disclosure's method captured above as 'Overview of the instant method'. Thereafter, the data obtained based on the response of the tumor with respect to specific drugs is obtained and presented in the below tables. The tables below represent the response of the patient towards the drugs tested, such that the table A depicts the drugs towards which the response was observed and table B depicts the drugs towards which the response was not observed.

Table A: Responder

| | Drugs Tested | M-Score |
|---|---|---|
| $R_x1$ | Oxaliplatin + 5-Fluorouracil + Leucovorin | 46 |
| $R_x3$ | Irinotecan + 5-Fluorouracil + Leucovorin | 61 |
| $R_x4$ | Irinotecan + 5-Fluorouracil + Bevacizumab | 39 |

Table B: Non-Responder

| | Drugs Tested | M-Score |
|---|---|---|
| $R_x2$ | 5-Fluorouracil + Leucovorin | 14 |
| $R_x5$ | Irinotecan + Cetuximab | 21 |
| $R_x6$ | Panitumumab | 27 |
| $R_x7$ | Epirubicin + Cisplatin, + Capecitabine | 18 |

Based on the M-Score obtained from the above tables and the efficacy data illustrated in Figure 13(D), "Clinical Response Predictor" analysis suggests that the most optimal therapeutic option for the patient is in the administration of the drugs/their combinations thereof in the following order:

1) Irinotecan + 5-Fluorouracil + Leucovorin
2) Oxaliplatin + 5-Fluorouracil + Leucovorin
3) Irinotecan + 5-Fluorouracil + Leucovorin + Bevacizumab

**Example 13.5:**

The instant "Clinical Response Predictor" Analysis was tested on a 49 year old male patient with Stomach Cancer, the tumor site being pylorus of stomach. The tumor sample was obtained with the consent of the patient through biopsy. The tumor obtained was analyzed, the tumor stage was unkown and the sample type was categorized as metastatic lymph node recc.

The tumor sample was obtained and subjected to the present disclosure's method captured above as 'Overview of the instant method'. Thereafter, the data obtained based on the response of the tumor with respect to specific drugs is obtained and presented in the below tables. The tables below represent the response of the patient towards the drugs tested, such that the table A depicts the drugs towards which the response was observed and table B depicts the drugs towards which the response was not observed.

Table A: Responder

| | Drugs Tested | M-Score |
|---|---|---|
| $R_x1$ | Epirubicin + Cisplatin + Capecitabine | 47 |
| $R_x3$ | Imatinib | 66 |

Table B: Non-Responder

| | Drugs Tested | M-Score |
|---|---|---|
| $R_x2$ | Herceptin | 14 |
| $R_x4$ | Sunitinib | 25 |

Based on the M-Score obtained from the above tables and the efficacy data illustrated in Figure 13(E), "Clinical Response Predictor" analysis suggests that the most optimal therapeutic option for the patient is in the administration of the drugs/their combinations thereof in the following order:

1) Imatinib
2) Epirubicin + Cisplatin + Capecitabine

**Example 13.6:**

The instant "Clinical Response Predictor" Analysis was tested on a 68 year old female patient with Stomach Cancer, the tumor site being stomach. The tumor sample was obtained with the consent of the patient through biopsy. The tumor obtained was analyzed, the tumor stage was unkown and the sample type was categorized as recc.

The tumor sample was obtained and subjected to the present disclosure's method captured above as 'Overview of the instant method'. Thereafter, the data obtained based on the response of the tumor with respect to specific drugs is obtained and presented in the below tables. The tables below represent the response of the patient towards the drugs tested, such that the table A depicts the drugs towards which the response was observed and table B depicts the drugs towards which the response was not observed.

Table A: Responder

| | Drugs Tested | M-Score |
|---|---|---|
| $R_x1$ | Epirubicin + Cisplatin + Capecitabine | 56 |

Table B: Non-Responder

| | Drugs Tested | M-Score |
|---|---|---|
| $R_x2$ | Herceptin | 15 |
| $R_x3$ | Imatinib | 24 |
| $R_x4$ | Sunitinib | 09 |

Based on the M-Score obtained from the above tables and the efficacy data illustrated in Figure 13(F), "Clinical Response Predictor" analysis suggests that the most optimal therapeutic option for the patient is in the administration of the drugs/their combinations thereof is the following:

1) Epirubicin + Cisplatin + Capecitabine

**Example 13.7:**

The instant "Clinical Response Predictor" Analysis was tested on a 45 year old female patient with Pancreatic Cancer, the tumor site being liver. The tumor sample was obtained with the consent of the patient through biopsy. The tumor obtained was analyzed, the tumor stage was unknown and the sample type was categorized as metastasis.

The tumor sample was obtained and subjected to the present disclosure's method captured above as 'Overview of the instant method'. Thereafter, the data obtained based on the response of the tumor with respect to specific drugs is obtained and presented in the below tables. The tables below represent the response of the patient towards the drugs tested, such that the table A depicts the drugs towards which the response was observed and table B depicts the drugs towards which the response was not observed.

Table A: Responder

| | Drugs Tested | M-Score |
|---|---|---|
| $R_x1$ | Cisplatin + Gemcitabine | 37 |
| $R_x3$ | 5-Fluorouracil + Leucovorin | 54 |

Table B: Non-Responder

| | Drugs Tested | M-Score |
|---|---|---|
| $R_x2$ | Erlotinib | 21 |

Based on the M-Score obtained from the above tables and the efficacy data illustrated in Figure 13(G), "Clinical Response Predictor" analysis suggests that the most optimal therapeutic option for the patient is in the administration of the drugs/their combinations thereof in the following order:

**Example 13.8:**

The instant "Clinical Response Predictor" Analysis was tested on a 50 year old male patient with Pancreatic Cancer, the tumor site being pancreas. The tumor sample was obtained with the consent of the patient through surgery. The tumor obtained was analyzed, the tumor stage was determined as T2N0M0 and the sample type was categorized as primary

The tumor sample was obtained and subjected to the present disclosure's method captured above as 'Overview of the instant method'. Thereafter, the data obtained based on the response of the tumor with respect to specific drugs is obtained and presented in the below tables. The tables below represent the response of the patient towards the drugs tested, such that the table A depicts the drugs towards which the response was observed and table B depicts the drugs towards which the response was not observed.

Table A: Responder

| | **Drugs Tested** | **M-Score** |
|---|---|---|
| $R_x1$ | Cisplatin + Gemcitabine | 72 |

Table B: Non-Responder

| | **Drugs Tested** | **M-Score** |
|---|---|---|
| $R_x2$ | Erlotinib | 14 |
| $R_x3$ | 5-FU + Leucovorin | 23 |

Based on the M-Score obtained from the above tables and the efficacy data illustrated in Figure 13(H), "Clinical Response Predictor" analysis suggests that the most optimal therapeutic option for the patient is in the administration of the drugs/their combinations thereof in the following order:

**Example 13.9:**

The instant "Clinical Response Predictor" Analysis was tested on a 40 year old female patient with Ovary Cancer, the tumor site being ovary. The tumor sample was obtained with the consent of the patient through biopsy. The tumor obtained was analyzed, the tumor stage was unknown and the sample type was categorized as metastasis.

The tumor sample was obtained and subjected to the present disclosure's method captured above as 'Overview of the instant method'. Thereafter, the data obtained based on the response of the tumor with respect to specific drugs is obtained and presented in the below tables. The tables below represent the response of the patient towards the drugs tested, such that the table A depicts the drugs towards which the response was observed and table B depicts the drugs towards which the response was not observed.

Table A: Responder

| | **Drugs Tested** | **M-Score** |
|---|---|---|
| $R_x1$ | Bleomycin + Etoposide + Cisplatin | 76 |
| $R_x2$ | Trabectidin + PLD Doxorubicin | 36 |
| $R_x4$ | Carboplatin + Gemcitabine | 73 |

Table B: Non-Responder

| | **Drugs Tested** | **M-Score** |
|---|---|---|
| $R_x3$ | Docetaxel | 26 |
| $R_x5$ | Doxorubicin (PLD) + Carboplatin | 19 |
| $R_x6$ | Carboplatin + Paclitaxel | 25 |

Based on the M-Score obtained from the above tables and the efficacy data illustrated in Figure 13(I), "Clinical Response Predictor" analysis suggests that the most optimal therapeutic option for the patient is in the administration

of the drugs/their combinations thereof in the following order:

1) Bleomycin + Etoposide + Cisplatin
2) Carboplatin + Gemcitabine
3) Trabectidin + PLD Doxorubicin

**Example 13.10:**

The instant "Clinical Response Predictor" Analysis was tested on a 56 year old female patient with Ovary Cancer, the tumor site being ovary. The tumor sample was obtained with the consent of the patient through surgery. The tumor obtained was analyzed, the tumor stage was unkown and the sample type was categorized as primary.

The tumor sample was obtained and subjected to the present disclosure's method captured above as 'Overview of the instant method'. Thereafter, the data obtained based on the response of the tumor with respect to specific drugs is obtained and presented in the below tables. The tables below represent the response of the patient towards the drugs tested, such that the table A depicts the drugs towards which the response was observed and table B depicts the drugs towards which the response was not observed.

Table A: Responder

| | **Drugs Tested** | **M-Score** |
|---|---|---|
| $R_x1$ | Bleomycin + Etoposide + Cisplatin | 53 |
| $R_x2$ | Trabectidin + PLD Doxorubicin | 64 |
| $R_x6$ | Carboplatin + Paclitaxel | 33 |

Table B: Non-Responder

| | **Drugs Tested** | **M-Score** |
|---|---|---|
| $R_x3$ | Docetaxel | 19 |
| $R_x4$ | Carboplatin + Gemcitabine | 12 |
| $R_x5$ | Doxorubicin (PLD) + Carboplatin | 15 |

Based on the M-Score obtained from the above tables and the efficacy data illustrated in Figure 13(J), "Clinical Response Predictor" analysis suggests that the most optimal therapeutic option for the patient is in the administration of the drugs/their combinations thereof in the following order:

1) Trabectidin + PLD Doxorubicin
2) Bleomycin + Etoposide + Cisplatin
3) Carboplatin + Gemcitabine

**Example 13.11:**

The instant "Clinical Response Predictor" Analysis was tested on a 49 year old female patient with Breast Cancer, the tumor site being Regional Lymph node (R) Breast. The tumor sample was obtained with the consent of the patient through biopsy. The tumor obtained was analyzed, the tumor stage was determined as T3N1M0 and the sample type was categorized as metastasis and recc.

The tumor sample was obtained and subjected to the present disclosure's method captured above as 'Overview of the instant method'. Thereafter, the data obtained based on the response of the tumor with respect to specific drugs is obtained and presented in the below tables. The tables below represent the response of the patient towards the drugs tested, such that the table A depicts the drugs towards which the response was observed and table B depicts the drugs towards which the response was not observed.

Table A: Responder

| | **Drugs Tested** | **M-Score** |
|---|---|---|
| $R_x2$ | Cyclophosphamide + Doxorubicin + 5-Fluorouracil | 52 |
| $R_x5$ | Docetaxel + Capecitabine | 39 |

(continued)

| | Drugs Tested | M-Score |
|---|---|---|
| $R_x$10 | Gemcitabine + Paclitaxel | 48 |

Table B: Non-Responder

| | Drugs Tested | M-Score |
|---|---|---|
| $R_x$1 | Anastrozole | 20 |
| $R_x$3 | Capecitabine | 26 |
| $R_x$4 | Docetaxel | 26 |
| $R_x$6 | Doxorubicin | 17 |
| $R_x$7 | Doxorubicin + Cyclophosphamide | 15 |
| $R_x$8 | Enanthate | 08 |
| $R_x$9 | Gemcitabine | 21 |
| $R_x$11 | Paclitaxel | 11 |
| $R_x$12 | Vinorelbine | 19 |

Based on the M-Score obtained from the above tables and the efficacy data illustrated in Figure 13(K), "Clinical Response Predictor" analysis suggests that the most optimal therapeutic option for the patient is in the administration of the drugs/their combinations thereof in the following order:

1) Cyclophosphamide + Doxorubicin + 5-Fluorouracil
2) Gemcitabine + Paclitaxel
3) Docetaxel + Capecitabine

**Example 13.12:**

The instant "Clinical Response Predictor" Analysis was tested on a 51 year old female patient with Breast Cancer, the tumor site being breast. The tumor sample was obtained with the consent of the patient through biopsy. The tumor obtained was analyzed, the tumor stage was undetermined and the sample type was categorized as primary.

The tumor sample was obtained and subjected to the present disclosure's method captured above as 'Overview of the instant method'. Thereafter, the data obtained based on the response of the tumor with respect to specific drugs is obtained and presented in the below tables. The tables below represent the response of the patient towards the drugs tested, such that the table A depicts the drugs towards which the response was observed and table B depicts the drugs towards which the response was not observed.

Table A: Responder

| | Drugs Tested | M-Score |
|---|---|---|
| $R_x$3 | Cyclophosphamide + Doxorubicin + Docetaxel | 43 |
| $R_x$6 | Filgrastim + Cyclophosphamide + Doxorubicin + 5-Fluorouracil | 77 |
| $R_x$7 | Filgrastim + Cyclophosphamide + Epirubicin, + 5-Fluorouracil | 42 |
| $R_x$8 | Gemcitabine + Docetaxel | 62 |

Table B: Non-Responder

| | Drugs Tested | M-Score |
|---|---|---|
| $R_x$1 | Cisplatin + Gemcitabine | 17 |
| $R_x$2 | Cyclophosphamide + Paclitaxel | 23 |
| $R_x$4 | Docetaxel + Cyclophosphamide | 22 |
| $R_x$5 | Docetaxel + Cyclophosphamide + Epirubicin + 5-Fluorouracil | 19 |

Based on the M-Score obtained from the above tables and the efficacy data illustrated in Figure 13(L), "Clinical Response Predictor" analysis suggests that the most optimal therapeutic option for the patient is in the administration of the drugs/their combinations thereof in the following order:

1) Filgrastim + Cyclophosphamide + Doxorubicin + 5-Fluorouracil
2) Gemcitabine + Docetaxel
3) Cyclophosphamide + Doxorubicin + Docetaxel
4) Filgrastim + Cyclophosphamide + Epirubicin + 5-Fluorouracil

**Example 13.13:**

The instant "Clinical Response Predictor" Analysis was tested on a 50 year old male patient with Liver Cancer, the tumor site being liver. The tumor sample was obtained with the consent of the patient through surgery. The tumor obtained was analyzed, the tumor stage was determined as T3NxM1 and the sample type was categorized as metastasis.

The tumor sample was obtained and subjected to the present disclosure's method captured above as 'Overview of the instant method'. Thereafter, the data obtained based on the response of the tumor with respect to specific drugs is obtained and presented in the below tables. The tables below represent the response of the patient towards the drugs tested, such that the table A depicts the drugs towards which the response was not observed.

Table A: Non-Responder

| | Drugs Tested | M-Score |
|---|---|---|
| $R_x1$ | Sorafenib | 16 |

Based on the M-Score obtained from the above table and the efficacy data illustrated in Figure 13(M), "Clinical Response Predictor" analysis suggests that Sorafenib is not an optimal therapeutic option for the instant patient. Further tests need to be carried out using other anticancer agents to see if any of the other SOCs can be used on this patient.

**Example 13.14:**

The instant "Clinical Response Predictor" Analysis was tested on a 56 year old male patient with Liver Cancer, the tumor site being liver. The tumor sample was obtained with the consent of the patient through surgery. The tumor obtained was analyzed, the tumor stage was determined as T4N0M0 and the sample type was categorized as primary.

The tumor sample was obtained and subjected to the present disclosure's method captured above as 'Overview of the instant method'. Thereafter, the data obtained based on the response of the tumor with respect to specific drugs is obtained and presented in the below tables. The table A depicts the drugs towards which the response was observed.

Table A: Responder

| | Drugs Tested | M-Score |
|---|---|---|
| $R_x1$ | Sorafenib | **46** |

Based on the M-Score obtained from the above tables and the efficacy data illustrated in Figure 13(N), "Clinical Response Predictor" analysis suggests that Sorafenib is an optimal therapeutic option for the treatment of the patient.

**Example 13.15:**

The instant "Clinical Response Predictor" Analysis was tested on a 56 year old male patient with Colorectum Cancer, the tumor site being perineal mass. The tumor sample was obtained with the consent of the patient through biopsy. The tumor obtained was analyzed, the tumor stage was determined as $T_3N_0M_0$ and the sample type was categorized as recurrent.

The tumor sample was obtained and subjected to the present disclosure's method captured above as 'Overview of the instant method'. Thereafter, the data obtained based on the response of the tumor with respect to specific drugs is obtained and presented in the below tables. The tables below represent the response of the patient towards the drugs tested, such that the table A depicts the drugs towards which the response was observed and table B depicts the drugs towards which the response was not observed.

Table A: Responder

| | Drugs Tested | M-Score |
|---|---|---|
| $R_x1$ | Oxaliplatin + 5-FU + Leucovorin | 75 |
| $R_x2$ | Irinotecan + 5-FU + Leucovorin | 72 |
| $R_x3$ | Oxciliplatin + 5-FU | 35 |

Table B: Non-Responder

| | Drugs Tested | M-Score |
|---|---|---|
| $R_x4$ | Capecitabine + Erbitux | 24 |
| $R_x5$ | Avastin | 20 |

Based on the M-Score obtained from the above tables and the efficacy data illustrated in Figure 13(O), "Clinical Response Predictor" analysis suggests that the most optimal therapeutic option for the patient is in the administration of the drugs/their combinations thereof in the following order:

1) Rx1 -Oxaliplatin + 5-FU + Leucovorin
2) Rx2 -Irinotecan + 5-FU + Leucovorin
3) Rx3 - Oxaliplatin + 5-FU
4) Rx4 - Capecitabine + Erbitux
5) Rx5 - Avastin

**Example 13.16:**

The instant "Clinical Response Predictor" Analysis was tested on a 59 year old male patient having Colorectum Cancer with lung metstatic(mets), the tumor site being rectosigmoid. The tumor sample was obtained with the consent of the patient through biopsy. The tumor obtained was analyzed, the tumor stage was determined as $T_4N_2M_X$ and the sample type was categorized as metastatic.

The tumor sample was obtained and subjected to the present disclosure's method captured above as 'Overview of the instant method'. Thereafter, the data obtained based on the response of the tumor with respect to specific drugs is obtained and presented in the below tables. The tables below represent the response of the patient towards the drugs tested, such that the table A depicts the drugs towards which the response was observed and table B depicts the drugs towards which the response was not observed.

Table A: Responder

| | Drugs Tested | M-Score |
|---|---|---|
| $R_x1$ | Oxaliplatin + Irinotecan | 73 |
| $R_x3$ | 5-FU + Leucovorin | 29 |

Table B: Non-Responder

| | Drugs Tested | M-Score |
|---|---|---|
| $R_x2$ | Erbitux + Capecitabine | 22 |
| $R_x4$ | Irinotecan + Erbitux | 24 |

Based on the M-Score obtained from the above tables and the efficacy data illustrated in Figure 13(P), "Clinical Response Predictor" analysis suggests that the most optimal therapeutic option for the patient is in the administration of the drugs/their combinations thereof in the following order:

1) $R_{x1}$ - Oxaliplatin + Irinotecan
2) $R_x2$ - Erbitux + Capecitabine

3) $R_x3$ - 5-FU + Leucovorin
4) $R_x4$ - Irinotecan + Erbitux

**Example 13.17:**

The instant "Clinical Response Predictor" Analysis was tested on a 45 year old female patient having Pancreatic Cancer with liver mets, the tumor site being pancreas. The tumor sample was obtained with the consent of the patient through biopsy. The tumor obtained was analyzed, the tumor stage was determined as $T_3N_2M_1$ and the sample type was categorized as metastatic.

The tumor sample was obtained and subjected to the present disclosure's method captured above as 'Overview of the instant method'. Thereafter, the data obtained based on the response of the tumor with respect to specific drugs is obtained and presented in the below tables. The tables below represent the response of the patient towards the drugs tested, such that the table A depicts the drugs towards which the response was observed and table B depicts the drugs towards which the response was not observed.

Table A: Responder

| | Drugs Tested | M-Score |
|---|---|---|
| $R_x3$ | Abraxane | 50 |
| $R_x4$ | Erlotinib + Gemcitabine | 68 |

Table B: Non-Responder

| | Drugs Tested | M-Score |
|---|---|---|
| $R_x1$ | Cisplatin + Gemcitabine | 23 |
| $R_x2$ | Oxaliplatin, + 5-FU | 23 |
| $R_x5$ | 5-FU + Leucovorin | 20 |

Based on the M-Score obtained from the above tables and the efficacy data illustrated in Figure 13(Q), "Clinical Response Predictor" analysis suggests that the most optimal therapeutic option for the patient is in the administration of the drugs/their combinations thereof in the following order:

1) $R_x1$ - Cisplatin + Gemcitabine
2) $R_x2$ - Oxaliplatin + 5-FU
3) $R_x3$ - Abraxane
4) $R_x4$ - Erlotinib + Gemcitabine
5) $R_x5$ - 5-FU + Leucovorin

**Example 13.18:**

The instant "Clinical Response Predictor" Analysis was tested on a 49 year old female patient having Breast Cancer with mets, the tumor site being Regional lymph node (Rt Br). The tumor sample was obtained with the consent of the patient through biopsy. The tumor obtained was analyzed, the tumor stage was determined as $T_3N_1M_1$ and the sample type was categorized as recurrent.

The tumor sample was obtained and subjected to the present disclosure's method captured above as 'Overview of the instant method'. Thereafter, the data obtained based on the response of the tumor with respect to specific drugs is obtained and presented in the below tables. The tables below represent the response of the patient towards the drugs tested, such that the table A depicts the drugs towards which the response was observed and table B depicts the drugs towards which the response was not observed.

Table A: Responder

| | Drugs Tested | M-Score |
|---|---|---|
| $R_x1$ | Cyclophosphamide + Methotrexate + 5-FU | 77 |
| $R_x3$ | Doxorubicin + Cyclophosphamide + 5-FU | 68 |
| $R_x4$ | Doxorubicin + Cyclophosphamide + Paclitaxel | 70 |

(continued)

| | Drugs Tested | M-Score |
|---|---|---|
| $R_x7$ | Doxorubicin + Cyclophosphamide | 29 |
| $R_x8$ | Doxorubicin + Capecitabine | 32 |

Table B: Non-Responder

| | Drugs Tested | M-Score |
|---|---|---|
| $R_x2$ | Abraxane | 20 |
| $R_x5$ | Avastin | 18 |
| $R_x6$ | Capecitabine + Lapatinib | 20 |

Based on the M-Score obtained from the above tables and the efficacy data illustrated in Figure 13(R), "Clinical Response Predictor" analysis suggests that the most optimal therapeutic option for the patient is in the administration of the drugs/their combinations thereof in the following order:

1) $R_x1$ - Cyclophosphamide+Methotrexate+5-FU
2) $R_x2$ - Abraxane
3) $R_x3$ - Doxorubicin+ Cyclophosphamide+5-FU
4) $R_x4$ - Doxorubicin+ Cyclophosphamide+Paclitaxel
5) $R_x5$ - Avastin
6) $R_x6$ - Capecitabine + Lapatinib
7) $R_x7$ - Doxorubicin+ Cyclophosphamide
8) $R_x8$ - Docetaxel + Capecitabine

**Example 13.19:**

The instant "Clinical Response Predictor" Analysis was tested on a 40 year old female patient having Breast Cancer with Sub Clavian Lymph Node Metastasis (SCLN mets), the tumor site being supraclaviculus lymph node. The tumor sample was obtained with the consent of the patient through biopsy. The tumor obtained was analyzed, the tumor stage was determined as $T_XN_XM_z$ and the sample type was categorized as metastatic.

The tumor sample was obtained and subjected to the present disclosure's method captured above as 'Overview of the instant method'. Thereafter, the data obtained based on the response of the tumor with respect to specific drugs is obtained and presented in the below tables. The tables below represent the response of the patient towards the drugs tested, such that the table A depicts the drugs towards which the response was observed and table B depicts the drugs towards which the response was not observed.

Table A: Responder

| | Drugs Tested | M-Score |
|---|---|---|
| $R_x1$ | Capecitabine + Lapatinib | 68 |
| $R_x2$ | Gemcitabine + Erlotinib | 48 |

Table B: Non- Responder

| | Drugs Tested | M-Score |
|---|---|---|
| $R_x3$ | Herceptin | 15 |
| $R_x4$ | Methotrexate + Cyclophosphamide | 19 |
| $R_x5$ | Avastin | 14 |
| $R_x6$ | 5-FU + Carboplatin | 17 |

Based on the M-Score obtained from the above tables and the efficacy data illustrated in Figure 13(S), "Clinical Response Predictor" analysis suggests that the most optimal therapeutic option for the patient is in the administration

of the drugs/their combinations thereof in the following order:

1) $R_x1$ - Capecitabine + Lapatinib
2) $R_x2$ - Gemcitabine +Erlotinb
3) $R_x3$ - Herceptin
4) $R_x4$ - Methotrexate + Cyclophosphamide
5) $R_x5$ - Avastin
6) $R_x6$ - 5-FU+ Carboplatin

**Example 14: "Clinical Response Predictor" to Test Efficacy of Drugs**

Primary H&N tumor sample from patients enrolled in the clinical trials slated to receive Cisplatin, Paclitaxel and 5-FU is subjected to "Clinical Response Predictor" analysis. The tumor sample is collected by punch biopsy. The tumor stage of the sample collected is clinical StageII/III. "Clinical Response Predictor" explant evaluation is carried out to predict clinical outcome as explained in Example 5. The Assays conducted to arrive at the M-score are WST, KI, and TUNEL. Independently, PET-CT imaging is carried out before and after the treatment to assess clinical outcome as per PERCIST criteria and the patient is subjected to clinical trials. The "Clinical Response Predictor" prediction is compared to clinical outcome to assess the predictive power of "Clinical Response Predictor" (Figure 12).

About 112 H&N tumor patients are enrolled in this study as depicted in table 15 captured below, wherein the 'Clinical Response Predictor' is used to determine the sensitivity index and correlate the same with the clinical outcome.

**Table 15**

| SI No. | Treatment | Clinical Response predictor | | | | Sensitivity Index | Senstivity Index Prediction |
|---|---|---|---|---|---|---|---|
| | | Viability Inhibition | Proliferation inhibition | Histology | TUNEL | | |
| 1 | Cisplatin+Docetaxel+5FU | 18 | 25 | 10 | 10 | 16 | Progressive Disease |
| 2 | Cisplatin+Docetaxel+5FU | 22 | 12 | 5 | 20 | 15 | Progressive Disease |
| 3 | Cisplatin+Docetaxel+5FU | 82 | 82 | 25 | 75 | 66 | Complete Response |
| 4 | Cisplatin+Docetaxel+5FU | 42 | 50 | 15 | 65 | 43 | Partial Response |
| 5 | Cisplatin+Docetaxel+5FU | 31 | 10 | 5 | 7 | 13 | Progressive Disease |
| 6 | Cisplatin+Docetaxel+5FU | 21 | 6 | 9 | 25 | 15 | Progressive Disease |
| 7 | Cisplatin+Docetaxel+5FU | 52 | 66 | 75 | 66 | 65 | Complete Response |
| 8 | Cisplatin+Docetaxel+5FU | 55 | 34 | 42 | 68 | 50 | Partial Response |
| 9 | Cisplatin+Docetaxel+5FU | 87 | 70 | 54 | 92 | 76 | Complete Response |
| 10 | Cisplatin+Docetaxel+5FU | 65 | 35 | 15 | 45 | 40 | Partial Response |
| 11 | Cisplatin+Docetaxel+5FU | 62 | 76 | 64 | 20 | 56 | Partial Response |
| 12 | Cisplatin+Docetaxel+5FU | 8 | 42 | 10 | 35 | 24 | Progressive Disease |
| 13 | Cisplatin+Docetaxel+5FU | 24 | 15 | 18 | 65 | 31 | Partial Response |
| 14 | Cisplatin+Docetaxel+5FU | 59 | 85 | 25 | 90 | 65 | Complete Response |
| 15 | Cisplatin+Docetaxel+5FU | 31 | 54 | 15 | 48 | 37 | Partial Response |
| 16 | Cisplatin+Docetaxel+5FU | 56 | 55 | 63 | 70 | 61 | Complete Response |
| 17 | Cisplatin+Docetaxel+5FU | 12 | 8 | 9 | 12 | 10 | Progressive Disease |
| 18 | Cisplatin+Docetaxel+5FU | 82 | 100 | 53 | 76 | 78 | Complete Response |
| 19 | Cisplatin+Docetaxel+5FU | 11 | 83 | 11 | 67 | 43 | Partial Response |
| 20 | Cisplatin+Docetaxel+5FU | 23 | 14 | 22 | 64 | 31 | Partial Response |
| 21 | Cisplatin+Docetaxel+5FU | 47 | 74 | 28 | 57 | 52 | Partial Response |
| 22 | Cisplatin+Docetaxel+5FU | 7 | 0 | 15 | 0 | 6 | Progressive Disease |
| 23 | Cisplatin+Docetaxel+5FU | 73 | 87 | 15 | 100 | 69 | Complete Response |
| 24 | Cisplatin+Docetaxel+5FU | 61 | 35 | 20 | 55 | 43 | Partial Response |

(continued)

| SI No. | Treatment | Clinical Response predictor | | | | Sensitivity Index | |
|---|---|---|---|---|---|---|---|
| | | Viability Inhibition | Proliferation inhibition | Histology | TUNEL | | Senstivity Index Prediction |
| 25 | Cisplatin+Docetaxel+5FU | 42 | 82 | 48 | 72 | 61 | Complete Response |
| 26 | Cisplatin+Docetaxel+5FU | 52 | 78 | 65 | 100 | 74 | Complete Response |
| 27 | Cisplatin+Docetaxel+5FU | 21 | 58 | 33 | 25 | 34 | Partial Response |
| 28 | Cisplatin+Docetaxel+5FU | 31 | 0 | 5 | 10 | 12 | Progressive Disease |
| 29 | Cisplatin+Docetaxel+5FU | 42 | 72 | 45 | 24 | 46 | Partial Response |
| 30 | Cisplatin+Docetaxel+5FU | 27 | 77 | 42 | 52 | 50 | Partial Response |
| 31 | Cisplatin+Docetaxel+5FU | 55 | 65 | 80 | 56 | 64 | Complete Response |
| 32 | Cisplatin+Docetaxel+5FU | 47 | 31 | 20 | 47 | 36 | Partial Response |
| 33 | Cisplatin+Docetaxel+5FU | 66 | 72 | 58 | 100 | 74 | Complete Response |
| 34 | Cisplatin+Docetaxel+5FU | 72 | 32 | 29 | 41 | 44 | Partial Response |
| 35 | Cisplatin+Docetaxel+5FU | 34 | 25 | 19 | 35 | 28 | Partial Response |
| 36 | Cisplatin+Docetaxel+5FU | 42 | 40 | 15 | 65 | 41 | Partial Response |
| 37 | Cisplatin+Docetaxel+5FU | 10 | 2 | 5 | 10 | 7 | Progressive Disease |
| 38 | Cisplatin+Docetaxel+5FU | 72 | 21 | 13 | 32 | 35 | Partial Response |
| 39 | Cisplatin+Docetaxel+5FU | 77 | 57 | 51 | 62 | 62 | Complete Response |
| 40 | Cisplatin+Docetaxel+5FU | 18 | 0 | 7 | 12 | 9 | Progressive Disease |
| 41 | Cisplatin+Docetaxel+5FU | 31 | 55 | 24 | 72 | 46 | Partial Response |
| 42 | Cisplatin+Docetaxel+5FU | 29 | 88 | 32 | 100 | 62 | Complete Response |
| 43 | Cisplatin+Docetaxel+5FU | 44 | 27 | 20 | 44 | 34 | Partial Response |
| 44 | Cisplatin+Docetaxel+5FU | 51 | 55 | 12 | 36 | 39 | Partial Response |
| 45 | Cisplatin+Docetaxel+5FU | 32 | 42 | 15 | 65 | 39 | Partial Response |
| 46 | Cisplatin+Docetaxel+5FU | 37 | 85 | 46 | 92 | 65 | Complete Response |
| 47 | Cisplatin+Docetaxel+5FU | 10 | 15 | 5 | 0 | 8 | Progressive Disease |
| 48 | Cisplatin+Docetaxel+5FU | 22 | 65 | 26 | 44 | 39 | Partial Response |

(continued)

| SI No. | Treatment | Clinical Response predictor | | | | Sensitivity Index | |
|---|---|---|---|---|---|---|---|
| | | Viability Inhibition | Proliferation inhibition | Histology | TUNEL | | Senstivity Index Prediction |
| 49 | Cisplatin+Docetaxel+5FU | 56 | 65 | 32 | 88 | 60 | Complete Response |
| 50 | Cisplatin+Docetaxel+5FU | 43 | 32 | 24 | 57 | 39 | Partial Response |
| 51 | Cisplatin+Docetaxel+5FU | 52 | 41 | 22 | 54 | 42 | Partial Response |
| 52 | Cisplatin+Docetaxel+5FU | 48 | 45 | 32 | 65 | 48 | Partial Response |
| 53 | Cisplatin+Docetaxel+5FU | 32 | 23 | 14 | 40 | 27 | Partial Response |
| 54 | Cisplatin+Docetaxel+5FU | 3 | 0 | 2 | 0 | 1 | Progressive Disease |
| 55 | Cisplatin+Docetaxel+5FU | 55 | 63 | 31 | 90 | 60 | Complete Response |
| 56 | Cisplatin+Docetaxel+5FU | 60 | 55 | 41 | 32 | 47 | Partial Response |
| 57 | Cisplatin+Docetaxel+5FU | 45 | 22 | 15 | 45 | 32 | Partial Response |
| 58 | Cisplatin+Docetaxel+5FU | 5 | 15 | 4 | 0 | 6 | Progressive Disease |
| 59 | Cisplatin+Docetaxel+5FU | 64 | 42 | 10 | 35 | 38 | Partial Response |
| 60 | Cisplatin+Docetaxel+5FU | 31 | 43 | 25 | 55 | 39 | Partial Response |
| 61 | Cisplatin+Docetaxel+5FU | 22 | 35 | 12 | 0 | 17 | Progressive Disease |
| 62 | Cisplatin+Docetaxel+5FU | 15 | 52 | 17 | 64 | 37 | Partial Response |
| 63 | Cisplatin+Docetaxel+5FU | 85 | 58 | 17 | 49 | 52 | Partial Response |
| 64 | Cisplatin+Docetaxel+5FU | 54 | 72 | 42 | 67 | 59 | Partial Response |
| 65 | Cisplatin+Docetaxel+5FU | 7 | 8 | 6 | 30 | 13 | Progressive Disease |
| 66 | Cisplatin+Docetaxel+5FU | 42 | 32 | 16 | 65 | 39 | Partial Response |
| 67 | Cisplatin+Docetaxel+5FU | 24 | 42 | 55 | 56 | 44 | Partial Response |
| 68 | Cisplatin+Docetaxel+5FU | 29 | 0 | 5 | 12 | 12 | Progressive Disease |
| 69 | Cisplatin+Docetaxel+5FU | 62 | 62 | 32 | 46 | 51 | Partial Response |
| 70 | Cisplatin+Docetaxel+5FU | 16 | 0 | 5 | 25 | 12 | Progressive Disease |
| 71 | Cisplatin+Docetaxel+5FU | 67 | 54 | 30 | 45 | 49 | Partial Response |
| 72 | Cisplatin+Docetaxel+5FU | 41 | 76 | 43 | 100 | 65 | Complete Response |

(continued)

| SI No. | Treatment | Clinical Response predictor | | | | Sensitivity Index | |
|---|---|---|---|---|---|---|---|
| | | Viability Inhibition | Proliferation inhibition | Histology | TUNEL | | Senstivity Index Prediction |
| 73 | Cisplatin+Docetaxel+5FU | 58 | 32 | 35 | 85 | 53 | Partial Response |
| 74 | Cisplatin+Docetaxel+5FU | 0 | 50 | 10 | 20 | 20 | Progressive Disease |
| 75 | Cisplatin+Docetaxel+5FU | 31 | 45 | 31 | 47 | 39 | Partial Response |
| 76 | Cisplatin+Docetaxel+5FU | 2 | 34 | 20 | 21 | 19 | Progressive Disease |
| 77 | Cisplatin+Docetaxel+5FU | 43 | 45 | 41 | 65 | 49 | Partial Response |
| 78 | Cisplatin+Docetaxel+5FU | 72 | 65 | 88 | 89 | 79 | Complete Response |
| 79 | Cisplatin+Docetaxel+5FU | 25 | 54 | 45 | 43 | 42 | Partial Response |
| 80 | Cisplatin+Docetaxel+5FU | 14 | 8 | 12 | 30 | 16 | Progressive Disease |
| 81 | Cisplatin+Docetaxel+5FU | 23 | 85 | 21 | 50 | 45 | Partial Response |
| 82 | Cisplatin+Docetaxel+5FU | 55 | 78 | 57 | 90 | 70 | Complete Response |
| 83 | Cisplatin+Docetaxel+5FU | 32 | 64 | 33 | 32 | 40 | Partial Response |
| 84 | Cisplatin+Docetaxel+5FU | 27 | 88 | 62 | 100 | 69 | Complete Response |
| 85 | Cisplatin+Docetaxel+5FU | 64 | 39 | 42 | 32 | 44 | Partial Response |
| 86 | Cisplatin+Docetaxel+5FU | 25 | 5 | 2 | 15 | 12 | Progressive Disease |
| 87 | Cisplatin+Docetaxel+5FU | 55 | 33 | 21 | 32 | 35 | Partial Response |
| 88 | Cisplatin+Docetaxel+5FU | 39 | 85 | 65 | 75 | 66 | Complete Response |
| 89 | Cisplatin+Docetaxel+5FU | 37 | 47 | 65 | 55 | 51 | Partial Response |
| 90 | Cisplatin+Docetaxel+5FU | 2 | 3 | 6 | 7 | 5 | Progressive Disease |
| 91 | Cisplatin+Docetaxel+5FU | 11 | 13 | 2 | 15 | 10 | Progressive Disease |
| 92 | Cisplatin+Docetaxel+5FU | 34 | 28 | 25 | 34 | 30 | Partial Response |
| 93 | Cisplatin+Docetaxel+5FU | 77 | 56 | 32 | 75 | 60 | Complete Response |
| 94 | Cisplatin+Docetaxel+5FU | 47 | 67 | 42 | 50 | 52 | Partial Response |
| 95 | Cisplatin+Docetaxel+5FU | 21 | 22 | 4 | 10 | 14 | Progressive Disease |
| 96 | Cisplatin+Docetaxel+5FU | 72 | 85 | 45 | 72 | 69 | Complete Response |

(continued)

| SI No. | Treatment | Clinical Response predictor | | | | Sensitivity Index | |
|---|---|---|---|---|---|---|---|
| | | Viability Inhibition | Proliferation inhibition | Histology | TUNEL | | Senstivity Index Prediction |
| 97 | Cisplatin+Docetaxel+5FU | 33 | 75 | 66 | 87 | 65 | Complete Response |
| 98 | Cisplatin+Docetaxel+5FU | 17 | 0 | 8 | 5 | 8 | Progressive Disease |
| 99 | Cisplatin+Docetaxel+5FU | 41 | 37 | 62 | 45 | 46 | Partial Response |
| 100 | Cisplatin+Docetaxel+5FU | 72 | 46 | 31 | 84 | 58 | Partial Response |
| 101 | Cisplatin+Docetaxel+5FU | 50 | 85 | 70 | 100 | 76 | Complete Response |
| 102 | Cisplatin+Docetaxel+5FU | 32 | 3 | 10 | 5 | 13 | Progressive Disease |
| 103 | Cisplatin+Docetaxel+5FU | 65 | 56 | 39 | 45 | 51 | Partial Response |
| 104 | Cisplatin+Docetaxel+5FU | 58 | 72 | 65 | 95 | 73 | Complete Response |
| 105 | Cisplatin+Docetaxel+5FU | 35 | 100 | 69 | 87 | 73 | Complete Response |
| 106 | Cisplatin+Docetaxel+5FU | 42 | 33 | 27 | 45 | 37 | Partial Response |
| 107 | Cisplatin+Docetaxel+5FU | 0 | 20 | 5 | 0 | 6 | Progressive Disease |
| 108 | Cisplatin+Docetaxel+5FU | 19 | 43 | 42 | 54 | 40 | Partial Response |
| 109 | Cisplatin+Docetaxel+5FU | 18 | 53 | 32 | 48 | 38 | Partial Response |
| 110 | Cisplatin+Docetaxel+5FU | 32 | 15 | 12 | 0 | 15 | Progressive Disease |
| 111 | Cisplatin+Docetaxel+5FU | 44 | 85 | 52 | 95 | 69 | Complete Response |
| 112 | Cisplatin+Docetaxel+5FU | 21 | 0 | 12 | 0 | 8 | Progressive Disease |

From the above table as well as from the figure 12 the following can be derived: Left panel of the Figure 12 (pre-dose and post-dose) dislplay the CT images showing the location of the tumors prior to and after chemotherapy. The top left panel shows that the tumor has shrunk and that the person has responded to therapy. The tumor from this person on being evaluated using oncoprint received an M-score of 62 indicative of clinical complete response which is alignment with actual clinical outcome. The top right panel shows the clinical response of the 30 tumor samples that had Mscore >/= 60, more than 90% of the patients had complete response, while 10% had partial response. However, none of them were non-responders.

Similarly the middle left panel is a representation of a partial responder whose M-score is determined to be 45. As predicted for M-score between 25 and <60 the patient is indicative of partial response. Of the 53 patient tumors having M-score in this category, more than 79% were partial responders with 8% having complete response and 13% having non-response.

The bottom panel is representative of non-responders, wherein the left post-dose CT shows that the patient has progressive disease after treatment and the tumor was accorded an M-score of 18 indicative of non-response. Of the 29 patients predicted to have non-response by "Clinical Response Predictor", 100% of them did indeed exhibit non-response indicating the power of this technology to reliably predict clinical outcome.

## APPLICATIONS:

### Drug development application:

*Matching patients to drugs:* In the context of drug development, it is important to know which patients are most likely to respond to the drug under development even before the drug is administered to the patients. Further, it is particularly important in the context of cancer as one needs to decide what existing drugs need to be combined with the drug under development under the "Combination" strategy that is used in cancer treatment. It is also useful in deciding which type of cancer to target (eg: colon cancer vs pancreatic cancer). Overall, it is useful in developing a better clinical trial strategy that results in faster time to develop, lower cost and increased chances of success.

### Diagnostic application:

*Treatment selection*: It is useful as a diagnostic model in helping the doctors decide which treatment option, from among the currently approved options, are best suitable for the patient under investigation. This is particularly useful in secondary (relapsed) as well as metastatic cancer patients, where the current treatment success rate is < 20% and varies from patient to patient. It is also useful in deciding the first line treatment where the current success rate is ~ 50%. Diagnostic application of "Clinical Response Predictor" has been validated in the context of Head & Neck Cancer, Breast cancer, Gastric cancer, Pancreatic cancer, Colorectal cancer, Liver cancer, Ovarian cancer, Esophageal cancer, AML & CML. The prediction power is ~100% in the case of non-responders, ~75% in the case of partial responders and ~90% in the case of responders.

### Translational biology application:

In the development of anti-cancer drugs, identification of the optimal cancer for the drugs being developed, selection of Standard of Care drug as a Co-development strategy for the drugs being developed, selection of patient profile most likely to respond to the drug or drug combination being studied, and the identification of prognostic biomarkers for the drug or drug combination being studied. Further, the present invention also utilises the patient segregation tool in development of companion diagnostic tools for anti cancer drugs including chemotherapeutics, targeted drugs, and biologics.

## Claims

1. An Extra Cellular Matrix, ECM, composition comprising components - collagen 1, collagen 3, collagen 4, collagen 6, Fibronectin, Vitronectin, Cadherin, Filamin A, Vimentin, Osteopontin, Laminin, Decorin, and Tenascin C.

2. The ECM composition as claimed in claim 1 further comprising one or more components selected from the group consisting of Basement membrane proteins, Cytoskeletal proteins and Matrix proteins.

3. A method to obtain Extra Cellular Matrix, ECM composition as claimed in claim 1, said method comprising combining the components of the ECM - collagen 1, collagen 3, collagen 4, collagen 6, Fibronectin,

VitroNectin , Cadherin, FilaminA, Vimentin, Osteopontin, Laminin, Decorin, Tenascin C to obtain the ECM composition.

4. The method as claimed in claim 3, wherein the components further comprise one or more components selected from the group consisting of Basement membrane proteins, Cytoskeletal proteins and Matrix proteins.

5. A tumor microenvironment platform for culturing tumor tissue, said microenvironment comprising the ECM composition as claimed in claim 1 or 2, culture medium optionally along with serum, plasma or PBMCs and drug.

6. A method for obtaining tumor microenvironment platform for culturing tumor tissue as claimed in claim 5, said method comprising act of coating platform with ECM composition as claimed in claim 1 or 2 and adding culture medium optionally along with serum, plasma or PBMCs and drug, to the platform to obtain the tumor microenvironment platform.

7. A method of organotypic culturing of tumor tissue, said method comprising act of culturing the tumor tissue on tumor microenvironment platform as claimed in claim 5 to obtain the organotypic culture.

8. A method of predicting response of a tumor subject to drug(s), said method comprising acts of:

a. culturing the subject's tumor tissue on tumor microenvironment platform as claimed in claim 5, to obtain cultured tumor tissue;
b. treating the cultured tumor tissue with the drug(s) and conducting assay;
c. converting the assay' s readout into numeric metric to obtain sensitivity index and thereby, predicting the response of the subject to the drug(s); and
d. optionally, correlating the sensitivity index to clinical response of the subject to the drug(s).

9. A method of predicting response of a tumor subject to drug(s), said method comprising acts of:

a. culturing the subject's tumor tissue on tumor microenvironment platform as claimed in claim 5, to obtain cultured tumor tissue;
b. treating the cultured tumor tissue with the drug(s);
c. assessing tumor response to the drug by plurality of assays to obtain assessment score for each of the plurality of assays;
d. assigning a weightage score for each of the plurality of assays;
e. multiplying the assessment score of each of the plurality of assays with weightage score of corresponding assay of the plurality of assays to obtain independent assay score for each of the plurality of assays;
f. combining the independent assay score of each of the plurality of assays to obtain sensitivity index and thereby predicting the response of the subject to the drug(s); and
g. optionally, correlating the sensitivity index with clinical response of the subject to the drug(s).

10. A method of screening or developing anti-cancer agent, said method comprising acts of:

a. culturing subject's tumor tissue on tumor microenvironment platform as claimed in claim 5, to obtain cultured tumor tissue; and
b. treating the cultured tumor tissue with the agent, assessing tumor response to the agent by assay to determine effect of said agent on the tumor cell.

11. A method for screening tumor cells for specific markers, said method comprising act of:

a. culturing subject's tumor tissue on tumor microenvironment platform as claimed in claim 5, to obtain cultured tumor tissue;
b. treating the cultured tumor tissue with drug(s) and assessing tumor response to the drug by assay; and
c. conducting microarray and Nucleic Acid analysis to screen for the biomarkers.

12. The Extra Cellular Matrix, ECM, composition as claimed in claim 1, wherein the Extra Cellular Matrix, ECM, composition is tumor specific; and wherein the collagen 1 is at concentration ranging from about 0.01 μg/ml to about 100 μg/ml, preferably at about 5 μg/ml or about 20 μg/ml or about 50 μg/ml; the collagen 3 is at concentration ranging from about 0.01 μg/ml to about 100 μg/ml, preferably at about 0.1 μg/ml or about 1 μg/ml or about 100

μg/ml; the collagen 4 is at concentration ranging from about 0.01 μg/ml to about 500 μg/ml, preferably at about 5 μg/ml or about 20 μg/ml or about 250 μg/ml; the collagen 6 is at concentration ranging from about 0.01 μg/ml to about 500 μg/ml, preferably at about 0.1 μg/ml or about 1 μg/ml or about 10 μg/ml; the Fibronectin is at concentration ranging from about 0.01 μg/ml to about 750 μg/ml, preferably at about 5 μg/ml or about 20 μg/ml or about 500 μg/ml; the Vitronectin is at concentration ranging from about 0.01 μg/ml to about 95 μg/ml, preferably at about 5 μg/ml or about 10 μg/ml; the Cadherin is at concentration ranging from about 0.01 μg/ml to about 500 μg/ml, preferably at about 1 μg/ml and about 5 μg/ml; the Filamin A is at concentration ranging from about 0.01 μg/ml to about 500 μg/ml, preferably at about 5 μg/ml or about 10 μg/ml; the Vimentin is at concentration ranging from about 0.01 μg/ml to about 100 μg/ml, preferably at about 1 μg/ml or about 10 μg/ml; the Laminin is at concentration ranging from about 0.01 μg/ml to about 100 μg/ml, preferably at about 5 μg/ml or about 10 μg/ml or about 20 μg/ml; the Decorin is at concentration ranging from about 0.01 μg/ml to about 100 μg/ml, preferably at about 10 μg/ml or about 20 μg/ml; the Tenascin C is at concentration ranging from about 0.01 μg/ml to about 500 μg/ml, preferably at about 10 μg/ml or about 25 μg/ml; and the Osteopontin is at concentration ranging from about 0.01 μg/ml to about 150 μg/ml, preferably at about 1 μg/ml or about 5 μg/ml.

**13.** The methods as claimed in claims 6 to 11, wherein said tumor tissue is obtained from source selected from group comprising central nervous system, bone marrow, blood, spleen, thymus, heart, mammary gland, liver, pancreas, thyroid, skeletal muscle, kidney, lung, intestine, stomach, oesophagus, ovary, bladder, testis, uterus, stromal tissue and connective tissue or any combinations thereof; and wherein the tumor or the tumor tissue is obtained surgically or by biopsy or as xenograft or any combinations thereof; and the tumor or the tumor tissue is divided into small pieces of about 100 μm to about 3000 μm sections.

**14.** The tumor microenvironment as claimed in claim 5 wherein the tumor tissue is obtained from source selected from group comprising central nervous system, bone marrow, blood, spleen, thymus, heart, mammary gland, liver, pancreas, thyroid, skeletal muscle, kidney, lung, intestine, stomach, oesophagus, ovary, bladder, testis, uterus, stromal tissue and connective tissue or any combinations thereof; wherein the tumor or the tumor tissue is obtained surgically or by biopsy or as xenograft or any combinations thereof; and the tumor or the tumor tissue is divided into small pieces of about 100 μm to about 3000 μm sections; wherein the culturing of the tumor tissue is carried out at temperature ranging from about 30°C to about 40°C, preferably about 37°C; for time duration of about 2 to 10 days, preferably about 3 to 7 days; and about 5% $CO_2$; and wherein the coating platform is selected from group comprising plate, base, flask, dish, petriplate and petridish.

**15.** The tumor microenvironment platform as claimed in claim 5 wherein said microenvironment platform is for maintaining signaling networks of tumor cell; and for maintaining an intact tissue micro-environment, cellular architecture and integrity of tumor stroma interaction.

**16.** The tumor microenvironment as claimed in claim 5 wherein the culture medium is selected from group comprising Dulbecco's Modified Eagle Medium, DMEM, or 15 RPMI1640, Roswell Park Memorial Institute Medium, at concentration ranging from about 60% to about 100%, preferably about 80% ; wherein the culture medium optionally further comprises heat inactivated Foetal Bovine Serum, PBS, at concentration ranging from about 0.1% to about 40%, preferably about 2% wt/wt; Penicillin-Streptomycin at concentration ranging from about 1% to about 2%, preferably about 1% wt/wt; sodium pyruvate at concentration ranging from about 10mM to about 500mM, preferably about 100mM; nonessential amino acid is L-glutamine at concentration ranging from about lmM to about 10mM, preferably about 5mM; and HEPES ((4-(2-hydroxyethyl)-1-piperazineethanesulfonic acid) at concentration ranging from about lmM to about 20mM, preferably about 10mM or any combination thereof; and the serum in the culture medium is at concentration ranging from about 0.1 % to about 10%, preferably about 2%.

**17.** The methods as claimed in claims 6 to 11, wherein the tumor is selected from group comprising stomach, colon, head & neck, brain, oral cavity, breast, gastric, gastrointestinal, oesophageal, colorectal, pancreatic, lung, liver, kidney, ovarian, uterine, bone, prostate, testicular, glioblastoma, astrocytoma, melanoma, thyroid, bladder, non-small cell lung, small cell lung, haemotological cancers including AML, Acute Myeloid Leukemia, CML, Chronic Myelogenous Leukemia, ALL, Acute Lymphocytic Leukemia, TALL, T-cell Acute Lymphoblastic Leukemia, NHL, Non-Hodgkins Lymphoma, DBCL, Diffuse B-cell Lymphoma, CLL, Chronic Lymphocytic Leukemia, and multiple myeloma or any combinations thereof.

**18.** The methods as claimed in claims 8 to 11, wherein the assay is selected from group comprising assay for cell viability, cell death, cell proliferation, tumor morphology, tumor stroma content, cell metabolism, senescence or any combinations thereof; and wherein the assay for the cell viability and the cell metabolism is selected from group

comprising WST assay, ATP uptake assay and glucose uptake assay; the assay for the cell death is selected from group comprising LDH assay, Activated Caspase 3 assay, Activated Caspase 8 assay and Nitric Oxide Synthase assay, TUNEL; the assay for the cell proliferation is selected from group comprising Ki67 assay, ATP/ADP ratio assay and glucose uptake assay; and the assay for the tumor morphology and the tumor stroma is H&E, Haemaotxylin & Eosin staining; or any combinations thereof.

**19.** The methods as claimed in claims 8 and 9, wherein the method is used for deciding treatment for the subject from group comprising chemotherapy, targeted therapy, surgery, radiation or any combinations thereof; and wherein the sensitivity index correlates to complete clinical response, partial clinical response and no clinical response when the sensitivity index is greater than 60, between 20 to 60 and less than 20 respectively.

**20.** The method as claimed in claim 9, wherein the assigning a weightage score for each of the plurality of assays is based on nature of the drug used.

**21.** The method as claimed in claim 11, wherein the microarray and the Nucleic Acid analysis of DNA, RNA or micro RNA is carried out to detect pathway modulation before and after the drug treatment; and wherein the microarray and the Nucleic Acid analysis is confirmed using assay selected from group comprising Real-time PCR, RTPCR, Immunohistochemical, IHC, analysis and phospho-proteomic profiling.

## Patentansprüche

**1.** Zusammensetzung einer extrazellulären Matrix (ECM), folgende Bestandteile aufweisend - Kollagen 1, Kollagen 3, Kollagen 4, Kollagen 6, Fibronektin, Vibronektin, Cadherin, Filamin A, Vimentin, Osteopontin, Laminin, Dekorin und Tenascin C.

**2.** ECM-Zusammensetzung nach Anspruch 1, darüber hinaus einen oder mehrere Bestandteile aufweisend, der bzw. die aus der Gruppe ausgewählt ist bzw. sind, die aus Basalmembranproteinen, Zytoskelett-Proteinen und Matrixproteinen besteht.

**3.** Verfahren zum Erhalten einer Zusammensetzung einer extrazellulären Matrix (ECM) nach Anspruch 1, wobei das Verfahren umfasst,
die Bestandteile der ECM - Kollagen 1, Kollagen 3, Kollagen 4, Kollagen 6, Fibronektin, Vibronektin, Cadherin, Filamin A, Vimentin, Osteopontin, Laminin, Dekorin, Tenascin C zu kombinieren, um die ECM-Zusammensetzung zu erhalten.

**4.** Verfahren nach Anspruch 3, wobei die Bestandteile darüber hinaus einen oder mehrere Bestandteile aufweisen, der bzw. die aus der Gruppe ausgewählt ist bzw. sind, die aus Basalmembranproteinen, Zytoskelett-Proteinen und Matrixproteinen besteht.

**5.** Tumor-Mikroumgebungsplattform zum Kultivieren von Tumorgewebe, wobei die Mikroumgebung die ECM-Zusammensetzung nach Anspruch 1 oder 2, Kulturmedium, optional zusammen mit Serum, Plasma oder PBMCs und ein Medikament umfasst.

**6.** Verfahren zum Erhalten einer Tumor-Mikroumgebungsplattform zum Kultivieren von Tumorgewebe nach Anspruch 5, wobei das Verfahren einen Vorgang umfasst, die Plattform mit einer ECM-Zusammensetzung nach Anspruch 1 oder 2 zu beschichten und Kulturmedium, optional zusammen mit Serum, Plasma oder PBMCs und einem Medikament, der Plattform zuzusetzen, um die Tumor-Mikroumgebungsplattform zu erhalten.

**7.** Verfahren zum organtypischen Kultivieren von Tumorgewebe, wobei das Verfahren einen Vorgang umfasst, das Tumorgewebe auf einer Tumor-Mikroumgebungsplattform nach Anspruch 5 zu kultivieren, um die organtypische Kultur zu erhalten.

**8.** Verfahren zum Vorhersagen des Ansprechens einer tumorbefallenen Person auf Medikament(e), wobei das Verfahren Vorgänge umfasst, die darin bestehen:

a. das Tumorgewebe der Person auf einer Tumor-Mikroumgebungsplattform nach Anspruch 5 zu kultivieren, um kultiviertes Tumorgewebe zu erhalten;

b. das kultivierte Tumorgewebe mit dem (den) Medikament(en) zu behandeln und einen Test durchzuführen;
c. das Testergebnis in eine numerische Metrik umzusetzen, um einen Sensitivitätsindex zu erhalten und dadurch das Ansprechen der Person auf das (die) Medikament(e) vorherzusagen; und
d. optional den Sensitivitätsindex in Korrelation zum klinischen Ansprechen der Person auf das (die) Medikament(e) zu setzen.

**9.** Verfahren zum Vorhersagen des Ansprechens einer tumorbefallenen Person auf Medikament(e), wobei das Verfahren Vorgänge umfasst, die darin bestehen:

a. das Tumorgewebe der Person auf einer Tumor-Mikroumgebungsplattform nach Anspruch 5 zu kultivieren, um kultiviertes Tumorgewebe zu erhalten;
b. das kultivierte Tumorgewebe mit dem (den) Medikament(en) zu behandeln;
c. das Tumoransprechen auf das Medikament durch mehrere Tests zu bewerten, um ein Bewertungsergebnis für jeden der mehreren Tests zu erhalten;
d. ein Gewichtungsergebnis für jeden der mehreren Tests zuzuteilen;
e. das Bewertungsergebnis jedes der mehreren Tests mit dem Gewichtungsergebnis eines entsprechenden Tests der mehreren Tests zu multiplizieren, um ein unabhängiges Testergebnis für jeden der mehreren Tests zu erhalten;
f. das unabhängige Testergebnis jedes der mehreren Tests zu kombinieren, um einen Sensitivitätsindex zu erhalten und dadurch das Ansprechen der Person auf das (die) Medikament(e) vorherzusagen; und
g. optional den Sensitivitätsindex in Korrelation zum klinischen Ansprechen der Person auf das (die) Medikament(e) zu setzen.

**10.** Verfahren zum Screening oder Entwickeln eines Antikrebsmittels, wobei das Verfahren Vorgänge umfasst, die darin bestehen:

a. das Tumorgewebe einer Person auf einer Tumor-Mikroumgebungsplattform nach Anspruch 5 zu kultivieren, um kultiviertes Tumorgewebe zu erhalten; und
b. das kultivierte Tumorgewebe mit dem Mittel zu behandeln, das Tumoransprechen auf das Mittel durch einen Test zu bewerten, um die Wirkung des Mittels auf die Tumorzelle zu bestimmen.

**11.** Verfahren zum Screening von Tumorzellen auf spezifische Marker hin, wobei das Verfahren Vorgänge umfasst, die darin bestehen:

a. das Tumorgewebe einer Person auf einer Tumor-Mikroumgebungsplattform nach Anspruch 5 zu kultivieren, um kultiviertes Tumorgewebe zu erhalten;
b. das kultivierte Tumorgewebe mit Medikament(en) zu behandeln und das Tumoransprechen auf das Medikament durch einen Test zu bewerten; und
c. eine Mikroarray- und Nukleinsäureanalyse zum Screening auf die Biomarker hin durchzuführen.

**12.** Zusammensetzung einer extrazellulären Matrix (ECM) nach Anspruch 1, wobei die Zusammensetzung der extrazellulären Matrix (ECM) tumorspezifisch ist; und wobei das Kollagen 1 in einer Konzentration, die von ca. 0,01 μg/ml bis ca. 100 μg/ml reicht, vorzugsweise in ca. 5 μg/ml oder ca. 20 μg/ml oder ca. 50 μg/ml vorliegt; das Kollagen 3 in einer Konzentration, die von ca. 0,01 μg/ml bis ca. 100 μg/ml reicht, vorzugsweise in ca. 0,1 μg/ml oder ca. 1 μg/ml oder ca. 100 μg/ml vorliegt; das Kollagen 4 in einer Konzentration, die von ca. 0,01 μg/ml bis ca. 500 μg/ml reicht, vorzugsweise in ca. 5 μg/ml oder ca. 20 μg/ml oder ca. 250 μg/ml vorliegt; das Kollagen 6 in einer Konzentration, die von ca. 0,01 μg/ml bis ca. 500 μg/ml reicht, vorzugsweise in ca. 0,1 μg/ml oder ca. 1 μg/ml oder ca. 10 μg/ml vorliegt; das Fibronektin in einer Konzentration, die von ca. 0,01 μg/ml bis ca. 750 μg/ml reicht, vorzugsweise in ca. 5 μg/ml oder ca. 20 μg/ml oder ca. 500 μg/ml vorliegt; das Vitronektin in einer Konzentration, die von ca. 0,01 μg/ml bis ca. 95 μg/ml reicht, vorzugsweise in ca. 5 μg/ml oder ca. 10 μg/ml vorliegt; das Cadherin in einer Konzentration, die von ca. 0,01 μg/ml bis ca. 500 μg/ml reicht, vorzugsweise in ca. 1 μg/ml und ca. 5 μg/ml vorliegt; das Filamin A in einer Konzentration, die von ca. 0,01 ug/ml bis ca. 500 μg/ml reicht, vorzugsweise in ca. 5 μg/ml oder ca. 10 μg/ml vorliegt; das Vimentin in einer Konzentration, die von ca. 0,01 μg/ml bis ca. 100 μg/ml reicht, vorzugsweise in ca. 1 ug/ml oder ca. 10 μg/ml vorliegt; das Laminin in einer Konzentration, die von ca. 0,01 μg/ml bis ca. 100 μg/ml reicht, vorzugsweise in ca. 5 μg/ml oder ca. 10 μg/ml oder ca. 20 μg/ml vorliegt; das Dekorin in einer Konzentration, die von ca. 0,01 μg/ml bis ca. 100 μg/ml reicht, vorzugsweise in ca. 10 μg/ml oder ca. 20 μg/ml vorliegt; das Tenascin C in einer Konzentration, die von ca. 0,01 μg/ml bis ca. 500 μg/ml reicht, vorzugsweise in ca. 10 μg/ml oder ca. 25 μg/ml vorliegt; und das Osteopontin in einer Konzentration, die von ca. 0,01 μg/ml bis ca.

150 μg/ml reicht, vorzugsweise in ca. 1 μg/ml oder ca. 5 μg/ml vorliegt.

**13.** Verfahren nach einem der Ansprüche 6 bis 11, wobei das Tumorgewebe aus einer Quelle gewonnen wird, die aus der Gruppe ausgewählt ist, die das zentrale Nervensystem, Knochenmark, Blut, Milz, Thymus, Herz, Brustdrüse, Leber, Pankreas, Schilddrüse, Skelettmuskel, Niere, Lunge, Darm, Magen, Oesophagus, Eierstock, Blase, Hoden, Uterus, Stroma und Bindegewebe oder irgendwelche Kombinationen davon umfasst; und wobei der Tumor oder das Tumorgewebe chirurgisch oder durch Biopsie oder als Xenotransplantat oder irgendwelche Kombinationen davon gewonnen wird; und der Tumor oder das Tumorgewebe in kleine Stücke von Abschnitten von ca. 100 μm bis ca. 3000 μm geteilt wird.

**14.** Tumor-Mikroumgebung nach Anspruch 5, wobei das Tumorgewebe aus einer Quelle gewonnen wird, die aus der Gruppe ausgewählt ist, die das zentrale Nervensystem, Knochenmark, Blut, Milz, Thymus, Herz, Brustdrüse, Leber, Pankreas, Schilddrüse, Skelettmuskel, Niere, Lunge, Darm, Magen, Oesophagus, Eierstock, Blase, Hoden, Uterus, Stroma und Bindegewebe oder irgendwelche Kombinationen davon umfasst; und wobei der Tumor oder das Tumorgewebe chirurgisch oder durch Biopsie oder als Xenotransplantat oder irgendwelche Kombinationen davon gewonnen wird; und der Tumor oder das Tumorgewebe in kleine Stücke von Abschnitten von ca. 100 μm bis ca. 3000 μm geteilt wird; wobei das Kultivieren des Tumorgewebes bei einer Temperatur, die von ca. 30°C bis ca. 40°C, vorzugsweise ca. 37°C, über eine Zeitdauer von ca. 2 bis 10 Tagen, vorzugsweise ca. 3 bis 7 Tagen; und ca. 5% $CO_2$ erfolgt; und wobei der Beschichtungsträger aus der Gruppe ausgewählt ist, die eine Platte, eine Unterlage, einen Kolben, eine Schale, eine Petriplatte und eine Petrischale umfasst.

**15.** Tumor-Mikroumgebungsplattform nach Anspruch 5, wobei die Mikroumgebungsplattform dazu bestimmt ist, Tumorzellen-Signalgebungsnetzwerke aufrechtzuerhalten; und eine intakte Gewebe-Mikroumgebung, Zellarchitektur und Integrität einer Tumorstromainteraktion aufrechtzuerhalten.

**16.** Tumor-Mikroumgebung nach Anspruch 5, wobei das Kulturmedium aus der Gruppe ausgewählt ist, die Dulbecco's Modified Eagle Medium, DMEM, oder 15 RPM1640, Roswell Park Memorial Institute Medium in einer Konzentration umfasst, die von ca. 60% bis ca. 100%, vorzugsweise ca. 80% umfasst; wobei das Kulturmedium darüber hinaus optional wärmeinaktiviertes fötales Kälberserum, PBS, in einer Konzentration, die von ca. 0,1% bis ca. 40% reicht, vorzugsweise ca. 2% pro Gewicht; Penicillin-Streptomycin in einer Konzentration, die von ca. 1% bis ca. 2% reicht, vorzugsweise ca. 1 % pro Gewicht; Natriumpyruvat in einer Konzentration, die von ca. 10 mM bis ca. 500 mM reicht, vorzugsweise 100 mM; es sich bei nicht essentieller Aminosäure um L-Glutamin in einer Konzentration handelt, die von ca. 1 mM bis ca. 10 mM reicht, vorzugsweise ca. 5 mM; und HEPES ((4-(2-Hydroxyethyl)-1-piperazinethansulfonsäure) in einer Konzentration, die von ca. 1 mM bis ca. 20 mM reicht, vorzugsweise ca. 10 mM oder irgendeine Kombination davon umfasst; und das Serum in dem Kulturmedium in einer Konzentration, die von ca. 0,1% bis ca. 10% reicht, vorzugsweise ca. 2% vorliegt.

**17.** Verfahren nach einem der Ansprüche 6 bis 11, wobei der Tumor aus der Gruppe ausgewählt ist, die Magen-, Darm-, Kopf/Hals-, Gehirn-, Mundhöhlen-, Brustkrebs, gastrischen, gastrointestinalen, oesophagalen, kolorektalen Krebs, Pankreas-, Lungen-, Leber-, Nieren-, Eierstock-, Gebärmutter-, Knochen-, Prostata-, Hodenkrebs, Glioblastom, Astrozytom, Melanom, Schilddrüsen-, Blasenkrebs, nicht kleinzelliges Lungenkarzinom, kleinzelliges Lungenkarzinom, Blutkrebsarten einschließlich AML, akute myeloische Leukämie, CML, chronische myeloische Leukämie, ALL, akute lymphatische Leukämie, TALL, akute lymphoblastische T-Zellen-Leukämie, NHL, Non-Hodgkin-Lymphom, DBCL, diffuses B-Zellen-Lymphom, CLL, chronische Lymphozitenleukämie and multiples Myelom oder irgendwelche Kombinationen davon umfasst.

**18.** Verfahren nach einem der Ansprüche 8 bis 11, wobei der Test aus der Gruppe ausgewählt ist, die Tests auf Zellviabilität, Zelltod, Zellproliferation, Tumormorphologie, Tumorstromagehalt, Zellmetabolismus, Alterung oder irgendwelche Kombinationen davon umfasst; und wobei der Test auf Zellviabilität und Zellmetabolismus aus der Gruppe ausgewählt ist, die einen WST-Test, einen ATP-Aufnahme- und einen Glukoseaufnahmetest umfasst; der Test auf Zelltod aus der Gruppe ausgewählt ist, die einen LDH-Test, einen Test mit aktivierter Caspase 2, einen Test mit aktivierter Caspase 8 und einen Stickoxid-Synthasetest, TUNEL, umfasst; der Test auf Zellproliferation aus der Gruppe ausgewählt ist, die einen Ki67-Test, einen ATP/ADP-Verhältnistest und einen Glukoseaufnahmetest umfasst; und es sich bei dem Test auf Tumormorphologie und Tumorstroma um H&E, Haematoxylin-Eosin-Färbung handelt; oder irgendwelche Kombinationen davon umfasst.

**19.** Verfahren nach einem der Ansprüche 8 und 9, wobei das Verfahren dazu verwendet wird, eine Behandlung für die Person aus der Gruppe zu bestimmen, die Chemotherapie, gezielte Therapie, Operation, Bestrahlung oder irgendwelche Kombinationen davon umfasst; und wobei der Sensitivitätsindex in Korrelation zu einem kompletten klinischen Ansprechen, einem teilweisen klinische Ansprechen und keinem klinischen Ansprechen steht, wenn der Sensitivitätsindex größer ist als 60, zwischen 20 und 60 liegt, bzw. kleiner als 20 ist.

**20.** Verfahren nach Anspruch 9, wobei das Zuteilen eines Gewichtungsergebnisses für jeden der mehreren Test auf der Art des verwendeten Medikaments beruht.

**21.** Verfahren nach Anspruch 11, wobei die Mikroarray- und Nukleinsäureanalyse von DNA, RNA oder Mikro-RNA zum Detektieren einer Verlaufsmodulation vor und nach der Medikamentenbehandlung durchgeführt wird; und wobei die Mikroarry- und Nukleinsäureanalyse anhand eines Tests bestätigt wird, der aus der Gruppe ausgewählt ist, die Echtzeit-PCR, RTPCR, immunhistochemische, IHC, Analyse und Phosphoproteomik-Profilierung umfasst.

**Revendications**

**1.** Composition de matrice extracellulaire (MEC) comprenant les composants : collagène 1, collagène 3, collagène 4, collagène 6, fibronectine, vitronectine, cadhérine, filamine A, vimentine, ostéopontine, laminine, décorine, et ténascine C.

**2.** La composition MEC telle que revendiquée dans la revendication 1, comprenant en outre un ou plusieurs composants sélectionnés dans le groupe constitué par les protéines de membrane basale, les protéines cytosquelettiques et les protéines de matrice.

**3.** Procédé destiné à obtenir une composition de matrice extracellulaire (MEC) telle que revendiquée dans la revendication 1, ledit procédé comprenant la combinaison des composants de la MEC - collagène 1, collagène 3, collagène 4, collagène 6, fibronectine, vitronectine, cadhérine, filamine A, vimentine, ostéopontine, laminine, décorine, ténascine C pour obtenir la composition MEC.

**4.** Le procédé tel que revendiqué dans la revendication 3, sachant que les composants comprennent en outre un ou plusieurs composants sélectionnés dans le groupe constitué par les protéines de membrane basale, les protéines cytosquelettiques et les protéines de matrice.

**5.** Plateforme à microenvironnement tumoral pour la mise en culture de tissu tumoral, ledit microenvironnement comprenant la composition MEC telle que revendiquée dans la revendication 1 ou 2, un milieu de culture facultativement avec du sérum, du plasma ou des PBMC et un médicament.

**6.** Procédé destiné à obtenir une plateforme à microenvironnement tumoral pour la mise en culture de tissu tumoral telle que revendiquée dans la revendication 5, ledit procédé comprenant une opération consistant à revêtir la plateforme avec une composition MEC telle que revendiquée dans la revendication 1 ou 2 et à ajouter un milieu de culture facultativement avec du sérum, du plasma ou des PBMC et un médicament à la plateforme pour obtenir la plateforme à microenvironnement tumoral.

**7.** Procédé de mise en culture organotypique de tissu tumoral, ledit procédé comprenant une opération de mise en culture du tissu tumoral sur une plateforme à microenvironnement tumoral telle que revendiquée dans la revendication 5 pour obtenir la culture organotypique.

**8.** Procédé de prédiction d'une réponse d'un sujet tumoral à un (des) médicament(s), ledit procédé comprenant les opérations consistant à :

a. mettre en culture le tissu tumoral du sujet sur une plateforme à microenvironnement tumoral telle que revendiquée dans la revendication 5 pour obtenir du tissu tumoral en culture ;
b. traiter le tissu tumoral en culture avec le(s) médicament(s) et effectuer un test ;
c. convertir le relevé du test en métrique numérique pour obtenir un indice de sensibilité et, de la sorte, prédire la réponse du sujet au(x) médicament(s) ; et
d. facultativement, mettre en corrélation l'indice de sensibilité avec une réponse clinique du sujet au(x) médicament(s).

**9.** Procédé de prédiction d'une réponse d'un sujet tumoral à un (des) médicament(s), ledit procédé comprenant les

opérations consistant à :

a. mettre en culture le tissu tumoral du sujet sur une plateforme à microenvironnement tumoral telle que revendiquée dans la revendication 5 pour obtenir du tissu tumoral en culture ;
b. traiter le tissu tumoral en culture avec le(s) médicament(s) ;
c. évaluer la réponse tumorale au médicament par une pluralité de tests pour obtenir un résultat d'évaluation pour chacun de la pluralité de tests ;
d. assigner un résultat de pondération pour chacun de la pluralité de tests ;
e. multiplier le résultat d'évaluation de chacun de la pluralité de tests par le résultat de pondération du test correspondant de la pluralité de tests pour obtenir un résultat de test indépendant pour chacun de la pluralité de tests ;
f. combiner le résultat de test indépendant de chacun de la pluralité de tests pour obtenir un indice de sensibilité et prédire de la sorte la réponse du sujet au(x) médicament(s) ; et
g. facultativement, mettre en corrélation l'indice de sensibilité avec une réponse clinique du sujet au(x) médicament(s).

**10.** Procédé de dépistage ou de développement d'un agent anticancer, ledit procédé comprenant les opérations consistant à :

a. mettre en culture du tissu tumoral du sujet sur une plateforme à microenvironnement tumoral telle que revendiquée dans la revendication 5 pour obtenir du tissu tumoral en culture ; et
b. traiter le tissu tumoral en culture avec l'agent, évaluer la réponse tumorale à l'agent par test pour déterminer l'effet dudit agent sur la cellule tumorale.

**11.** Procédé de dépistage de cellules tumorales pour identifier des marqueurs spécifiques, ledit procédé comprenant les opérations consistant à :

a. mettre en culture du tissu tumoral du sujet sur une plateforme à microenvironnement tumoral telle que revendiquée dans la revendication 5 pour obtenir du tissu tumoral en culture ;
b. traiter le tissu tumoral en culture avec un (des) médicament(s) et évaluer la réponse tumorale au médicament par test ; et
c. effectuer une analyse par puce et acide nucléique pour dépister les biomarqueurs.

**12.** La composition de matrice extracellulaire, MEC, telle que revendiquée dans la revendication 1, sachant que la composition de matrice extracellulaire, MEC, est spécifique de tumeur ; et sachant que le collagène 1 est présent dans une concentration allant d'environ 0,01 μg/ml à environ 100 μg/ml et qui est de préférence d'environ 5 μg/ml ou d'environ 20 μg/ml ou d'environ 50 μg/ml ; le collagène 3 est présent dans une concentration allant d'environ 0,01 μg/ml à environ 100 μg/ml et qui est de préférence d'environ 0,1 μg/ml ou d'environ 1 μg/ml ou d'environ 100 μg/ml ; le collagène 4 est présent dans une concentration allant d'environ 0,01 μg/ml à environ 500 μg/ml et qui est de préférence d'environ 5 μg/ml ou d'environ 20 μg/ml ou d'environ 250 μg/ml ; le collagène 6 est présent dans une concentration allant d'environ 0,01 μg/ml à environ 500 μg/ml et qui est de préférence d'environ 0,1 μg/ml ou d'environ 1 μg/ml ou d'environ 10 μg/ml ; la fibronectine est présente dans une concentration allant d'environ 0,01 μg/ml à environ 750 μg/ml et qui est de préférence d'environ 5 μg/ml ou d'environ 20 μg/ml ou d'environ 500 μg/ml ; la vitronectine est présente dans une concentration allant d'environ 0,01 μg/ml à environ 95 μg/ml et qui est de préférence d'environ 5 μg/ml ou d'environ 10 μg/ml ; la cadhérine est présente dans une concentration allant d'environ 0,01 μg/ml à environ 500 μg/ml et qui est de préférence d'environ 1 μg/ml ou d'environ 5 μg/ml ; la filamine A est présente dans une concentration allant d'environ 0,01 μg/ml à environ 500 μg/ml et qui est de préférence d'environ 5 μg/ml ou d'environ 10 μg/ml ; la vimentine est présente dans une concentration allant d'environ 0,01 μg/ml à environ 100 μg/ml et qui est de préférence d'environ 1 μg/ml ou d'environ 10 μg/ml; la laminine est présente dans une concentration allant d'environ 0,01 μg/ml à environ 100 μg/ml et qui est de préférence d'environ 5 μg/ml ou d'environ 10 μg/ml ou d'environ 20 μg/ml ; la décorine est présente dans une concentration allant d'environ 0,01 μg/ml à environ 100 μg/ml et qui est de préférence d'environ 10 μg/ml ou d'environ 20 μg/ml; la ténascine C est présente dans une concentration allant d'environ 0,01 μg/ml à environ 500 μg/ml et qui est de préférence d'environ 10 μg/ml ou d'environ 25 μg/ml ; et l'ostéopontine est présente dans une concentration allant d'environ 0,01 μg/ml à environ 150 μg/ml et qui est de préférence d'environ 1 μg/ml ou d'environ 5 μg/ml.

**13.** Les procédés tels que revendiqués dans les revendications 6 à 11, sachant que ledit tissu tumoral est obtenu à partir d'une source sélectionnée dans le groupe constitué par le système nerveux central, la moelle osseuse, le

sang, la rate, le thymus, le coeur, la glande mammaire, le foie, le pancréas, la thyroïde, le muscle squelettique, le rein, le poumon, l'intestin, l'estomac, l'oesophage, l'ovaire, la vessie, le testicule, l'utérus, le tissu stromal et le tissu conjonctif ou toute combinaison de ceux-ci ; et sachant que la tumeur ou le tissu tumoral est obtenu(e) chirurgiquement ou par biopsie ou comme xénogreffe ou toute combinaison de ceux-ci ; et la tumeur ou le tissu tumoral est divisé(e) en petits morceaux d'environ 100 $\mu$m à environ 3000 $\mu$m de section.

**14.** Le microenvironnement tumoral tel que revendiqué dans la revendication 5, sachant que le tissu tumoral est obtenu à partir d'une source sélectionnée dans le groupe constitué par le système nerveux central, la moelle osseuse, le sang, la rate, le thymus, le coeur, la glande mammaire, le foie, le pancréas, la thyroïde, le muscle squelettique, le rein, le poumon, l'intestin, l'estomac, l'oesophage, l'ovaire, la vessie, le testicule, l'utérus, le tissu stromal et le tissu conjonctif ou toute combinaison de ceux-ci ; et sachant que la tumeur ou le tissu tumoral est obtenu(e) chirurgiquement ou par biopsie ou comme xénogreffe ou toute combinaison de ceux-ci ; et la tumeur ou le tissu tumoral est divisé(e) en petits morceaux d'environ 100 $\mu$m à environ 3000 $\mu$m de section ; sachant que la mise en culture du tissu tumoral est effectuée à une température allant d'environ 30°C à environ 40°C et qui est de préférence d'environ 37°C ; pendant une durée d'environ 2 à 10 jours, de préférence d'environ 3 à 7 jours ; et environ 5 % de $CO_2$ ; et sachant que la plateforme support est sélectionnée dans le groupe comprenant une plaque, une embase, une flasque, une boîte, une plaque de Petri et une boîte de Petri.

**15.** La plateforme à microenvironnement tumoral telle que revendiquée dans la revendication 5, sachant que ladite plateforme à microenvironnement est destinée à maintenir des réseaux de signalisation de cellule tumorale ; et à maintenir une architecture cellulaire de microenvironnement de tissu intacte et une intégrité de l'interaction du stroma tumoral.

**16.** Le microenvironnement tumoral tel que revendiqué dans la revendication 5, sachant que le milieu de culture est sélectionné dans le groupe constitué par le milieu Eagle modifié de Dulbecco, le DMEM, ou le 15 RPMI1640, le milieu de Roswell Park Memorial Institute, dans une concentration allant d'environ 60% à environ 100% et qui est de préférence d'environ 80% ; sachant que le milieu de culture comprend en outre facultativement un sérum bovin foetal, PBS, inactivé par de la chaleur, dans une concentration allant d'environ 0,1% à environ 40% et qui est de préférence d'environ 2% en poids ; de la streptomycine de pénicilline dans une concentration allant d'environ 1% à environ 2% et qui est de préférence d'environ 1% en poids ; du pyruvate de sodium dans une concentration allant d'environ 10mM à environ 500mM et qui est de préférence d'environ 100mM ; de l'acide aminé non essentiel est de la glutamine L dans une concentration allant d'environ 1mM à environ 10mM et qui est de préférence d'environ 5mM ; et HEPES (acide 4-(2-hydroxyéthyl)-1-pipérazineéthanesulfonique) dans une concentration allant d'environ 1mM à environ 20mM et qui est de préférence d'environ 10mM ou toute combinaison de ceux-ci ; et le sérum dans le milieu de culture est présent dans une concentration allant d'environ 0,1% à environ 10% et qui est de préférence d'environ 2%.

**17.** Les procédés tels que revendiqués dans les revendications 6 à 11, sachant que la tumeur est sélectionnée dans le groupe constitué par les cancers de l'estomac, du colon, de la tête et de la nuque, du cerveau, de la cavité buccale, du sein, gastrique, gastrointestinal, de l'oesophage, colorectal, pancréatique, du poumon, du foie, du rein, de ovaire, de l'utérus, des os, de la prostate, des testicules, le glioblastome, l'astrocytome, le mélanome, les cancers de la thyroïde, de la vessie, du poumon non à petites cellules, du poumon à petites cellules, hématologiques incluant la leucémie myéloïde aiguë (LMA), la leucémie myélogène chronique (LMC), la leucémie lymphocytaire aiguë (LLA), la leucémie lymphoblastique aiguë à cellules T (LALT), le lymphome non hodgkinien (LNH), le lymphome diffus à cellules B (LDCB), la leucémie lymphocytaire chronique (LLC), et le myélome multiple ou toute combinaison de ceux-ci.

**18.** Les procédés tels que revendiqués dans les revendications 8 à 11, sachant que le test est sélectionné dans le groupe comprenant le test de viabilité de cellules, mort cellulaire, prolifération cellulaire, morphologie tumorale, teneur de stroma tumoral, métabolisme cellulaire, sénescence ou toute combinaison de ceux-ci ; et sachant que le test de viabilité cellulaire et de métabolisme cellulaire est sélectionné dans le groupe comprenant le test WST, le test d'apport d'ATP et le test d'apport de glucose ; le test de mort cellulaire est sélectionné dans le groupe comprenant le test LDH, le test de caspase 3 activée, le test de caspase 8 activée et le test de synthase d'oxyde nitrique, TUNEL ; le test de prolifération cellulaire est sélectionné dans le groupe comprenant le test Ki67, le test de rapport ATP/ADP et le test d'apport de glucose ; et le test de morphologie tumorale et de stroma tumoral est l'H&E, la coloration à l'hématoxyline et à l'éosine ; ou toute combinaison de ceux-ci.

**19.** Les procédés tels que revendiqués dans les revendications 8 et 9, sachant que le procédé est utilisé pour décider

d'un traitement pour le sujet à partir du groupe comprenant la chimiothérapie, la thérapie ciblée, la chirurgie, le rayonnement ou toute combinaison de ceux-ci ; et sachant que l'indice de sensibilité correspond à une réponse clinique complète, une réponse clinique partielle et une absence de réponse clinique lorsque l'indice de sensibilité est supérieur à 60, compris entre 20 et 60 et inférieur à 20, respectivement.

**20.** Le procédé tel que revendiqué dans la revendication 9, sachant que l'assignation d'un résultat de pondération pour chacun de la pluralité de tests est basée sur la nature du médicament utilisé.

**21.** Le procédé tel que revendiqué dans la revendication 11, sachant que l'analyse par puce et acide nucléique d'ADN, d'ARN ou de micro-ARN est effectuée pour détecter une modulation de voie avant et après le traitement par médicament ; et sachant que l'analyse d'acide nucléique par puce est confirmée au moyen d'un test sélectionné dans le groupe comprenant le PCR en temps réel, le RTPCR, le test immunohistochimique, l'IHC, l'analyse et le profilage phosphoprotéomique.

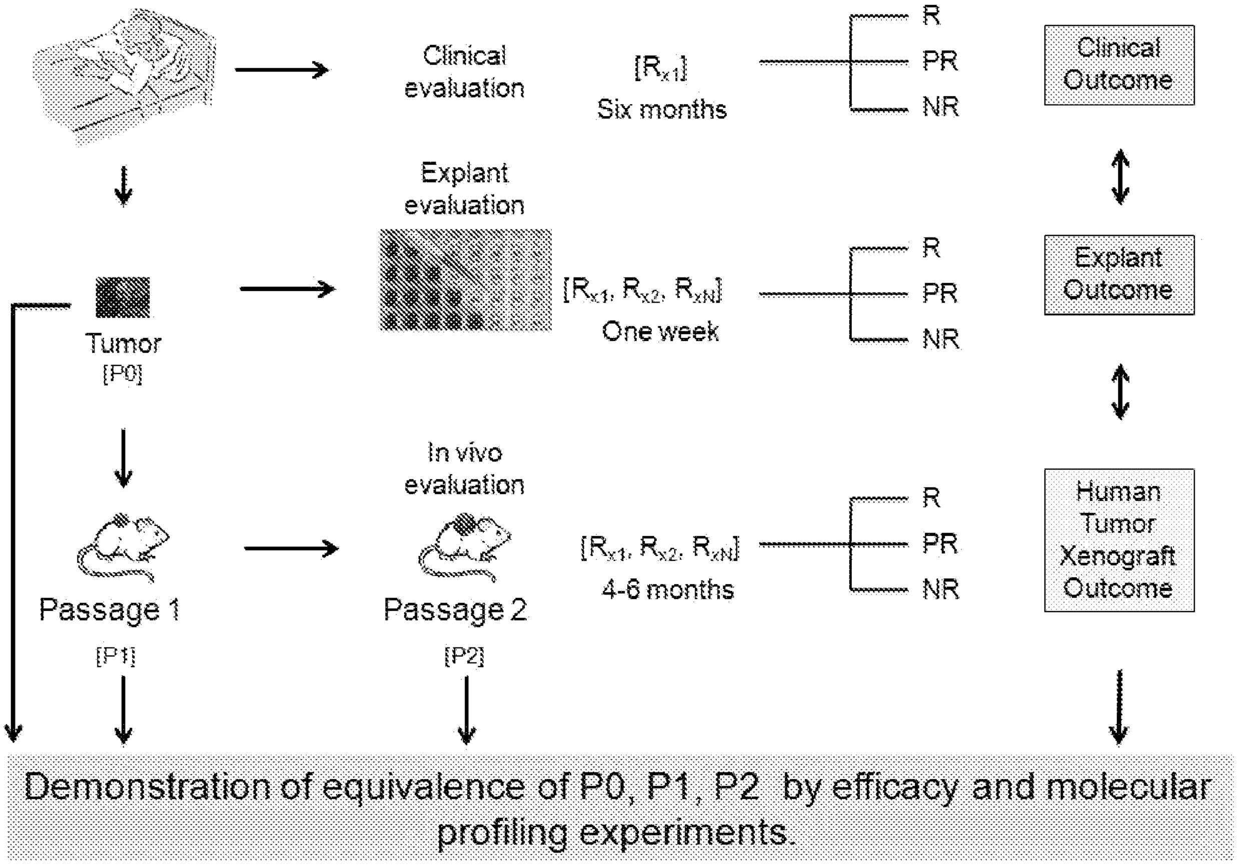
Clinical
evaluation
[Rx1]
Six months
R
PR
NR
Clinical
Outcome
Explant
evaluation
Tumor
[P0]
[Rx1, Rx2, RxN]
One week
R
PR
NR
Explant
Outcome
In vivo
evaluation
Passage 1
[P1]
Passage 2
[P2]
[Rx1, Rx2, RxN]
4-6 months
R
PR
NR
Human
Tumor
Xenograft
Outcome
Demonstration of equivalence of P0, P1, P2 by efficacy and molecular profiling experiments.

**Figure 1**

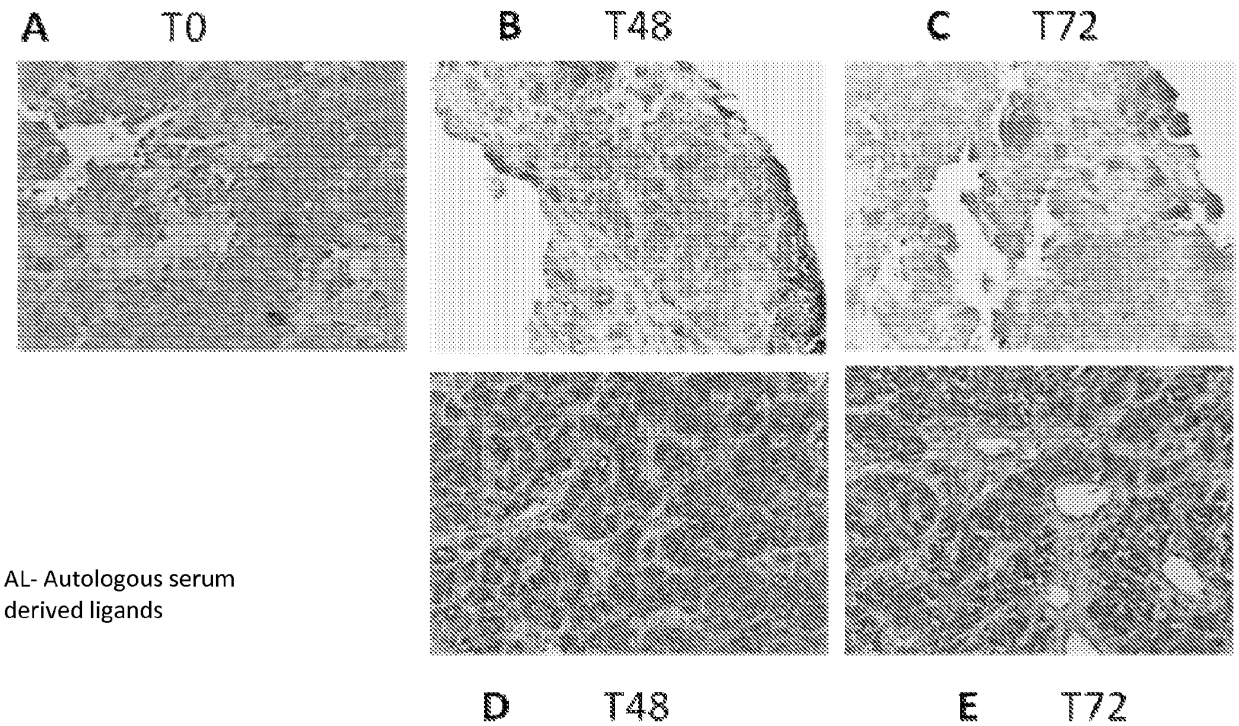
A
T0
B
T48
C
T72
AL- Autologous serum
derived ligands
D
T48
E
T72

Figure 2

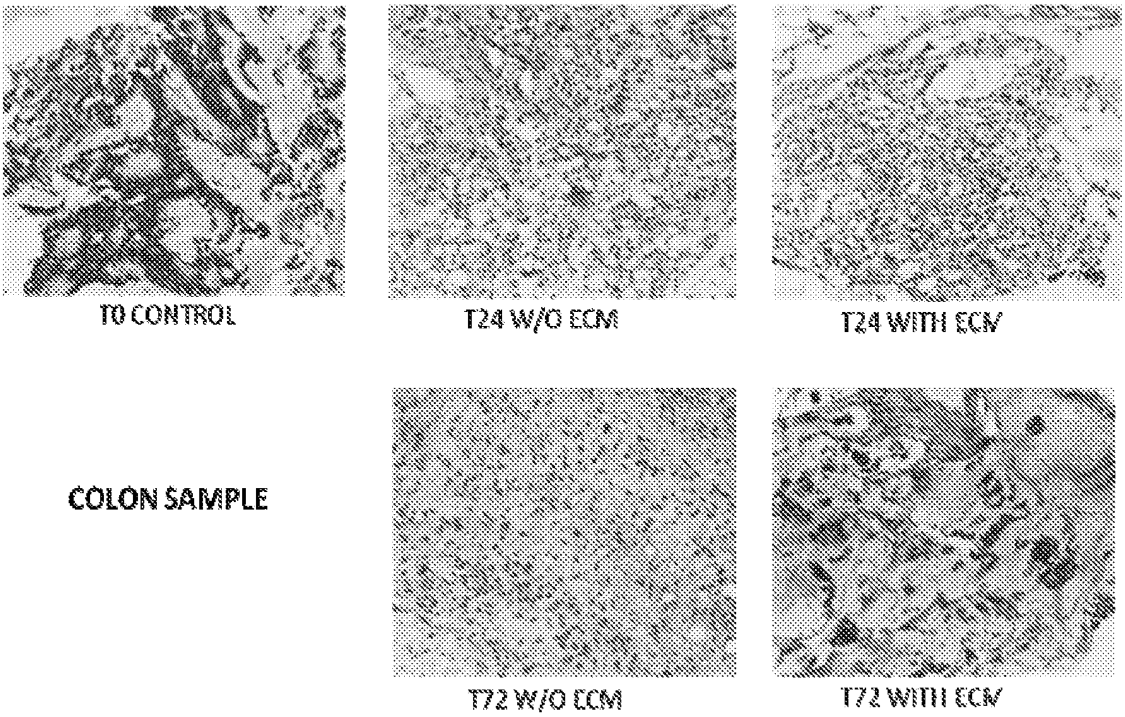


Figure 3A

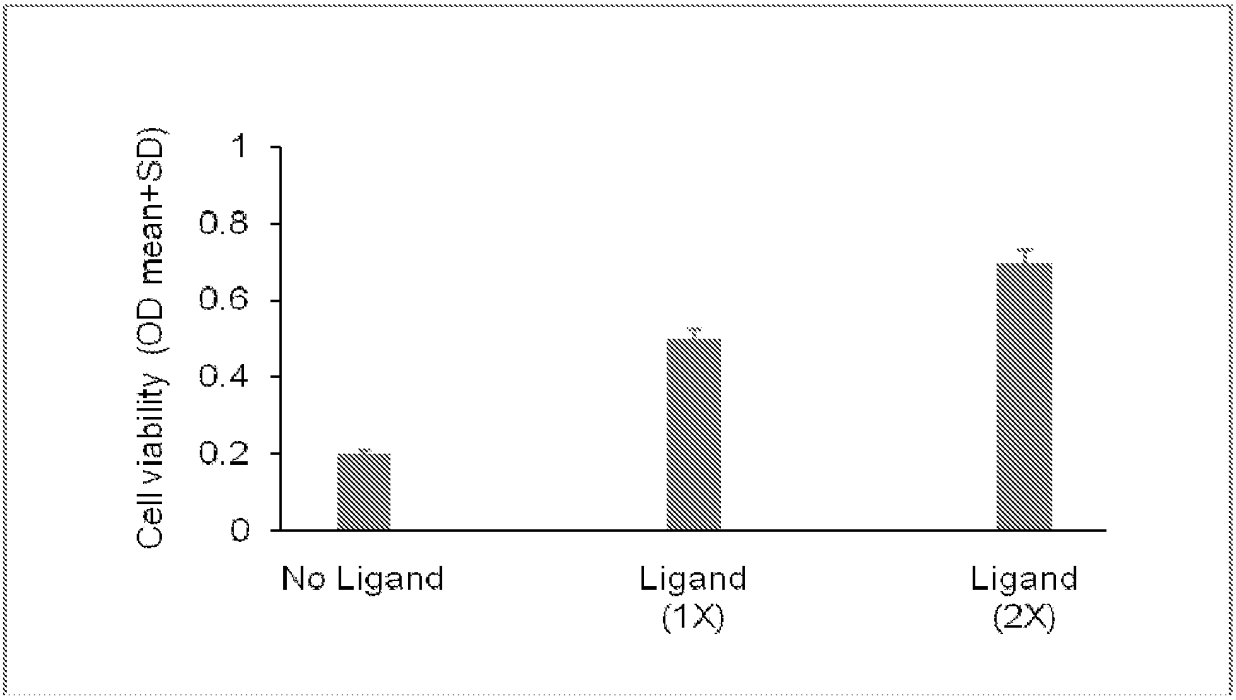


Figure 3B

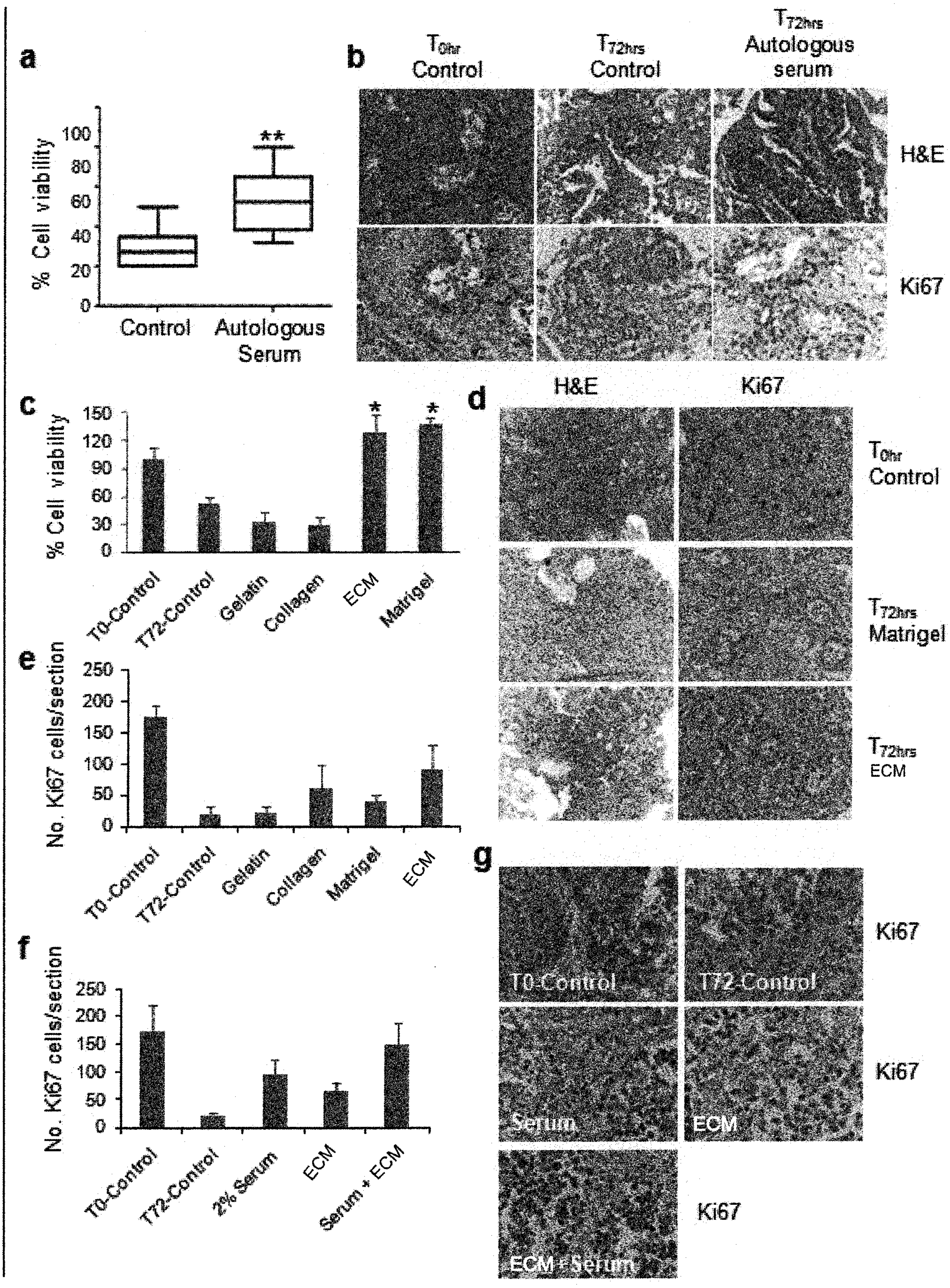
a
% Cell viability
100
80
60
40
20
0
**
Control
Autologous Serum
b
T0hr Control
T72hrs Control
T72hrs Autologous serum
H&E
Ki67
c
% Cell viability
150
120
90
60
30
0
*
*
T0-Control
T72-Control
Gelatin
Collagen
ECM
Matrigel
d
H&E
Ki67
T0hr Control
T72hrs Matrigel
T72hrs ECM
e
No. Ki67 cells/section
250
200
150
100
50
0
T0-Control
T72-Control
Gelatin
Collagen
Matrigel
ECM
f
No. Ki67 cells/section
250
200
150
100
50
0
T0-Control
T72-Control
2% Serum
ECM
Serum + ECM
g
Ki67
T0-Control
T72-Control
Ki67
Serum
ECM
Ki67
ECM+Serum

Figure 4

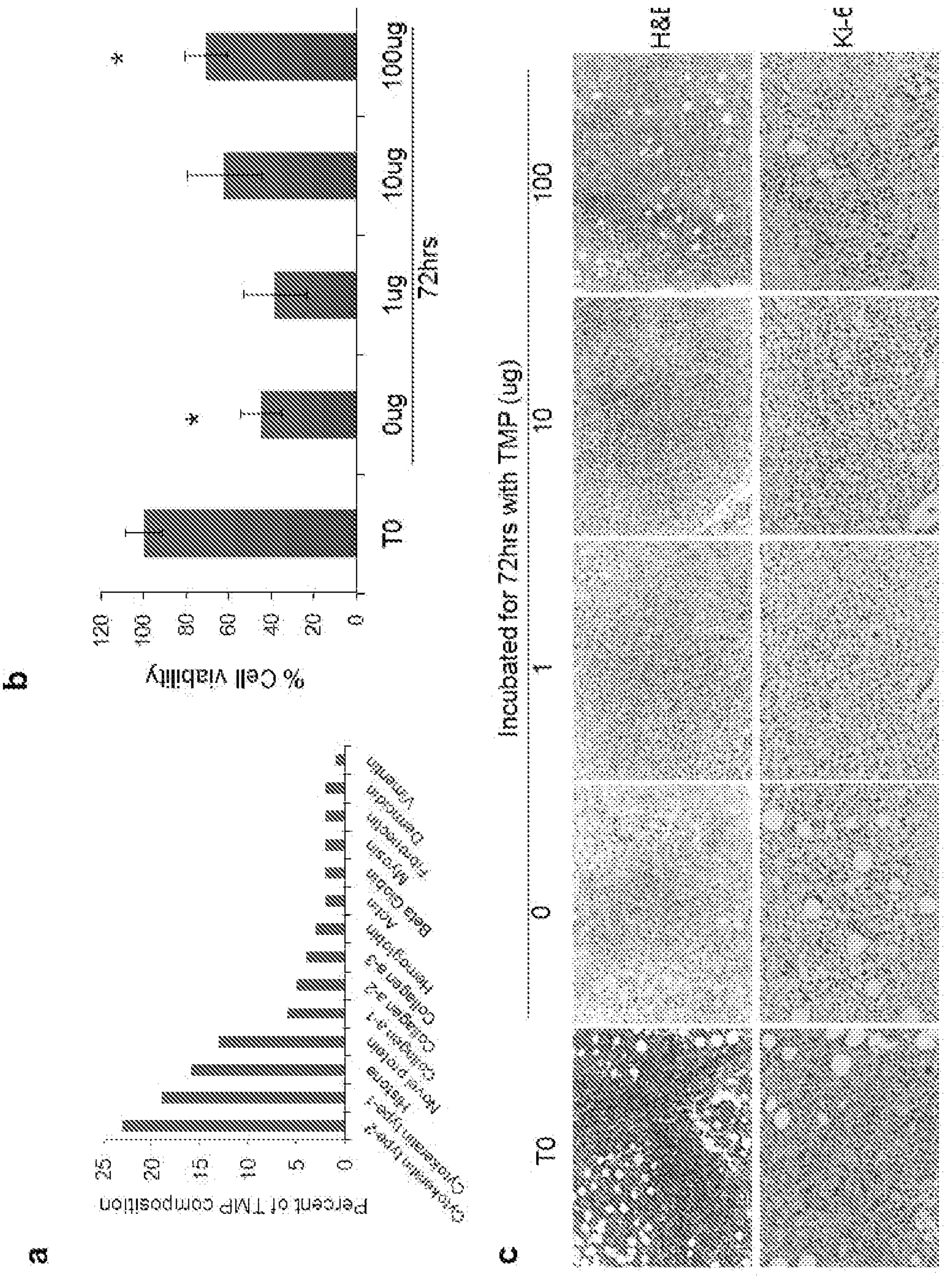
a
Percent of TMP composition
25
20
15
10
5
0
Cytokeratin type-2
Cytokeratin type-1
Histone
Novel protein
Collagen a-1
Collagen a-2
Collagen a-3
Hemoglobin
Actin
Beta Globin
Myosin
Fibronectin
Dermcidin
Vimentin
b
% Cell viability
120
100
80
60
40
20
0
T0
0ug
1ug
10ug
100ug
72hrs
*
*
c
Incubated for 72hrs with TMP (ug)
T0
0
1
10
100
H&E

Figure 5

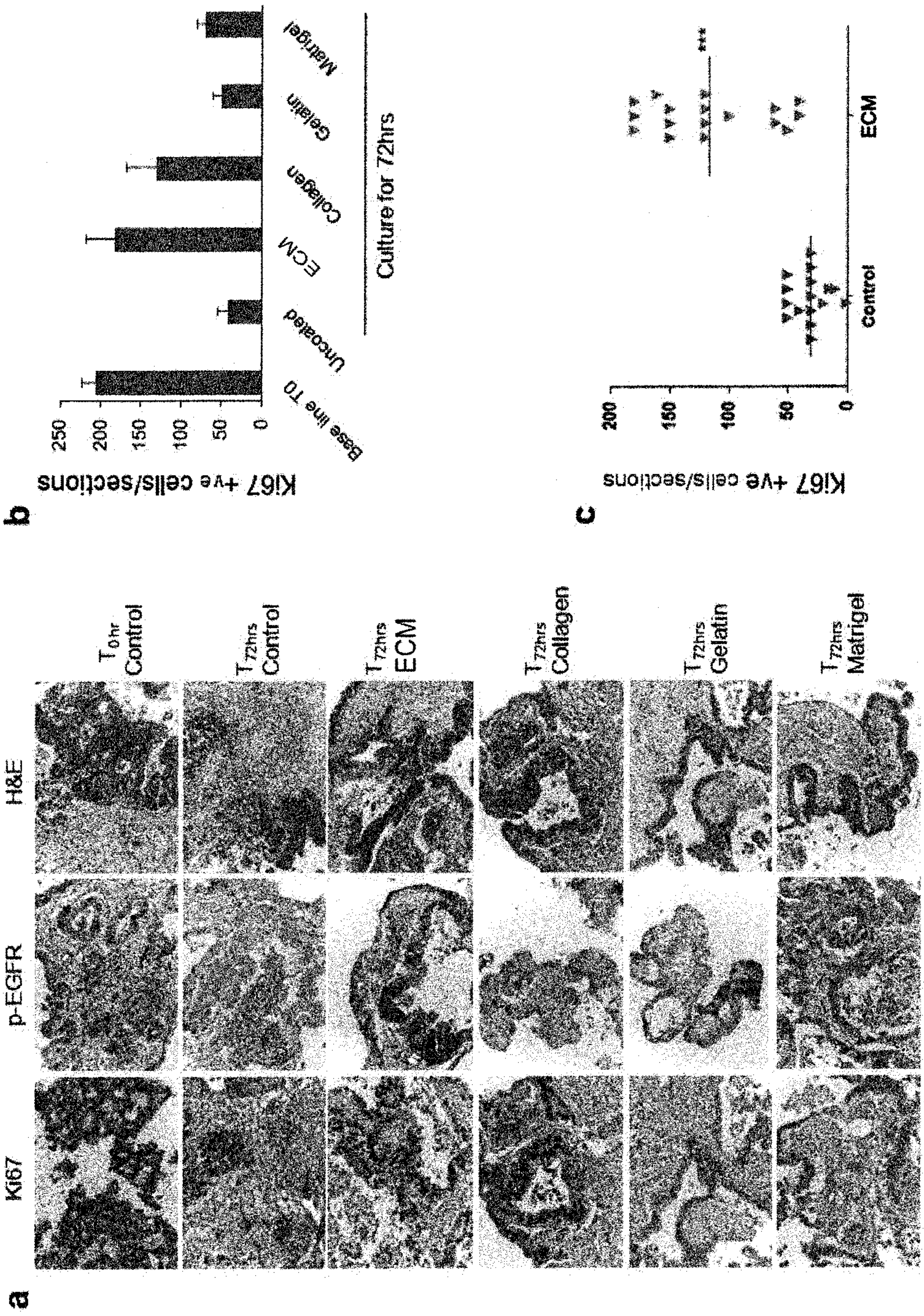
a
Ki67
p-EGFR
H&E
$T_{0hr}$ Control
$T_{72hrs}$ Control
$T_{72hrs}$ ECM
$T_{72hrs}$ Collagen
$T_{72hrs}$ Gelatin
$T_{72hrs}$ Matrigel
b
Ki67 +ve cells/sections
250
200
150
100
50
0
Base line T0
Uncoated
ECM
Collagen
Gelatin
Matrigel
Culture for 72hrs
c
Ki67 +ve cells/sections
200
150
100
50
0
***
Control
ECM

Figure 6

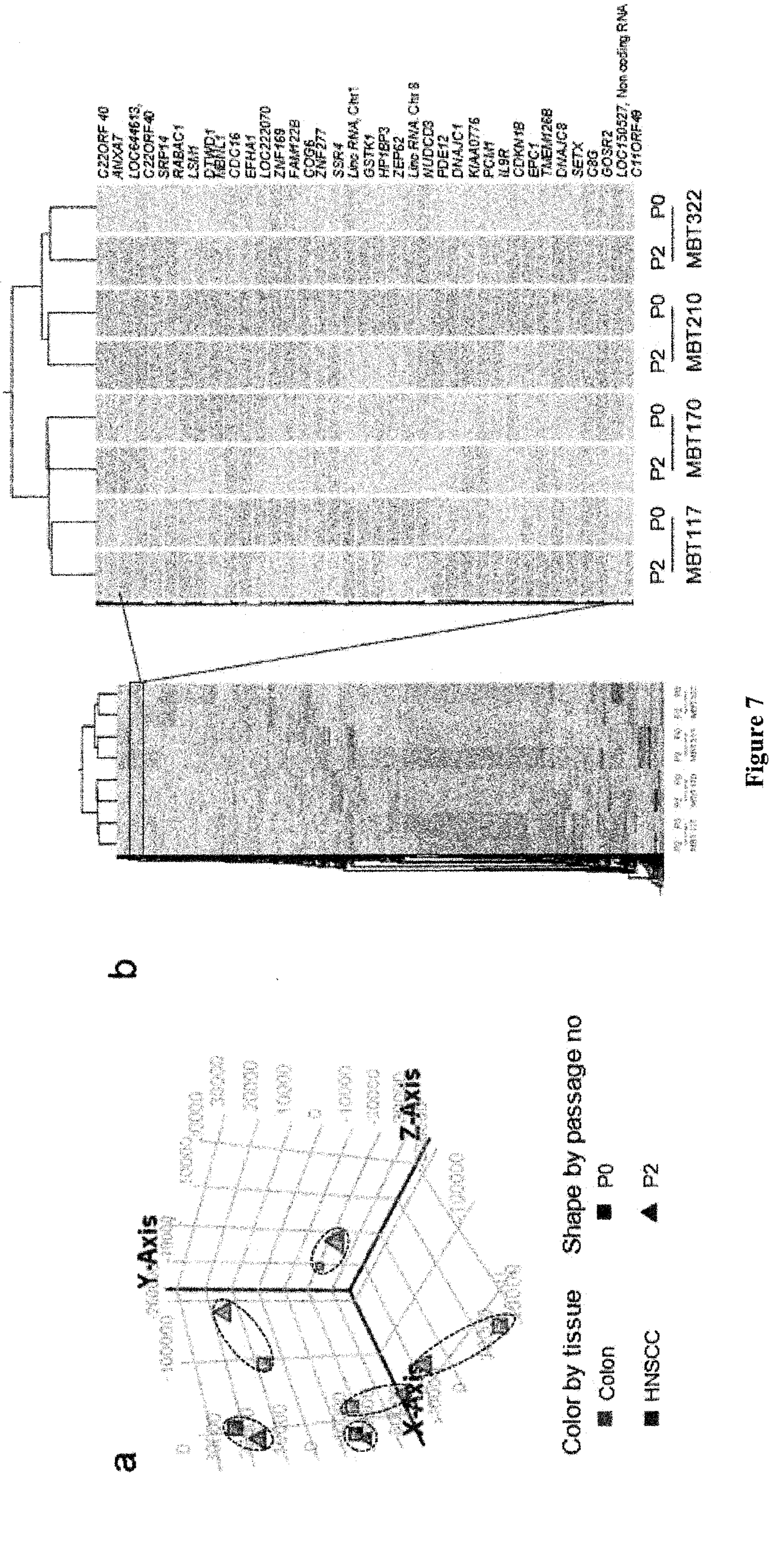
a
Y-Axis
X-Axis
Z-Axis
Color by tissue
Colon
HNSCC
Shape by passage no
P0
P2
b
C22ORF 40
ANXA7
LOC644613, C22ORF40
SRP14
RABAC1
LSM1
DTWD1
MBNL1
CDC16
EFHA1
LOC222070
ZNF169
FAM22B
COG6
ZNF277
SSR4
Linc RNA, Chr1
GSTK1
HP1BP3
ZEP62
Linc RNA, Chr 8
NUDCD3
PDE12
DNAJC1
KIAA0776
PCM1
IL9R
CDKN1B
EPC1
TMEM26B
DNAJC8
SETX
C8G
GOSR2
LOC150527, Non coding RNA
C11ORF49
P2
P0
MBT117
P2
P0
MBT170
P2
P0
MBT210
P2
P0
MBT322

Figure 7

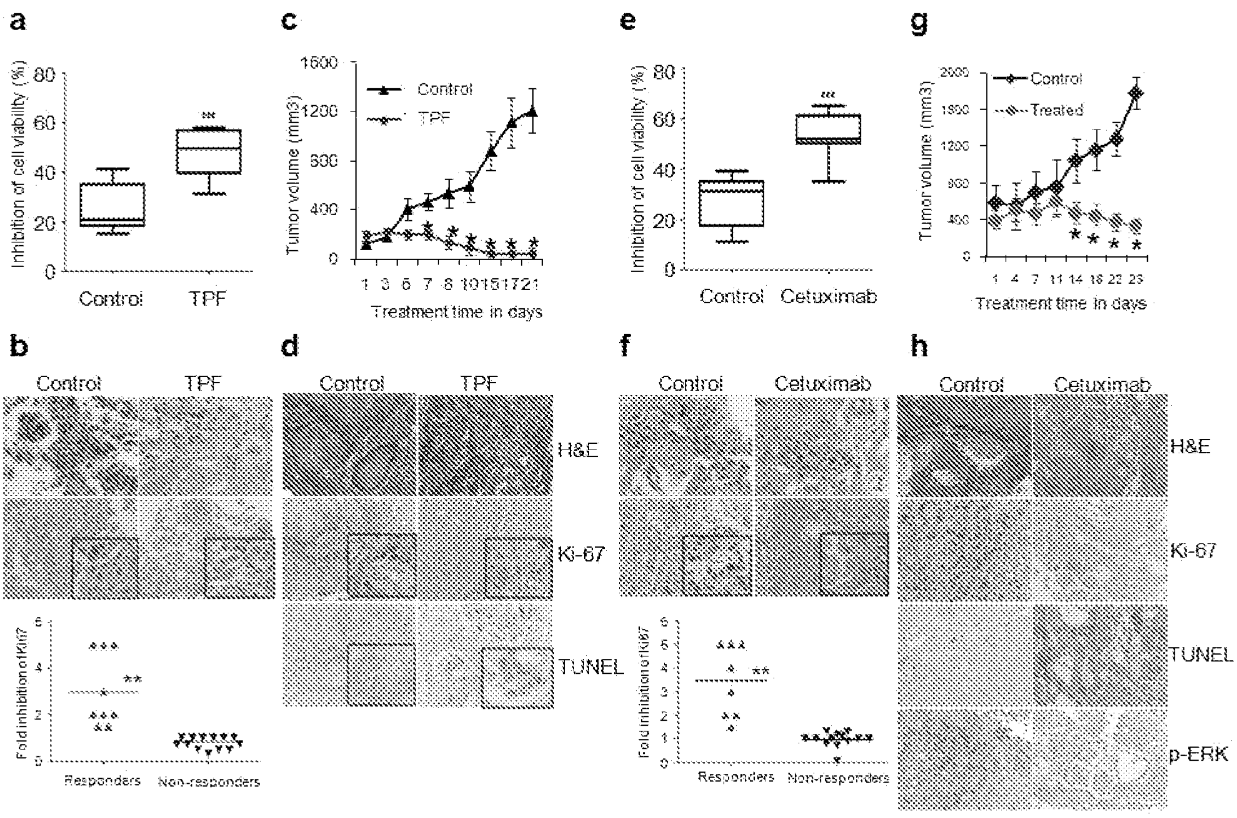


**Figure 9**

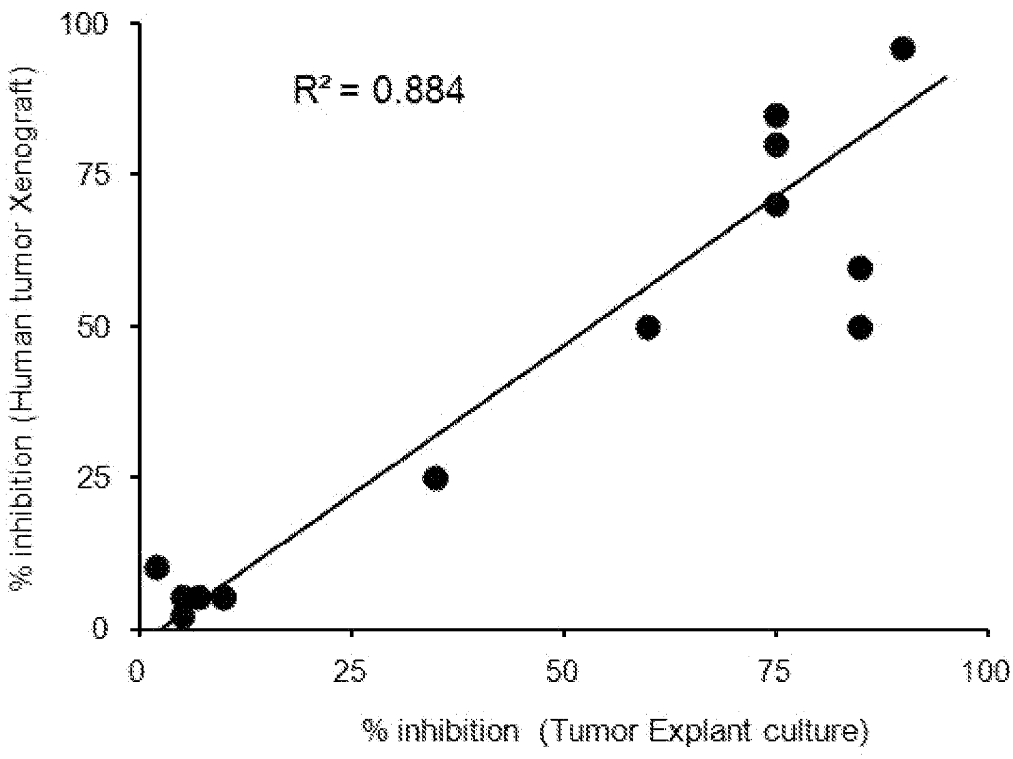


**Figure 10**

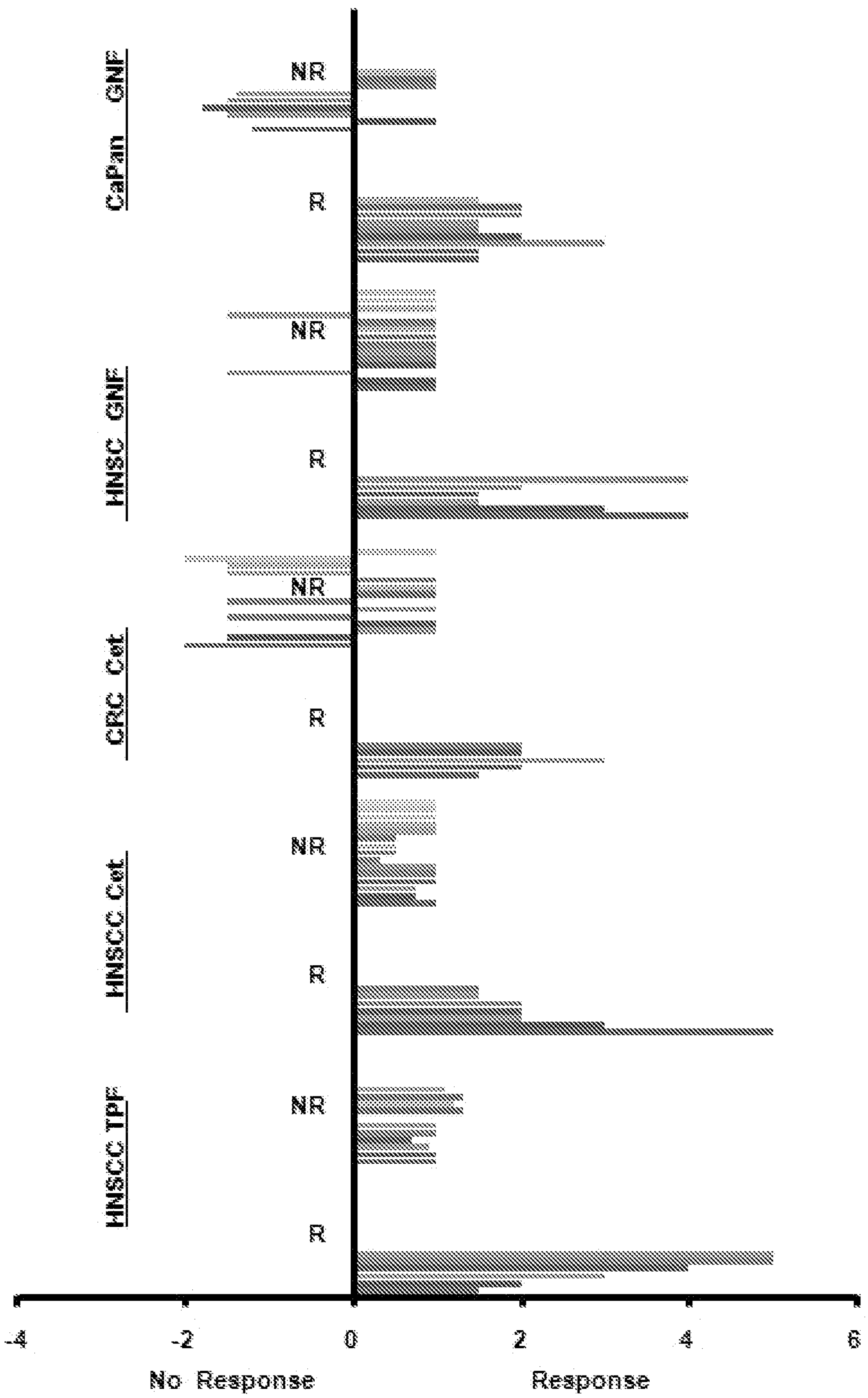
CaPan GNF
NR
R
HNSC GNF
NR
R
CRC Cet
NR
R
HNSCC Cet
NR
R
HNSCC TPF
NR
R
-4
-2
0
2
4
6
No Response
Response

Figure 11

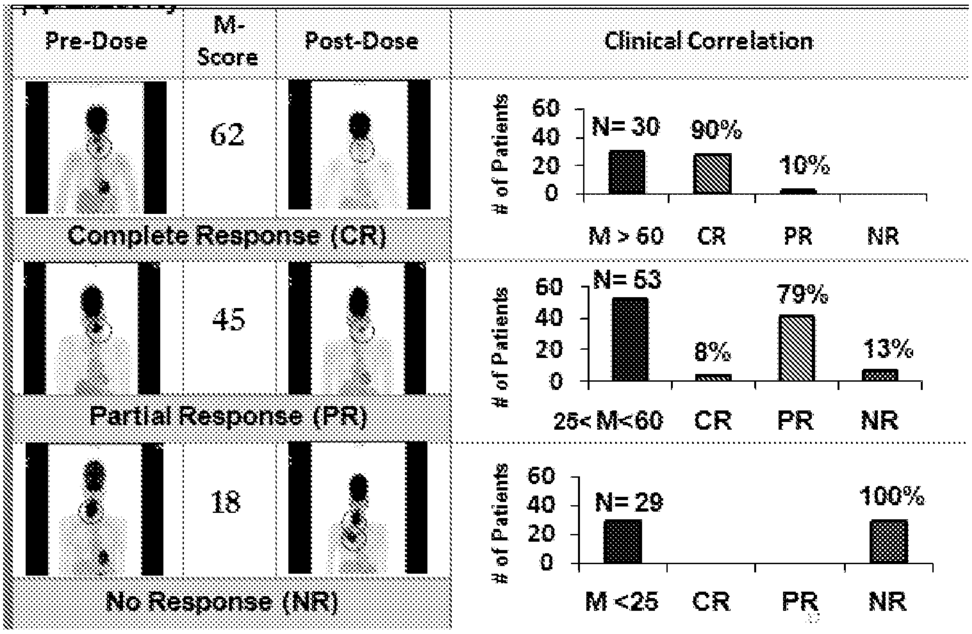


Figure 12

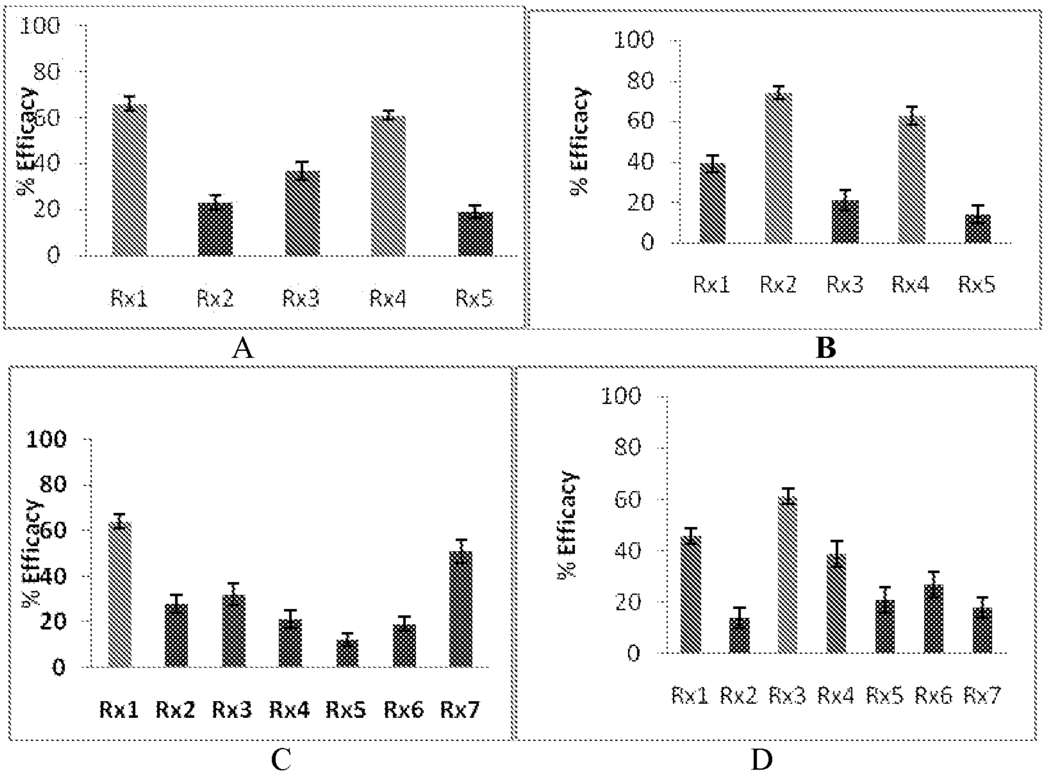


Figure 13

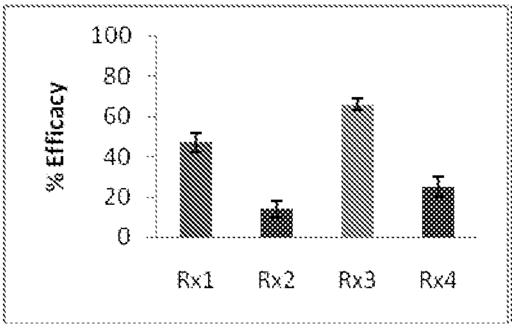


E

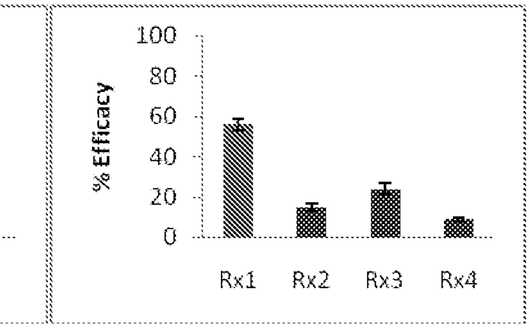


F

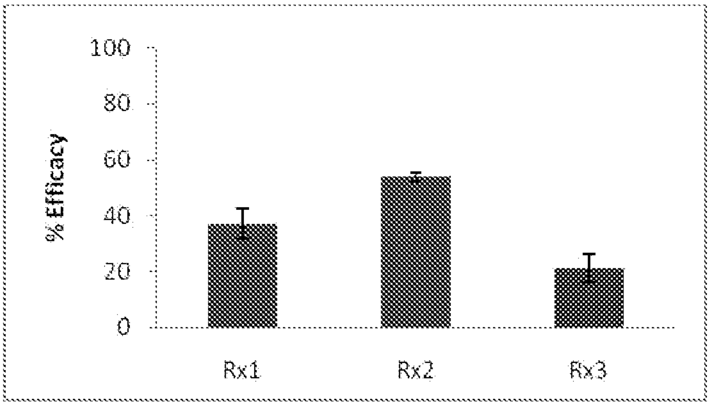


G

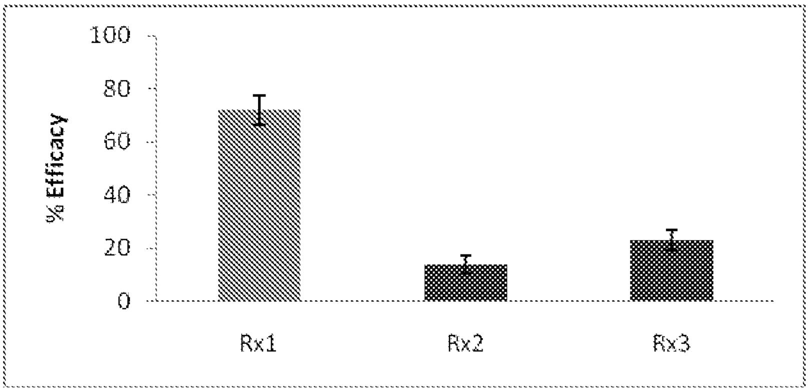


H

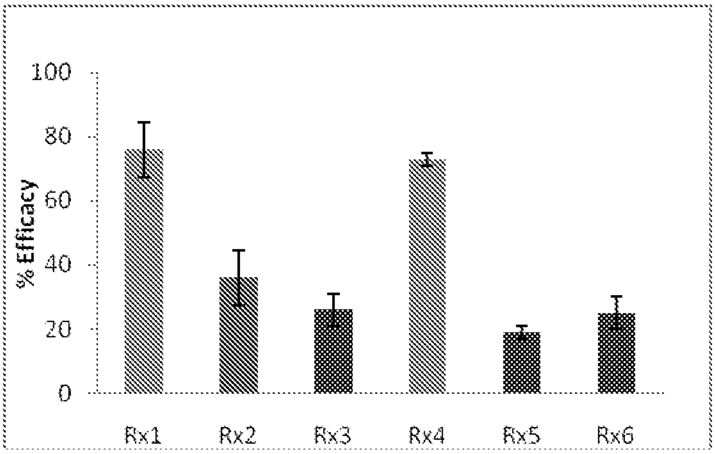


I

**Figure 13**

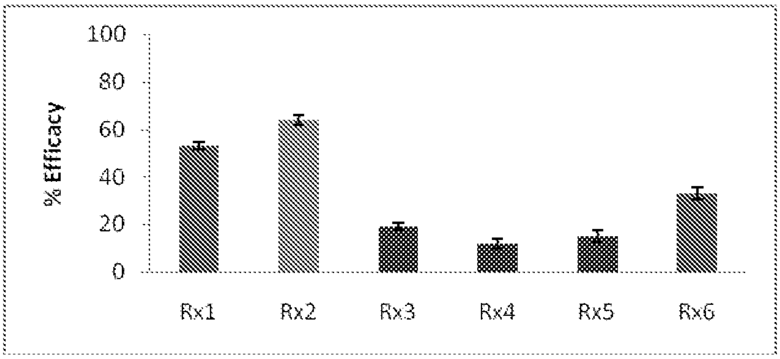


J

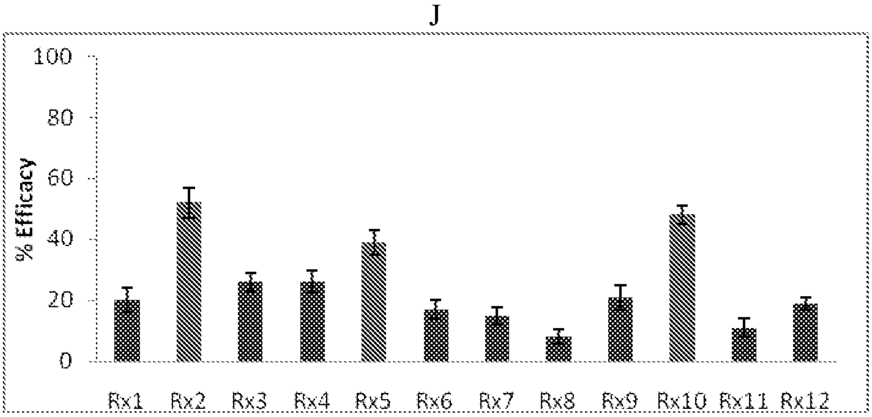


K

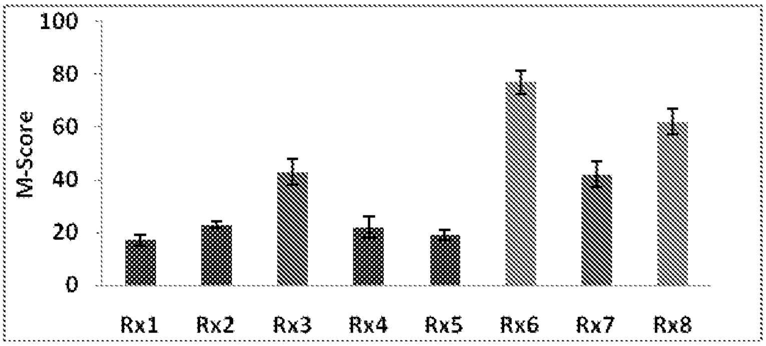


L

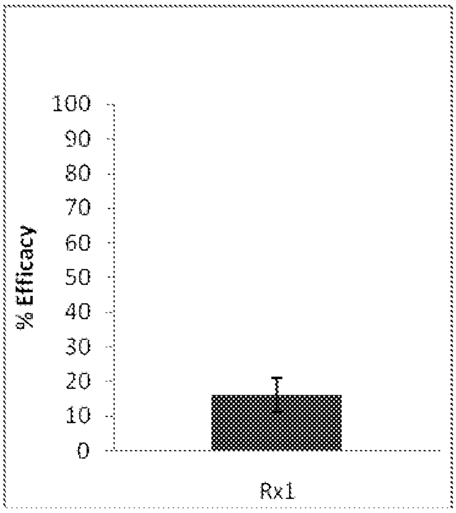


M

**Figure 13**

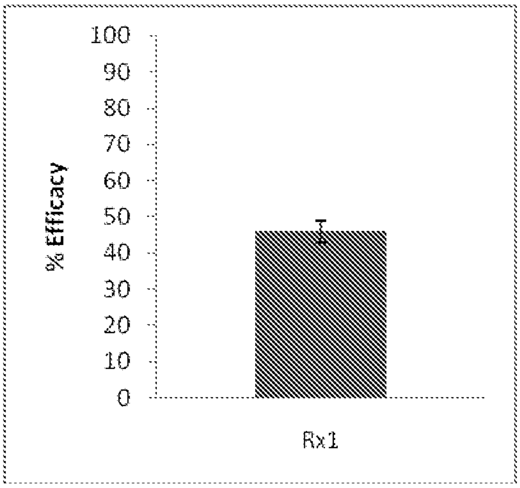


N

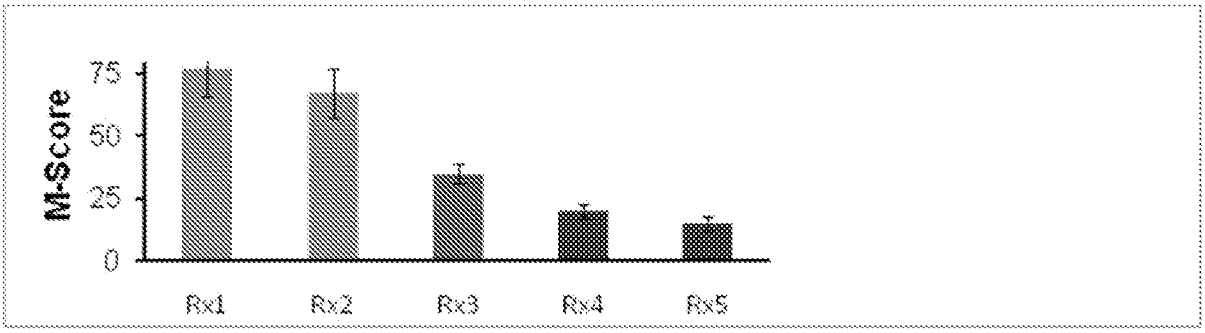


O

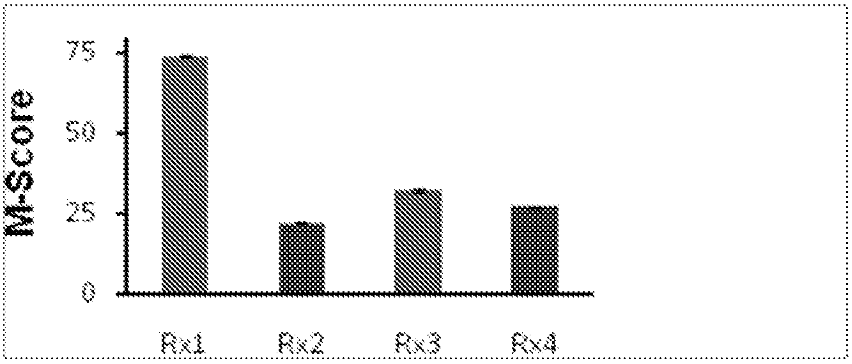


P

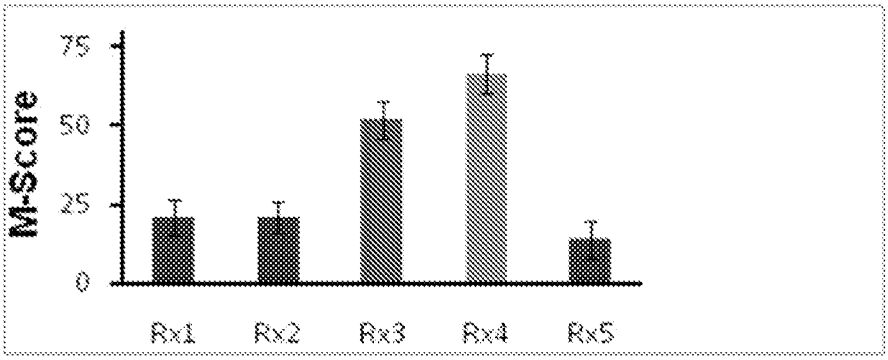


Q

Figure 13

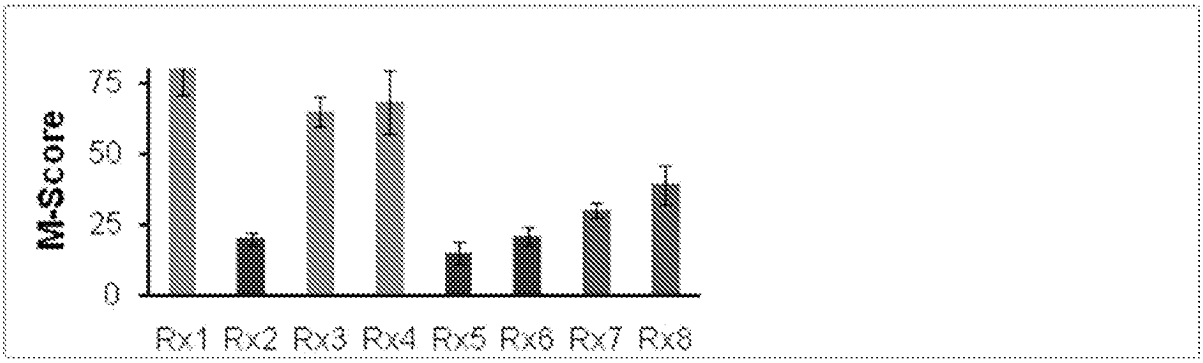
M-Score
75
50
25
0
Rx1 Rx2 Rx3 Rx4 Rx5 Rx6 Rx7 Rx8

R

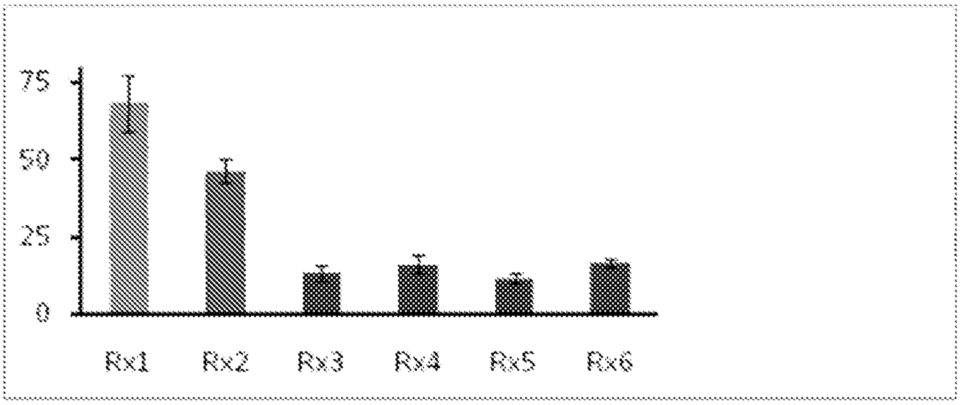
M-Score
75
50
25
0
Rx1 Rx2 Rx3 Rx4 Rx5 Rx6

S

**Figure 13**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **CHRIS S. HUGHES et al.** Matrigel: A complex protein mixture required for optimal growth of cell culture. *PROTEOMICS,* 16 May 2010, vol. 10 (9
- **SANDRA Z HASLAM et al.** Tumour-stroma interactions: Reciprocal regulation of extracellular matrix proteins and ovarian steroid activity in the mammary gland. *BREAST CANCER RESEARCH,* 02 August 2001, vol. 3 (6
- **N. E. CAMPBELL et al.** Extracellular Matrix Proteins and Tumor Angiogenesis. *JOURNAL OF ONCOLOGY,* 01 January 2010, vol. 72 (6), ISSN 1687-8450, 787-13
- **VEIRA V et al.** *Max Loda Lab PNAS,* 2010